# EUROPEAN PATENT APPLICATION

(11) **EP 4 818 202 A2**
(43) Date of publication of application: **07.10.2026**
(21) Application number: 26185841.9
(22) Date of filing: 18.03.2022
(51) Int. Cl.: C07K 14/72

(54) **MULTIPLEX EDITING WITH CAS ENZYMES**

(30) Priority: 19.03.2021 US 202163163510 P; 10.05.2021 US 202163186506 P; 08.09.2021 US 202163241916 P
(62) Document number(s) of the earlier application(s) in accordance with Art. 76 EPC: 22772310.3 / 4 308 699
(71) Applicant: Metagenomi Therapeutics, Inc., Emeryville, CA 94608 (US)
(72) Inventor: THOMAS, Brian, Emeryville, CA 94608 (US); BROWN, Christopher, Emeryville, CA 94608 (US); DEVOTO, Audra, Emeryville, CA 94608 (US); BUTTERFIELD, Cristina, Emeryville, CA 94608 (US); ALEXANDER, Lisa, Emeryville, CA 94608 (US); GOLTSMAN, Daniela S A, Emeryville, CA 94608 (US); COST, Greg, Emeryville, CA 94608 (US); LAMOTHE, Rebecca, Emeryville, CA 94608 (US); ESPINOSA, Diego, Emeryville, CA 94608 (US)
(74) Representative: Mewburn Ellis LLP

(57) **Abstract**

Described herein are methods, compositions, and systems for multiplex editing using Cas enzymes or editing of T-cells or related cells using Cas enzymes.

## Description

### CROSS-REFERENCE

This application claims the benefit of U.S. Provisional Application No. 63/163,510, entitled "MULTIPLEX EDITING WITH CAS ENZYMES", filed on March 19, 2021; U.S. Provisional Application No. 63/186,506, entitled "MULTIPLEX EDITING WITH CAS ENZYMES", filed on May 10, 2021; and U.S. Provisional Application No. 63/241,916, entitled "MULTIPLEX EDITING WITH CAS ENZYMES", filed on September 8, 2021; each of which are incorporated by reference herein in their entireties.

### SEQUENCE LISTING

The instant application contains a Sequence Listing which has been submitted electronically in ASCII format and is hereby incorporated by reference in its entirety. Said ASCII copy, created on March 17, 2022, is named 55921-719-601-SL.txt and is 70,612 bytes in size.

### BACKGROUND

Cas enzymes along with their associated Clustered Regularly Interspaced Short Palindromic Repeats (CRISPR) guide ribonucleic acids (RNAs) appear to be a pervasive (~45% of bacteria, ~84% of archaea) component of prokaryotic immune systems, serving to protect such microorganisms against non-self nucleic acids, such as infectious viruses and plasmids by CRISPR-RNA guided nucleic acid cleavage. While the deoxyribonucleic acid (DNA) elements encoding CRISPR RNA elements may be relatively conserved in structure and length, their CRISPR-associated (Cas) proteins are highly diverse, containing a wide variety of nucleic acid-interacting domains. While CRISPR DNA elements have been observed as early as 1987, the programmable endonuclease cleavage ability of CRISPR/Cas complexes has only been recognized relatively recently, leading to the use of recombinant CRISPR/Cas systems in diverse DNA manipulation and gene editing applications.

### SUMMARY

In some aspects, the present disclosure provides for a method of editing two or more loci within a cell, comprising contacting to said cell: (a) a class 2, type II Cas endonuclease complex comprising: (i) a class 2, type II Cas endonuclease; and (ii) a first engineered guide RNA comprising: an RNA sequence configured to bind to the class 2, type II Cas endonuclease, and a spacer sequence configured to hybridize to a first set of one or more target loci; (b) a class 2, type V Cas endonuclease complex comprising: (i) a class 2, type V Cas endonuclease; and (ii) a second engineered guide RNA comprising: an RNA sequence configured to bind to the class 2, type V Cas endonuclease, and a spacer sequence configured to hybridize to a second set of one or more target loci. In some embodiments, said class 2, type II Cas endonuclease is not a Cas9 endonuclease. In some embodiments, said class 2, type II Cas endonuclease is a Cas12a endonuclease. In some embodiments, said class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof. In some embodiments, said class 2, type V Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to SEQ ID NO: 7 or a variant thereof. In some embodiments, said first engineered guide RNA or said second engineered guide RNA comprises a sequence having at least 80%, 85%, 90%, or 95% sequence identity to any one of SEQ ID NOs: 3, 6, or 9. In some embodiments, said method edits genomic sequences of said first locus with at least about 25%, 30%, 35%, 40%, 45%, 50%, 55%, 60%, 65%, 70%, 75%, 80%, 85%, 90%, or more efficiency and/or said second locus with at least about 25%, 30%, 35%, 40%, 45%, 50%, 55%, 60%, 65%, 70%, 75%, 80%, 85%, 90%, or more efficiency. In some embodiments, said first RNA-guided endonuclease or said second RNA-guided endonuclease is introduced at a concentration of 200 pmol or less, 100 pmol or less, 50 pmol or less, 25 pmol or less, 5 pmol or less, or 1 pmol or less. In some embodiments, off-target sites within said cell are disrupted at a frequency of less than 0.2% as determined by a genome-wide off-target double-strand break analysis. In some embodiments, off-target sites within said cell are disrupted at a frequency of less than 0.01% as determined by a genome-wide off-target double-strand break analysis. In some embodiments, said first set of one or more target loci or said second set of one or more target loci comprises a T-cell receptor (TCR) locus. In some embodiments, said spacer sequence configured to hybridize to said first set of one or more target loci or said spacer sequence configured to hybridize to said second set of one or more target loci has at least 80%, 85%, 86%, 87%, 88%, 89%, 90%, 91%, 92%, 93%, 94%, 95%, 96%, 97%, 98%, or 99% sequence identity to any one of SEQ ID NOs: 10-15, a complement thereof, or a reverse complement thereof. In some embodiments, said first set of one or more target loci or said second set of one or more target loci comprises an albumin (ALB) locus. In some embodiments, said spacer sequence configured to hybridize to said first set of one or more target loci or said spacer sequence configured to hybridize to said second set of one or more target loci has at least 80%, 85%, 86%, 87%, 88%, 89%, 90%, 91%, 92%, 93%, 94%, 95%, 96%, 97%, 98%, or 99% sequence identity to any one of SEQ ID NOs: 17-19, a complement thereof, or a reverse complement thereof. In some embodiments, said first set of one or more target loci or said second set of one or more target loci comprises a Nuclear Receptor Subfamily 3 Group C Member 1 (NR3C1) locus. In some embodiments, said spacer sequence configured to hybridize to said first set of one or more target loci or said spacer sequence configured to hybridize to said second set of one or more target loci has at least 80%, 85%, 86%, 87%, 88%, 89%, 90%, 91%, 92%, 93%, 94%, 95%, 96%, 97%, 98%, or 99% sequence identity to any one of SEQ ID NOs: 16, 20, 21, or 22, a complement thereof, or a reverse complement thereof. In some embodiments, the method further comprises introducing to said cell a donor DNA sequence comprising an open reading frame encoding a heterologous engineered T-cell receptor molecule, a first homology arm comprising a DNA sequence located on a first side of said first set of one or more target loci and a second homology arm comprising a DNA sequence located on a second side of said first set of one or more target loci. In some embodiments, editing comprises insertion of an indel, a premature termination codon, a missense codon, a frameshift mutation, an adenine deamination, a cytosine deamination, or any combination thereof.

In some aspects, the present disclosure provides for a method of making a glucocorticoid-resistant engineered T cell, comprising introducing to a T-cell or a precursor thereof: (a) an RNA guided endonuclease complex targeting a T-cell receptor (TCR) locus, comprising: (i) a first RNA guided endonuclease or DNA encoding said first RNA guided endonuclease; and (ii) a first engineered guide RNA comprising an RNA sequence configured to form a complex with said first RNA guided endonuclease, and a first spacer sequence configured to hybridize to at least part of said TCR locus; and (b) an RNA guided endonuclease complex targeting a T-cell receptor Nuclear Receptor Subfamily 3 Group C Member 1 (NR3C1) locus, comprising: (i) a second RNA guided endonuclease; and (ii) a second engineered guide RNA comprising: an RNA sequence configured to form a complex with said second RNA guided endonuclease, and a second spacer sequence configured to hybridize to at least part of said NR3C1 locus. In some embodiments, said at least part of said TCR locus is within said T-cell locus. In some embodiments, the method further comprises introducing to said cell (b) a donor DNA sequence comprising an open reading frame encoding a heterologous engineered T-cell receptor molecule, a first homology arm comprising a DNA sequence located on a first side of said target sequence and a second homology arm comprising a DNA sequence located on a second side of said target sequence within said TCR locus. In some embodiments, said first RNA guided endonuclease or said second RNA guided endonuclease comprises a class 2, type II or a class 2, type V Cas endonuclease. In some embodiments, said first RNA guided endonuclease comprises said class 2, type II Cas endonuclease and said second RNA guided endonuclease comprises said class 2, type V Cas endonuclease. In some embodiments, said second RNA guided endonuclease comprises said class 2, type II Cas endonuclease and said first RNA guided endonuclease comprises said class 2, type V Cas endonuclease. In some embodiments, said heterologous engineered T-cell receptor is a CAR molecule. In some embodiments, said at least part of said T cell receptor locus is a T Cell Receptor Alpha Constant (TRAC) locus or a T Cell Receptor Beta Constant (TRBC) locus. In some embodiments, said homology arms comprise intronic or exonic regions within said TCR locus proximal to said at least part of said T cell receptor locus. In some embodiments, said at least part of said T cell receptor locus is a first or third exon of TRAC. In some embodiments, said method disrupts genomic sequences of said TCR locus and said NR3C1 locus with at least about 25%, 30%, 35%, 40%, 45%, 50%, 55%, 60%, 65%, 70%, 75%, 80%, 85%, 90%, or more efficiency. In some embodiments, said efficiency is determined by flow cytometry for a protein expressed from said TCR and NR3C1 loci. In some embodiments, said at least part of said NR3C1 locus is exon 2 or exon 3. In some embodiments, said method produces cells positive for the CAR molecule and negative for NR3C1 with at least about 25%, 30%, 35%, 40%, 45%, 50%, 55%, 60%, 65%, 70%, 75%, 80%, 85%, 90%, or more efficiency. In some embodiments, the method further comprises introducing (a)-(c) to said T-cell or precursor thereof simultaneously. In some embodiments, said first RNA-guided endonuclease or said second RNA-guided endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1, 4, or 7, or a variant thereof. In some embodiments, said first engineered guide RNA or said second engineered guide RNA comprises a sequence having at least 80%, 85%, 86%, 87%, 88%, 89%, 90%, 91%, 92%, 93%, 94%, 95%, 96%, 97%, 98%, or 99% sequence identity to any one of SEQ ID NOs: 3, 6, or 9, a complement thereof, or a reverse complement thereof. In some embodiments, said first RNA-guided endonuclease or said second RNA-guided endonuclease is present at a concentration of 100 pmol or less, 50 pmol or less, 25 pmol or less, 5 pmol or less, or 1 pmol or less. In some embodiments, said T-cell or said precursor thereof comprises a T-cell, a hematopoietic stem cell (HSC), or peripheral blood mononuclear cell (PBMC). In some embodiments, said second spacer sequence comprises a sequence having at least 80%, 85%, 86%, 87%, 88%, 89%, 90%, 91%, 92%, 93%, 94%, 95%, 96%, 97%, 98%, or 99% sequence identity to any one of SEQ ID NOs: 16, 20, 21, or 22, a complement thereof, or a reverse complement thereof. In some embodiments, said first or said second spacer sequence comprises at least about 19-24 nucleotides, at least about 19 nucleotides, at least about 20 nucleotides, at least about 22 nucleotides, or at least about 24 nucleotides. In some embodiments, said donor DNA sequence is delivered in a viral vector. In some embodiments, said viral vector is an AAV or AAV-6 vector.

In some aspects, the present disclosure provides for a population of glucocorticoid-resistant T cells or precursors thereof, comprising: (a) an heterologous sequence within 100, 75, 50, 25, or 10 nucleotides of a hybridization region of any one of SEQ ID NOs: 10-15 within a TCR locus. In some embodiments, the T cell or precursor thereof further comprises (b) an NR3C1 locus comprising an indel. In some embodiments, said heterologous sequence is an indel. In some embodiments, said heterologous sequence comprises an open reading frame comprising a nucleotide sequence encoding a heterologous T-cell receptor or a CAR molecule. In some embodiments, said NR3C1 locus comprises an indel within 100, 75, 50, 25, or 10 nucleotides of a hybridization region of any one of SEQ ID NOs: 16, 20, 21, or 22. In some embodiments, less than 0.2% of said cells have indels at off-target loci as determined by a genome-wide off-target double-strand break analysis. In some embodiments, less than 0.01% of said cells have indels at off-target loci as determined by a genome-wide off-target double-strand break analysis. In some embodiments, said population of cells is substantially free of chromosomal translocations.

In some aspects, the present disclosure provides for a method of editing two or more loci within a cell, comprising contacting to said cell: (a) a first Cas endonuclease complex comprising: (i) a first Cas endonuclease; and (ii) one or more engineered guide RNAs comprising: an RNA sequence configured to bind to the class 2, type II Cas endonuclease, and a spacer sequence configured to hybridize to a first target sequence; (b) a second Cas endonuclease complex comprising: (i) a second Cas endonuclease; and (ii) one or more engineered guide RNAs comprising: an RNA sequence configured to bind to the class 2, type II Cas endonuclease, and a spacer sequence configured to hybridize to a second target sequence. In some embodiments, the method further comprises introducing to said cell (c) a first donor DNA sequence comprising an open reading frame encoding a first transgene, a 5' homology arm comprising a DNA sequence located on a 5' side of said first target sequence and a 3' homology arm comprising a DNA sequence located on a 3' side of said first target sequence; and (d) a second donor DNA sequence comprising an open reading frame encoding a second transgene, a 5' homology arm comprising a DNA sequence located on a 5' side of said second target sequence and a 3' homology arm comprising a DNA sequence located on a 3' side of said second target sequence. In some embodiments, said first transgene and said second transgene are different. In some embodiments, said first target sequence or said second target sequence is a target sequence within a T-cell receptor locus, TRAC, TRBC, NR3C1, or AAVS1 locus, or any combination thereof. In some embodiments, said first or second transgene is an alpha, beta, alpha-D3, or beta-D3 isoform of GR, a CAR molecule, a truncated low-affinity nerve growth factor receptor (tLNGFR) sequence, a truncated version of the epithelial growth factor receptor (tEGFR), a GFP coding sequence, or any combination thereof. In some embodiments, said 5' homology arm comprising a DNA sequence located on a 5' side of said first target sequence or said 5' homology arm comprising a DNA sequence located on a 5' side of said second target sequence comprises SEQ ID NOs: 42 or 23 or a sequence having at least 80%, 85%, 86%, 87%, 88%, 89%, 90%, 91%, 92%, 93%, 94%, 95%, 96%, 97%, 98%, or 99% sequence identity thereto. In some embodiments, said 3' homology arm comprising a DNA sequence located on a 5' side of said first target sequence or said 3' homology arm comprising a DNA sequence located on a 5' side of said second target sequence comprises SEQ ID NOs: 43 or 24 or a sequence having at least 80%, 85%, 86%, 87%, 88%, 89%, 90%, 91%, 92%, 93%, 94%, 95%, 96%, 97%, 98%, or 99% sequence identity thereto. In some embodiments, said first or said second class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof. In some embodiments, said first engineered guide RNA or said second engineered guide RNA comprises a sequence having at least 80%, 85%, 86%, 87%, 88%, 89%, 90%, 91%, 92%, 93%, 94%, 95%, 96%, 97%, 98%, or 99% sequence identity to any one of SEQ ID NOs: 3, 6, or 9, a complement thereof, or a reverse complement thereof. In some embodiments, said spacer sequence configured to hybridize to said first target sequence or said spacer sequence configured to hybridize to said second target sequence has at least 80%, 85%, 86%, 87%, 88%, 89%, 90%, 91%, 92%, 93%, 94%, 95%, 96%, 97%, 98%, or 99% sequence identity to any one of SEQ ID NOs: 16, 20, 21, 22, or 41, or a complement thereof, a complement thereof, or a reverse complement thereof. In some embodiments, said first or said second endonuclease comprises a class 2, type II Cas endonuclease or a class 2, type V Cas endonuclease, or any combination thereof.
In some aspects, the present disclosure provides for an isolated nucleic acid comprising the sequence of any one of SEQ ID NOs: 63-65, or a sequence having at least about 75%, 80%, 85%, 86%, 87%, 88%, 89%, 90%, 91%, 92%, 93%, 94%, 95%, 96%, 97%, 98%, or 99% sequence identity thereto
In some aspects, the present disclosure provides for an isolated nucleic acid comprising any of the sequences described herein, a complement thereof, or a reverse complement thereof. In some embodiments, the isolated nucleic acid is a guide RNA
In some aspects, the present disclosure provides for a cell comprising any of the nucleic acids described herein. In some embodiments, said cell is a T-cell or precursor thereof. In some embodiments, said T-cell or precursor thereof comprises a T-cell, a hematopoietic stem cell (HSC), or a peripheral blood mononuclear cell (PBMC)
In some embodiments, the present disclosure provides for a vector comprising any of the nucleic acids described herein. In some embodiments, said vector is an adeno-associated viral (AAV) vector. In some embodiments, said AAV vector is an AAV-6 serotype vector.

In some aspects, the present disclosure provides for a vector comprising a sequence having at least about 75%, 80%, 85%, 86%, 87%, 88%, 89%, 90%, 91%, 92%, 93%, 94%, 95%, 96%, 97%, 98%, or 99% sequence identity to any one of SEQ ID NOs: 23, 24, 42, or 43. In some embodiments, the vector further comprises a transgene flanked by said sequence having at least about 75%, 80%, 85%, 86%, 87%, 88%, 89%, 90%, 91%, 92%, 93%, 94%, 95%, 96%, 97%, 98%, or 99% sequence identity to any one of SEQ ID NOs: 23, 24, 42, or 43. In some embodiments, said transgene comprises an alpha, beta, alpha-D3, or beta-D3 isoform of GR, a CAR molecule, a truncated low-affinity nerve growth factor receptor (tLNGFR) sequence, a truncated version of the epithelial growth factor receptor (tEGFR), a GFP coding sequence, or any combination thereof. In some embodiments, the vector further comprises a tEGFR coding sequence of SEQ ID NO: 63 or a variant having at least about 75%, 80%, 85%, 86%, 87%, 88%, 89%, 90%, 91%, 92%, 93%, 94%, 95%, 96%, 97%, 98%, or 99% sequence identity thereto. In some embodiments, the vector comprises a tLNGFR coding sequence of SEQ ID NO: 64 or a variant having at least about 75%, 80%, 85%, 86%, 87%, 88%, 89%, 90%, 91%, 92%, 93%, 94%, 95%, 96%, 97%, 98%, or 99% sequence identity thereto. In some embodiments, the vector further comprises an MND promoter of SEQ ID NO: 63 or a variant having at least about 75%, 80%, 85%, 86%, 87%, 88%, 89%, 90%, 91%, 92%, 93%, 94%, 95%, 96%, 97%, 98%, or 99% sequence identity thereto. In some embodiments, the vector further comprises an MSCV promoter of SEQ ID NO: 64 or a variant having at least about 75%, 80%, 85%, 86%, 87%, 88%, 89%, 90%, 91%, 92%, 93%, 94%, 95%, 96%, 97%, 98%, or 99% sequence identity thereto.

In some aspects, the present disclosure provides for a method of editing two or more loci within a cell, comprising contacting or introducing to said cell: (a) a class 2, type II Cas endonuclease complex comprising or a polynucleotide encoding: (i) a class 2, type II Cas endonuclease; and (ii) one or more engineered guide RNAs comprising: an RNA sequence configured to bind to the class 2, type II Cas endonuclease, and a spacer sequence configured to hybridize to a first set of one or more target loci. In some embodiments the method further comprises contacting or introducing to said cell: (b) a class 2, type V Cas endonuclease complex comprising: (i) a class 2, type V Cas endonuclease; and (ii) one or more engineered guide RNAs comprising: an RNA sequence configured to bind to the class 2, type V Cas endonuclease, and a spacer sequence configured to hybridize to a second set of one or more target loci. In some embodiments, said class 2, type II Cas endonuclease is not a Cas9 endonuclease. In some embodiments, class 2, type II Cas endonuclease is a Cas12a endonuclease. In some embodiments, said class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof. In some embodiments, said class 2, type V Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to SEQ ID NO: 7 or a variant thereof. In some embodiments, said first engineered guide RNA or said second engineered guide RNA comprises a sequence having at least 80%, 85%, 90%, or 95% to any one of SEQ ID NOs: 3, 6, or 9, or a complement thereof. In some embodiments, said method edits genomic sequences of said first locus with at least about 25%, 30%, 35%, 40%, 45%, 50%, 55%, 60%, 65%, 70%, 75%, 80%, 85%, 90%, or more efficiency and/or said second locus with at least about 25%, 30%, 35%, 40%, 45%, 50%, 55%, 60%, 65%, 70%, 75%, 80%, 85%, 90%, or more efficiency. In some embodiments, said first RNA-guided endonuclease or said second RNA-guided endonuclease is introduced at a concentration of 200 pmol or less, 100 pmol or less, 50 pmol or less, 25 pmol or less, 5 pmol or less, or 1 pmol or less. In some embodiments, off-target sites within said cell are disrupted at a frequency of less than 0.2% as determined by a genome-wide off-target double-strand break analysis. In some embodiments, off-target sites within said cell are disrupted at a frequency of less than 0.01% as determined by a genome-wide off-target double-strand break. In some embodiments, the genome-wide off-target double-strand break analysis comprises an HTGTS assay (high-throughput, genome-wide translocation sequencing; see e.g. Chiarle et al. Cell. 2011 Sep 30;147(1):107-19. doi: 10.1016/j.cell.2011.07.049, which is explicitly incorporated by reference herein for all purposes), a LAM-HTGTS assay (linear amplification mediated high-throughput genome-wide sequencing; see e.g. Hu et al. Nat Protoc. 2016. 11(5):853-71. doi:10.1038/nprot.2016.043, which is explicitly incorporated by reference herein for all purposes), or a Digenome-Seq (in vitro Cas-digested whole genome sequencing; see e.g. Kim et al. Nat Methods. 2015. 12(3):237-43. doi:10.1038/nmeth.3284, which is explicitly incorporated by reference herein for all purposes) assay. In some embodiments, said first set of one or more target loci or said second set of one or more target loci comprises a T-cell receptor (TCR) locus. In some embodiments, said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 10-15, or a complement thereof. In some embodiments, said first set of one or more target loci or said second set of one or more target loci comprises a Nuclear Receptor Subfamily 3 Group C Member 1 (NR3C1) locus. In some embodiments, said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 16, 20, 21, or 22, or a complement thereof. In some embodiments, the method further comprises introducing to said cell a donor DNA sequence comprising an open reading frame encoding a heterologous engineered T-cell receptor molecule, a first homology arm comprising a DNA sequence located on a first side of said first set of one or more target loci and a second homology arm comprising a DNA sequence located on a second side of said first set of one or more target loci. In some embodiments, editing comprises insertion of an indel, a premature termination codon, a missense codon, a frameshift mutation, an adenine deamination, a cytosine deamination, or any combination thereof.

In some aspects, the present disclosure provides for a method of making a glucocorticoid-resistant engineered T cell, comprising introducing to a T-cell or a precursor thereof: (a) an RNA guided endonuclease complex targeting a T-cell receptor (TCR) locus, comprising or a polynucleotide encoding:(i) a first RNA guided endonuclease or DNA encoding said first RNA guided endonuclease; and (ii) a first engineered guide RNA comprising an RNA sequence configured to form a complex with said first RNA guided endonuclease, and a first spacer sequence configured to hybridize to at least part of said TCR locus; and (b) an RNA guided endonuclease complex targeting a T-cell receptor Nuclear Receptor Subfamily 3 Group C Member 1 (NR3C1) locus, comprising or a polynucleotide encoding: (i) a second RNA guided endonuclease; and (ii) a second engineered guide RNA comprising: an RNA sequence configured to form a complex with said second RNA guided endonuclease, and a second spacer sequence configured to hybridize to at least part of said NR3C1 locus. In some embodiments, said at least part of said TCR locus is within said T-cell locus. In some embodiments, the method further comprises introducing to said cell (b) a donor DNA sequence comprising an open reading frame encoding a heterologous engineered T-cell receptor molecule, a first homology arm comprising a DNA sequence located on a first side of said target sequence and a second homology arm comprising a DNA sequence located on a second side of said target sequence within said TCR locus. In some embodiments, said first RNA guided endonuclease or said second RNA guided endonuclease comprises a class 2, type II or a class 2, type V Cas endonuclease. In some embodiments, said first RNA guided endonuclease comprises said class 2, type II Cas endonuclease and said second RNA guided endonuclease comprises said class 2, type V Cas endonuclease. In some embodiments, said second RNA guided endonuclease comprises said class 2, type II Cas endonuclease and said first RNA guided endonuclease comprises said class 2, type V Cas endonuclease. In some embodiments, said heterologous engineered T-cell receptor is a CAR molecule. In some embodiments, said at least part of said T cell receptor locus is a T Cell Receptor Alpha Constant (TRAC) locus or a T Cell Receptor Beta Constant (TRBC) locus. In some embodiments, said homology arms comprise intronic or exonic regions within said TCR locus proximal to said at least part of said T cell receptor locus. In some embodiments, said at least part of said T cell receptor locus is a first or third exon of TRAC. In some embodiments, said method disrupts genomic sequences of said TCR locus and said NR3C1 locus with at least about 25%, 30%, 35%, 40%, 45%, 50%, 55%, 60%, 65%, 70%, 75%, 80%, 85%, 90%, or more efficiency. In some embodiments, said efficiency is determined by flow cytometry for a protein expressed from said TCR and NR3C1 loci. In some embodiments, said at least part of said NR3C1 locus is exon 2 or exon 3. In some embodiments, said method produces cells positive for the CAR molecule and negative for NR3C1 with at least about 25%, 30%, 35%, 40%, 45%, 50%, 55%, 60%, 65%, 70%, 75%, 80%, 85%, 90%, or more efficiency. In some embodiments, the method comprises introducing (a)-(c) to said T-cell or precursor thereof simultaneously. In some embodiments, said first RNA-guided endonuclease or said second RNA-guided endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1, 4, or 7. In some embodiments, said first engineered guide RNA or said second engineered guide RNA comprises a sequence having at least 80%, 85%, 90%, or 95% sequence identity to any one of SEQ ID NOs: 3, 6, or 9, or a complement thereof. In some embodiments, said first RNA-guided endonuclease or said second RNA-guided endonuclease is present at a concentration of 100 pmol or less, 50 pmol or less, 25 pmol or less, 5 pmol or less, or 1 pmol or less. In some embodiments, said T-cell or said precursor thereof comprises a T-cell, a hematopoietic stem cell (HSC), or peripheral blood mononuclear cell (PBMC). In some embodiments, said second spacer sequence comprises a sequence having at least 80%, 85%, 90%, or 95% sequence identity to any one of SEQ ID NOs: 16, 20, 21, or 22, or a complement thereof. In some embodiments, said first or said second spacer sequence comprises at least about 19-24 nucleotides, at least about 19 nucleotides, at least about 20 nucleotides, at least about 22 nucleotides, or at least about 24 nucleotides. In some embodiments, said donor DNA sequence is delivered in a viral vector. In some embodiments, said viral vector is an AAV or AAV-6 vector.

In some aspects, the present disclosure provides for a population of T cells, comprising: (a) an heterologous sequence within 100, 75, 50, 25, or 10 nucleotides of a hybridization region of any one of SEQ ID NOs: 10-15 within a TCR locus or an heterologous sequence within 100, 75, 50, 25, or 10 nucleotides of a hybridization region of SEQ ID NO: 42. In some embodiments, the population of T-cells further comprises (b) an NR3C1 locus comprising an indel. In some embodiments, said indel in said NR3C1 locus confers glucocorticoid-resistance on said T-cells. an heterologous sequence within 100, 75, 50, 25, or 10 nucleotides of a hybridization region of said heterologous sequence is an indel. In some embodiments, said heterologous sequence comprises an open reading frame comprising a nucleotide sequence encoding a heterologous T-cell receptor or a CAR molecule. In some embodiments, said NR3C1 locus comprises an indel within 100, 75, 50, 25, or 10 nucleotides of a hybridization region of any one of SEQ ID NOs: 16, 20, 21, or 22. In some embodiments, less than 0.2% have indels at off-target loci as determined by a genome-wide off-target double-strand break analysis. In some embodiments, less than 0.01% have indels at off-target loci as determined by a genome-wide off-target double-strand break analysis. In some embodiments, said population of cells is substantially free of chromosomal translocations.

In some aspects, the present disclosure provides for a method of editing two or more loci within a cell, comprising contacting to said cell: (a) a first Cas endonuclease complex comprising or a polynucleotide encoding: (i) a first Cas endonuclease; and (ii) one or more engineered guide RNAs comprising: an RNA sequence configured to bind to the class 2, type II Cas endonuclease, and a spacer sequence configured to hybridize to a first target sequence; (b) a second Cas endonuclease complex comprising: (i) a second Cas endonuclease; and (ii) one or more engineered guide RNAs comprising: an RNA sequence configured to bind to the class 2, type II Cas endonuclease, and a spacer sequence configured to hybridize to a second target sequence. In some embodiments, the method further comprises introducing to said cell (c) a first donor DNA sequence comprising an open reading frame encoding a first transgene, a 5' homology arm comprising a DNA sequence located on a 5' side of said first target sequence and a 3' homology arm comprising a DNA sequence located on a 3' side of said first target sequence; and (d) a second donor DNA sequence comprising an open reading frame encoding a second transgene, a 5' homology arm comprising a DNA sequence located on a 5' side of said second target sequence and a 3' homology arm comprising a DNA sequence located on a 3' side of said second target sequence. In some embodiments, said second transgene are different. In some embodiments, said first target sequence or said second target sequence is a target sequence within a T-cell receptor locus, TRAC, TRBC, NR3C1, or AAVS1 locus, or any combination thereof. In some embodiments, said first or second transgene is an alpha, beta, alpha-D3, or beta-D3 isoform of GR, a CAR molecule, or any combination thereof. In some embodiments, said 5' homology arm comprising a DNA sequence located on a 5' side of said first target sequence or said 5' homology arm comprising a DNA sequence located on a 5' side of said second target sequence comprises SEQ ID NOs: 42 or 23. In some embodiments, said 3' homology arm comprising a DNA sequence located on a 5' side of said first target sequence or said 3' homology arm comprising a DNA sequence located on a 5' side of said second target sequence comprises SEQ ID NOs: 43 or 24. In some embodiments, said first or said second class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof. In some embodiments, said first engineered guide RNA or said second engineered guide RNA comprises a sequence having at least 80%, 85%, 90%, or 95% sequence identity to any one of SEQ ID NOs: 3, 6, or 9, or a complement thereof. In some embodiments, said spacer sequence configured to hybridize to said first target sequence or said spacer sequence configured to hybridize to said second target sequence has at least 80%, 85%, 90%, or 95% sequence identity to any one of SEQ ID NOs: 16, 20, 21, 22, or 41, or a complement thereof. In some embodiments, said first or said second endonuclease comprises a class 2, type II Cas endonuclease or a class 2, type V Cas endonuclease, or any combination thereof.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** depicts a scheme for producing an allogeneic CAR-T cell using Cas endonucleases described herein in combination with AAV vectors delivering CAR-T donor sequences.
**FIG. 2** depicts results of the experiment in Example 1 testing indel formation in TRAC by MG3-6, MG3-8, and MG29-1 RNPs containing guide RNAs targeting TRAC alongside a Cas9 control. The left panel indicates % formation of indels as measured by next generation sequencing (NGS), while the right panel indicates cell phenotype (TCR+ or TCR-) assessed by flow cytometry
**FIG. 3** depicts results of the experiment in Example 1 testing targeted CAR-T integration using RNP nuclease complexes described herein targeting TRAC in combination with an AAV donor vector containing a CAR-T sequence. Shown are flow cytometry plots showing TCR expression status (TCR- or TCR+, x-axis) alongside expression of the CAR antigen binding domain (y-axis). Similar results were obtained for all of MG3-6, MG3-8, and MG29-1.
**FIG. 4** depicts multiplex editing of two loci (one being TRAC) using a combination of MG3-6 and MG29-1 RNP complexes as described in Example 2.
**FIG. 5** depicts multiplex editing of three loci (one being TRAC) using a combination of MG3-6 and MG29-1 RNP complexes as described in Example 2.

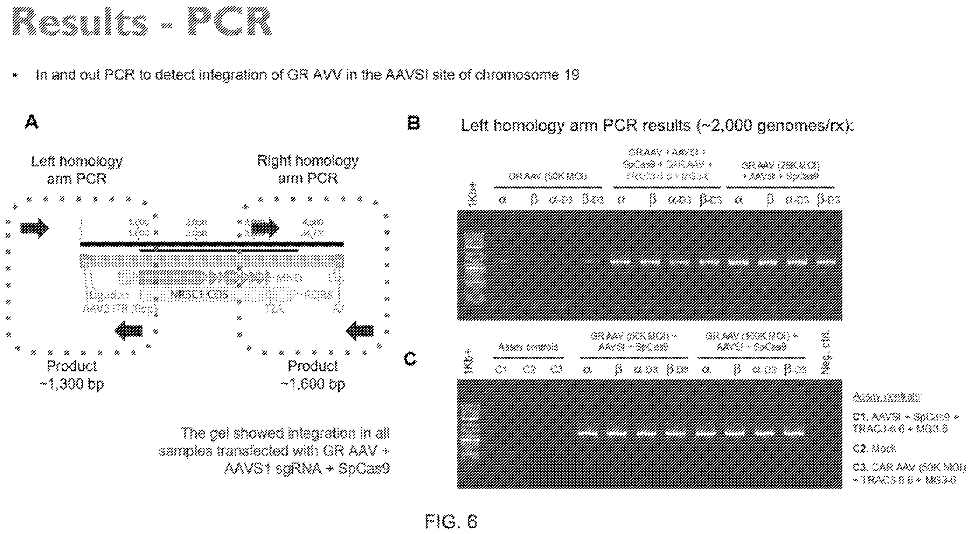

**FIG. 6** shows a design of a PCR experiment as in Example 3 to test integration of a GR transgene into the AAVS1 locus (A) or agarose gel photographs (B and C) depicting the results of experiments where AAVS1 and TRAC loci were simultaneously targeted using different Cas enzymes alongside exposure to separate donor DNAs targeting each site as in Example 3. (B) depicts amplification of GR transgenes from either conditions where T-cells were exposed to GR transgene-bearing AAV constructs (lanes 2-5), GR transgene AAV constructs/SpCas9 targeting AAVS1/MG3-6-targeting TRAC/CAR transgene AAV (lanes 6-9) construct, or GR AAV constructs/SpCas9 targeting AAVS1 alone at 25K multiplicity of infection (MOI) (lanes 10-13). (C) depicts amplification of GR transgenes from either conditions where T-cells were exposed to assay controls (mock transfection or Cas complexes without transgene; lanes 2-4), GR AAV constructs at 50K MOI/SpCas9 targeting AAVS1 alone (lanes 5-8), or GR AAV constructs at 100K MOI/SpCas9 targeting AAVS1 alone at 50K multiplicity of infection (MOI) (lanes 9-12). Results indicate GR transgenes integrated into the AAVS1 locus at similar efficiencies whether or not the additional TRAC locus was targeted.

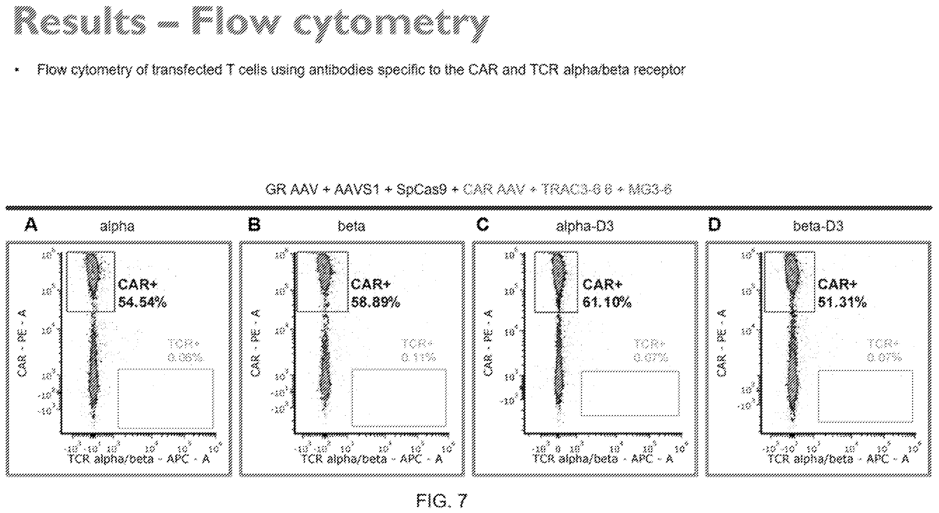

**FIG. 7** depicts flow cytometry plots depicting the results of experiments as in Example 3 where AAVS1 and TRAC loci were simultaneously targeted using different Cas enzymes alongside exposure to separate donor DNAs targeting each site as in Example 3. Shown are individual plots (A-D) where AAVs bearing each GR transgene were introduced to T-cells alongside AAVS1-targeting SpCas9 complex, TRAC-targeting MG3-6 complex, and a CAR-bearing AAV. Results indicate TCR knockout and CAR integration was similarly efficient with all GR transgenes, and that it was high (51.31%-61.1% efficiency) despite simultaneous targeting of the AAVS1 locus.
**FIG. 8** depicts results of a genome-wide off-target double-strand break analysis assay performed to assess off-target specificity of MG3-6, MG3-8, and MG29-1 endonucleases alongside SpCas9 ("Cas9") as in Example 4.
**FIG. 9** is a depiction of the assembly of delta, gamma and epsilon chains making an active full TCR.
**FIG. 10** shows multiplex TRAC/TRBC editing in primary T cells as described in Example 5, as assessed by percentage of sequences at the targeted loci containing indels. The results indicate high frequency disruption at both sites when both sites are simultaneously targeted.

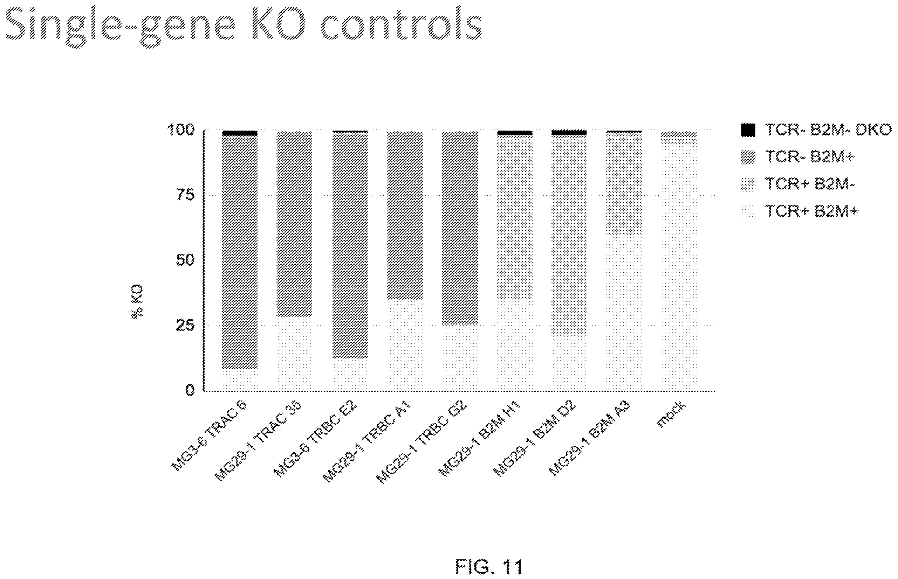

**FIG. 11** depicts the gene editing outcomes by flow cytometry for the single-gene knock-out experiment described in Example 6. Shown is a bar graph illustrating percentage of analyzed cells containing each of 4 phenotypes assessing knockout of TCR and B2M (TCR- B2M- DKO, TCR- B2M+, TCR+ B2M-, and TCR+ B2M+). The graph illustrates that: (a) all of the TCR targeting conditions efficiently produced TCR knockout, with the MG3-6 TRAC6 and MG3-6 TRBC E2 sgRNAs producing the most efficient TCR knockout; and (b) all of the B2M targeting conditions produced B2M knockout, with B2M H1 and B2M D2 producing the most efficient B2M knockout.

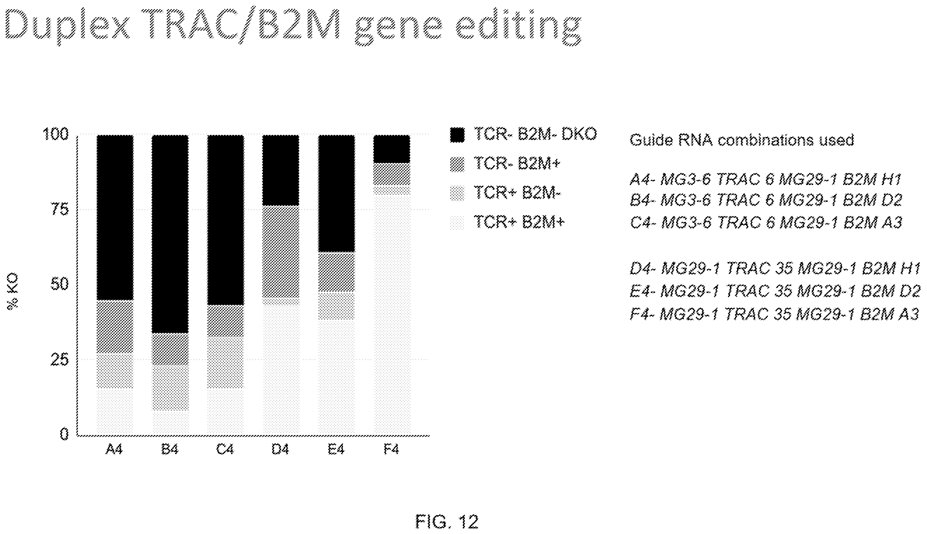

**FIG. 12** depicts the gene editing outcomes by flow cytometry for the double-gene knock-out experiment described in Example 7, which uses the B2M and TRAC conditions in Example 6 but in combination. Shown is a bar graph illustrating percentage of analyzed cells containing each of 4 phenotypes assessing knockout of TCR and B2M (TCR- B2M- DKO, TCR- B2M+, TCR+ B2M-, and TCR+ B2M+). The graph illustrates that the most efficient dual targeting conditions were A4, B4, and C4, involving the MG3-6 TRAC6 condition with the MG29-1 B2M H1, D2, or A3 condition. The most efficient dual targeting condition appeared to be B4, which used the MG3-6 TRAC6 sgRNA and the MG29-1 B2M D2 sgRNA.
**FIG. 13** depicts the gene editing outcomes by flow cytometry for the triple-gene knock-out experiment described in Example 8, which uses the B2M, TRAC, and TRBC conditions from Example 6 but in combination.
**FIG. 14** depicts the gene editing outcomes at the DNA level for the triple-gene knock-out experiment described in Example 8, which uses the B2M, TRAC, and TRBC conditions from Example 6 but in combination.
**FIG. 15** depicts analysis of gene editing outcomes determined by next-generation sequencing (NGS) for the triple-gene knock-out experiment described in Example 8.
**FIG. 16** depicts gene-editing outcomes at the protein level in T cells for the experiments described in Example 9. Shown are bar graphs indicating percentage (%) of T-cells positive for GFP/tEGFR, tLNGFR, double targeted integration (GFP/tLNGFR), double targeted integration (tEGFR/tLNGFR), or TCR, as determined by fluorescent-activated cell sorting (FACS), using the combinations of nucleases, guides, and AAVs described in Example 9.
**FIG. 17** depicts the gene-editing outcomes at the DNA level in T cells for the AAVS1 site and the TRAC locus for the experiment described in Example 10. Shown is a bar graph of the percentage of sequences detected by next-generation sequencing (Illumina MiSeq) with at least one indel (% indels) detected at the AAVS1 locus using the conditions described in Example 10.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The practice of some methods disclosed herein employ, unless otherwise indicated, techniques of immunology, biochemistry, chemistry, molecular biology, microbiology, cell biology, genomics, and recombinant DNA. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)) (which is entirely incorporated by reference herein).

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, i.e., the limitations of the measurement system. For example, "about" can mean within one or more than one standard deviation, per the practice in the art. Alternatively, "about" can mean a range of up to 20%, up to 15%, up to 10%, up to 5%, or up to 1% of a given value.

As used herein, a "cell" generally refers to a biological cell. A cell may be the basic structural, functional and/or biological unit of a living organism. A cell may originate from any organism having one or more cells. Some non-limiting examples include: a prokaryotic cell, eukaryotic cell, a bacterial cell, an archaeal cell, a cell of a single-cell eukaryotic organism, a protozoa cell, a cell from a plant (e.g., cells from plant crops, fruits, vegetables, grains, soy bean, corn, maize, wheat, seeds, tomatoes, rice, cassava, sugarcane, pumpkin, hay, potatoes, cotton, cannabis, tobacco, flowering plants, conifers, gymnosperms, ferns, clubmosses, hornworts, liverworts, mosses), an algal cell, (e.g., *Botryococcus braunii, Chlamydomonas reinhardtii, Nannochloropsis gaditana, Chlorella pyrenoidosa, Sargassum patens C. Agardh,* and the like), seaweeds (e.g., kelp), a fungal cell (e.g.,, a yeast cell, a cell from a mushroom), an animal cell, a cell from an invertebrate animal (e.g., fruit fly, cnidarian, echinoderm, nematode, etc.), a cell from a vertebrate animal (e.g., fish, amphibian, reptile, bird, mammal), a cell from a mammal (e.g., a pig, a cow, a goat, a sheep, a rodent, a rat, a mouse, a non-human primate, a human, etc.), and etcetera. Sometimes a cell is not originating from a natural organism (e.g., a cell can be a synthetically made, sometimes termed an artificial cell).

The term "nucleotide," as used herein, generally refers to a base-sugar-phosphate combination. A nucleotide may comprise a synthetic nucleotide. Nucleotides may be monomeric units of a nucleic acid sequence (e.g., deoxyribonucleic acid (DNA) and ribonucleic acid (RNA)). The term nucleotide may include ribonucleoside triphosphates adenosine triphosphate (ATP), uridine triphosphate (UTP), cytosine triphosphate (CTP), guanosine triphosphate (GTP) and deoxyribonucleoside triphosphates such as dATP, dCTP, dITP, dUTP, dGTP, dTTP, or derivatives thereof. Such derivatives may include, for example, [αS]dATP, 7-deaza-dGTP and 7-deaza-dATP, and nucleotide derivatives that confer nuclease resistance on the nucleic acid molecule containing them. The term nucleotide as used herein may refer to dideoxyribonucleoside triphosphates (ddNTPs) and their derivatives. Illustrative examples of dideoxyribonucleoside triphosphates may include, but are not limited to, ddATP, ddCTP, ddGTP, ddITP, and ddTTP. A nucleotide may be unlabeled or detectably labeled, such as using moieties comprising optically detectable moieties (e.g., fluorophores). Labeling may also be carried out with quantum dots. Detectable labels may include, for example, radioactive isotopes, fluorescent labels, chemiluminescent labels, bioluminescent labels, and enzyme labels. Fluorescent labels of nucleotides may include but are not limited fluorescein, 5-carboxyfluorescein (FAM), 2'7'-dimethoxy-4'5-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'dimethylaminophenylazo) benzoic acid (DABCYL), Cascade Blue, Oregon Green, Texas Red, Cyanine and 5-(2'-aminoethyl)aminonaphthalene-1-sulfonic acid (EDANS). Specific examples of fluorescently labeled nucleotides can include [R6G]dUTP, [TAMRA]dUTP, [R110]dCTP, [R6G]dCTP, [TAMRA]dCTP, [JOE]ddATP, [R6G]ddATP, [FAM]ddCTP, [R110]ddCTP, [TAMRA]ddGTP, [ROX]ddTTP, [dR6G]ddATP, [dR110]ddCTP, [dTAMRA]ddGTP, and [dROX]ddTTP available from Perkin Elmer, Foster City, Calif; FluoroLink DeoxyNucleotides, FluoroLink Cy3-dCTP, FluoroLink Cy5-dCTP, FluoroLink Fluor X-dCTP, FluoroLink Cy3-dUTP, and FluoroLink Cy5-dUTP available from Amersham, Arlington Heights, Il.; Fluorescein-15-dATP, Fluorescein-12-dUTP, Tetramethyl-rodamine-6-dUTP, IR770-9-dATP, Fluorescein-12-ddUTP, Fluorescein-12-UTP, and Fluorescein-15-2'-dATP available from Boehringer Mannheim, Indianapolis, Ind.; and Chromosome Labeled Nucleotides, BODIPY-FL-14-UTP, BODIPY-FL-4-UTP, BODIPY-TMR-14-UTP, BODIPY-TMR-14-dUTP, BODIPY-TR-14-UTP, BODIPY-TR-14-dUTP, Cascade Blue-7-UTP, Cascade Blue-7-dUTP, fluorescein-12-UTP, fluorescein-12-dUTP, Oregon Green 488-5-dUTP, Rhodamine Green-5-UTP, Rhodamine Green-5-dUTP, tetramethylrhodamine-6-UTP, tetramethylrhodamine-6-dUTP, Texas Red-5-UTP, Texas Red-5-dUTP, and Texas Red-12-dUTP available from Molecular Probes, Eugene, Oreg. Nucleotides can also be labeled or marked by chemical modification. A chemically-modified single nucleotide can be biotin-dNTP. Some non-limiting examples of biotinylated dNTPs can include, biotin-dATP (e.g., bio-N6-ddATP, biotin-14-dATP), biotin-dCTP (e.g., biotin-11-dCTP, biotin-14-dCTP), and biotin-dUTP (e.g., biotin-11-dUTP, biotin-16-dUTP, biotin-20-dUTP).A nucleotide may comprise a nucleotide analog. In some embodiments, nucleotide analogs may comprise structures of natural nucleotides that are modified at any position so as to alter certain chemical properties of the nucleotide yet retain the ability of the nucleotide analog to perform its intended function (e.g. hybridization to other nucleotides in RNA or DNA). Examples of positions of the nucleotide which may be derivatized include the 5 position, e.g., 5-(2-amino)propyl uridine, 5-bromo uridine, 5-propyne uridine, 5-propenyl uridine, etc.; the 6 position, e.g., 6-(2-amino)propyl uridine; the 8-position for adenosine and/or guanosines, e.g., 8-bromo guanosine, 8-chloro guanosine, 8-fluoroguanosine, etc. Nucleotide analogs also include deaza nucleotides, e.g., 7-deaza-adenosine: O- and N-modified (e.g., alkylated, e.g., N6-methyl adenosine, or as otherwise suitably modified) nucleotides; and other heterocyclically modified nucleotide analogs such as those described in Herdewijn, Antisense Nucleic Acid Drug Dev., 2000 Aug. 10(4):297-310. Nucleotide analogs may also comprise modifications to the sugar portion of the nucleotides. For example the 2' OH-group may be replaced by a group selected from H, OR, R, F, Cl, Br, I, SH, SR, NH2, NHR, NR2, COOR, or OR, wherein R is substituted or unsubstituted C1-C6 alkyl, alkenyl, alkynyl, aryl, etc. Other possible modifications include those described in U.S. Pat. Nos. 5,858,988, and 6,291,438. Examples of positions of the nucleotide which may be derivatized include the 5 position, e.g., 5-(2-amino)propyl uridine, 5-bromo uridine, 5-propyne uridine, 5-propenyl uridine, etc.; the 6 position, e.g., 6-(2-amino)propyl uridine; the 8-position for adenosine and/or guanosines, e.g., 8-bromo guanosine, 8-chloro guanosine, 8-fluoroguanosine, etc. Nucleotide analogs also include deaza nucleotides, e.g., 7-deaza-adenosine: O- and N-modified (e.g., alkylated, e.g., N6-methyl adenosine, or as otherwise suitably modified) nucleotides; and other heterocyclically modified nucleotide analogs such as those described in Herdewijn, Antisense Nucleic Acid Drug Dev., 2000 Aug. 10(4):297-310.
Nucleotide analogs may also comprise modifications to the sugar portion of the nucleotides. For example the 2' OH-group may be replaced by a group selected from H, OR, R, F, Cl, Br, I, SH, SR, NH2, NHR, NR2, COOR, or OR, wherein R is substituted or unsubstituted C1-C6 alkyl, alkenyl, alkynyl, aryl, etc. Other possible modifications include those described in U.S. Pat. Nos. 5,858,988, and 6,291,438.

The terms "polynucleotide," "oligonucleotide," and "nucleic acid" are used interchangeably to generally refer to a polymeric form of nucleotides of any length, either deoxyribonucleotides or ribonucleotides, or analogs thereof, either in single-, double-, or multi-stranded form. A polynucleotide may be exogenous or endogenous to a cell. A polynucleotide may exist in a cell-free environment. A polynucleotide may be a gene or fragment thereof. A polynucleotide may be DNA. A polynucleotide may be RNA. A polynucleotide may have any three-dimensional structure and may perform any function. A polynucleotide may comprise one or more analogs (e.g., altered backbone, sugar, or nucleobase). If present, modifications to the nucleotide structure may be imparted before or after assembly of the polymer. Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol-containing nucleotides, biotin-linked nucleotides, fluorescent base analogs, CpG islands, methyl-7-guanosine, methylated nucleotides, inosine, thiouridine, pseudouridine, dihydrouridine, queuosine, and wyosine. Non-limiting examples of polynucleotides include coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, cell-free polynucleotides including cell-free DNA (cfDNA) and cell-free RNA (cfRNA), nucleic acid probes, and primers. The sequence of nucleotides may be interrupted by non-nucleotide components. A polynucleotide may comprise a mixture of nucleotides found in nature and nucleotide analogs (e.g. synthetic nucleotide analogs).

The terms "transfection" or "transfected" generally refer to introduction of a nucleic acid into a cell by non-viral or viral-based methods. The nucleic acid molecules may be gene sequences encoding complete proteins or functional portions thereof. See, e.g., Sambrook et al., 1989, Molecular Cloning: A Laboratory Manual, 18.1-18.88 (which is entirely incorporated by reference herein).

The terms "peptide," "polypeptide," and "protein" are used interchangeably herein to generally refer to a polymer of at least two amino acid residues joined by peptide bond(s). This term does not connote a specific length of polymer, nor is it intended to imply or distinguish whether the peptide is produced using recombinant techniques, chemical or enzymatic synthesis, or is naturally occurring. The terms apply to naturally occurring amino acid polymers as well as amino acid polymers comprising at least one modified amino acid. In some cases, the polymer may be interrupted by non-amino acids. The terms include amino acid chains of any length, including full length proteins, and proteins with or without secondary and/or tertiary structure (e.g., domains). The terms also encompass an amino acid polymer that has been modified, for example, by disulfide bond formation, glycosylation, lipidation, acetylation, phosphorylation, oxidation, and any other manipulation such as conjugation with a labeling component. The terms "amino acid" and "amino acids," as used herein, generally refer to natural and non-natural amino acids, including, but not limited to, modified amino acids and amino acid analogues. Modified amino acids may include natural amino acids and non-natural amino acids, which have been chemically modified to include a group or a chemical moiety not naturally present on the amino acid. Amino acid analogues may refer to amino acid derivatives. The term "amino acid" includes both D-amino acids and L-amino acids.

As used herein, the "non-native" can generally refer to a nucleic acid or polypeptide sequence that is not found in a native nucleic acid or protein. Non-native may refer to affinity tags. Non-native may refer to fusions. Non-native may refer to a naturally occurring nucleic acid or polypeptide sequence that comprises mutations, insertions and/or deletions. A non-native sequence may exhibit and/or encode for an activity (e.g., enzymatic activity, methyltransferase activity, acetyltransferase activity, kinase activity, ubiquitinating activity, etc.) that may also be exhibited by the nucleic acid and/or polypeptide sequence to which the non-native sequence is fused. A non-native nucleic acid or polypeptide sequence may be linked to a naturally-occurring nucleic acid or polypeptide sequence (or a variant thereof) by genetic engineering to generate a chimeric nucleic acid and/or polypeptide sequence encoding a chimeric nucleic acid and/or polypeptide.

The term "promoter", as used herein, generally refers to the regulatory DNA region which controls transcription or expression of a gene and which may be located adjacent to or overlapping a nucleotide or region of nucleotides at which RNA transcription is initiated. A promoter may contain specific DNA sequences which bind protein factors, often referred to as transcription factors, which facilitate binding of RNA polymerase to the DNA leading to gene transcription. A 'basal promoter', also referred to as a 'core promoter', may generally refer to a promoter that contains all the basic elements to promote transcriptional expression of an operably linked polynucleotide. Eukaryotic basal promoters typically, though not necessarily, contain a TATA-box and/or a CAAT box.

The term "expression", as used herein, generally refers to the process by which a nucleic acid sequence or a polynucleotide is transcribed from a DNA template (such as into mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

As used herein, "operably linked", "operable linkage", "operatively linked", or grammatical equivalents thereof generally refer to juxtaposition of genetic elements, e.g., a promoter, an enhancer, a polyadenylation sequence, etc., wherein the elements are in a relationship permitting them to operate in the expected manner. For instance, a regulatory element, which may comprise promoter and/or enhancer sequences, is operatively linked to a coding region if the regulatory element helps initiate transcription of the coding sequence. There may be intervening residues between the regulatory element and coding region so long as this functional relationship is maintained.

A "vector" as used herein, generally refers to a macromolecule or association of macromolecules that comprises or associates with a polynucleotide and which may be used to mediate delivery of the polynucleotide to a cell. Examples of vectors include plasmids, viral vectors, liposomes, and other gene delivery vehicles. The vector generally comprises genetic elements, e.g., regulatory elements, operatively linked to a gene to facilitate expression of the gene in a target.

As used herein, "an expression cassette" and "a nucleic acid cassette" are used interchangeably generally to refer to a combination of nucleic acid sequences or elements that are expressed together or are operably linked for expression. In some cases, an expression cassette refers to the combination of regulatory elements and a gene or genes to which they are operably linked for expression.

As used herein, an "engineered" object generally indicates that the object has been modified by human intervention. According to non-limiting examples: a nucleic acid may be modified by changing its sequence to a sequence that does not occur in nature; a nucleic acid may be modified by ligating it to a nucleic acid that it does not associate with in nature such that the ligated product possesses a function not present in the original nucleic acid; an engineered nucleic acid may synthesized in vitro with a sequence that does not exist in nature; a protein may be modified by changing its amino acid sequence to a sequence that does not exist in nature; an engineered protein may acquire a new function or property. An "engineered" system comprises at least one engineered component.

As used herein, "synthetic" and "artificial" can generally be used interchangeably to refer to a protein or a domain thereof that has low sequence identity (e.g., less than 50% sequence identity, less than 25% sequence identity, less than 10% sequence identity, less than 5% sequence identity, less than 1% sequence identity) to a naturally occurring human protein. For example, VPR and VP64 domains are synthetic transactivation domains.

As used herein, the term "Cas12a" generally refers to a family of Cas endonucleases that are class 2, Type V-A Cas endonucleases and that (a) use a relatively small guide RNA (about 42-44 nucleotides) that is processed by the nuclease itself following transcription from the CRISPR array, and (b) cleave DNA to leave staggered cut sites. Further features of this family of enzymes can be found, e.g. in Zetsche B, Heidenreich M, Mohanraju P, et al. Nat Biotechnol 2017;35:31-34, and Zetsche B, Gootenberg JS, Abudayyeh OO, et al. Cell 2015;163:759-771, which are incorporated by reference herein.

As used herein, a "guide nucleic acid" or variants thereof can generally refer to a nucleic acid that may hybridize to another nucleic acid. A guide nucleic acid may be RNA. A guide nucleic acid may be DNA. The guide nucleic acid may be programmed to bind to a sequence of nucleic acid site-specifically. The nucleic acid to be targeted, or the target nucleic acid, may comprise nucleotides. The guide nucleic acid may comprise nucleotides. A portion of the target nucleic acid may be complementary to a portion of the guide nucleic acid. The strand of a double-stranded target polynucleotide that is complementary to and hybridizes with the guide nucleic acid may be called the complementary strand. The strand of the double-stranded target polynucleotide that is complementary to the complementary strand, and therefore may not be complementary to the guide nucleic acid may be called noncomplementary strand. A guide nucleic acid may comprise a polynucleotide chain and can be called a "single guide nucleic acid." A guide nucleic acid may comprise two polynucleotide chains and may be called a "double guide nucleic acid." If not otherwise specified, the term "guide nucleic acid" may be inclusive, referring to both single guide nucleic acids and double guide nucleic acids. A guide nucleic acid may comprise a segment that can be referred to as a "nucleic acid-targeting segment" or a "nucleic acid-targeting sequence" or "spacer sequence." A nucleic acid-targeting segment may comprise a sub-segment that may be referred to as a "protein binding segment" or "protein binding sequence" or "Cas protein binding segment". A guide nucleic acid can comprise an sgRNA. A guide nucleic acid can comprise a crRNA.

The term "sequence identity" or "percent identity" in the context of two or more nucleic acids or polypeptide sequences, generally refers to two (e.g., in a pairwise alignment) or more (e.g., in a multiple sequence alignment) sequences that are the same or have a specified percentage of amino acid residues or nucleotides that are the same, when compared and aligned for maximum correspondence over a local or global comparison window, as measured using a sequence comparison algorithm. Suitable sequence comparison algorithms for polypeptide sequences include, e.g., BLASTP using parameters of a wordlength (W) of 3, an expectation (E) of 10, and the BLOSUM62 scoring matrix setting gap costs at existence of 11, extension of 1, and using a conditional compositional score matrix adjustment for polypeptide sequences longer than 30 residues; BLASTP using parameters of a wordlength (W) of 2, an expectation (E) of 1000000, and the PAM30 scoring matrix setting gap costs at 9 to open gaps and 1 to extend gaps for sequences of less than 30 residues (these are the default parameters for BLASTP in the BLAST suite available at https://blast.ncbi.nlm.nih.gov); CLUSTALW with the Smith-Waterman homology search algorithm parameters with a match of 2, a mismatch of -1, and a gap of -1; MUSCLE with default parameters; MAFFT with parameters of a retree of 2 and max iterations of 1000; Novafold with default parameters; HMMER hmmalign with default parameters.

As used herein, the terms "Chimeric Antigen Receptor", "CAR", or "CAR molecule" generally refer to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule as defined herein. In some embodiments, the stimulatory molecule is the zeta chain associated with the T cell receptor complex or the signaling domain of NKG2D. In some embodiments, the intracellular signaling domain further comprises one or more functional signaling domains derived from at least one costimulatory molecule as defined below. In some embodiments, the costimulatory molecule is chosen from 4-1BB (i.e., CD137), CD27, and/or CD28. In some embodiments, the CAR comprises a chimeric fusion protein comprising an extracellular antigen recognition domain, a transmembrane domain and a cytoplasmic signaling domain comprising a functional signaling domain derived from a stimulatory molecule. In some embodiments, the CAR comprises a chimeric fusion protein comprising an extracellular antigen recognition domain, a transmembrane domain and a cytoplasmic signaling domain comprising a functional signaling domain derived from a co-stimulatory molecule and a functional signaling domain derived from a stimulatory molecule. In some embodiments, the CAR comprises a chimeric fusion protein comprising an extracellular antigen recognition domain, a transmembrane domain and an intracellular signaling domain comprising two functional signaling domains derived from one or more co-stimulatory molecule(s) and a functional signaling domain derived from a stimulatory molecule. In some embodiments, the CAR comprises a chimeric fusion protein comprising an extracellular antigen recognition domain, a transmembrane domain and an intracellular signaling domain comprising at least two functional signaling domains derived from one or more co-stimulatory molecule(s) and a functional signaling domain derived from a stimulatory molecule. In some embodiments the CAR comprises an optional leader sequence at the amino-terminus (N-term) of the CAR fusion protein. In some embodiments, the CAR further comprises a leader sequence at the N-terminus of the extracellular antigen recognition domain, wherein the leader sequence is optionally cleaved from the antigen recognition domain, e.g., a scFv) during cellular processing and localization of the CAR to the cellular membrane.

The term "signaling domain" generally refers to the functional portion of a protein which acts by transmitting information within the cell to regulate cellular activity via defined signaling pathways by generating second messengers or functioning as effectors by responding to such messengers.

The term "antibody," as used herein, generally refers to a protein, or polypeptide sequence derived from an immunoglobulin molecule which specifically binds with an antigen, e.g., non-covalently, reversibly, and in a specific manner. An antibody can be polyclonal or monoclonal, multiple or single chain, or an intact immunoglobulin, and may be derived from natural sources or from recombinant sources. An antibody can be a tetramer of immunoglobulin molecule. For example, a naturally occurring IgG antibody is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hyper variability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. The term "antibody" includes, but is not limited to, monoclonal antibodies, human antibodies, humanized antibodies, camelid antibodies, and chimeric antibodies. The antibodies can be of any isotype/class (e.g., IgG, IgE, IgM, IgD, IgA and IgY), or subclass (e.g., IgG1, IgG2, IgG3, IgG4, IgA1 and IgA2).

The term "antibody fragment" refers to at least one portion of an intact antibody, or recombinant variants thereof, and refers to the antigen binding domain, e.g., an antigenic determining variable regions of an intact antibody that is sufficient to confer recognition and specific binding of the antibody fragment to a target, such as an antigen. Examples of antibody fragments include, but are not limited to, Fab, Fab', F(ab')2, and Fv fragments, single chain or "scFv" antibody fragments, linear antibodies, single domain antibodies such as sdAb (either VL or VH), camelid VHH domains, and multi-specific antibodies formed from antibody fragments. The term "scFv" refers to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of a CAR composition comprising an antibody or antibody fragment thereof may exist in a variety of forms where the antigen binding domain is expressed as part of a contiguous polypeptide chain including, for example, a single domain antibody fragment (sdAb), a single chain antibody (scFv) and a humanized antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, N.Y.; Houston et al., 1988, Proc. Natl. Acad. Sci. USA 85:5879-5883; Bird et al., 1988, Science 242:423-426). In some embodiments, the antigen binding domain of a CAR composition of the invention comprises an antibody fragment. In some embodiments, the CAR comprises an antibody fragment that comprises a scFv.

Included in the current disclosure are variants of any of the enzymes described herein with one or more conservative amino acid substitutions. Such conservative substitutions can be made in the amino acid sequence of a polypeptide without disrupting the three-dimensional structure or function of the polypeptide. Conservative substitutions can be accomplished by substituting amino acids with similar hydrophobicity, polarity, and R chain length for one another. Additionally, or alternatively, by comparing aligned sequences of homologous proteins from different species, conservative substitutions can be identified by locating amino acid residues that have been mutated between species (e.g., non-conserved residues) without altering the basic functions of the encoded proteins. Such conservatively substituted variants may include variants with at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of the endonuclease protein sequences described herein. In some embodiments, such conservatively substituted variants are functional variants. Such functional variants can encompass sequences with substitutions such that the activity of one or more critical active site residues or guide RNA binding residues of the endonuclease are not disrupted. In some embodiments, a functional variant of any of the proteins described herein lacks substitution of at least one of the conserved or functional residues characteristic of Cas endonucleases. In some embodiments, a functional variant of any of the proteins described herein lacks substitution of all of the conserved or functional residues characteristic of Cas endonucleases.

Also included in the current disclosure are variants of any of the enzymes described herein with substitution of one or more catalytic residues to decrease or eliminate activity of the enzyme (e.g. decreased-activity variants). In some embodiments, a decreased activity variant as a protein described herein comprises a disrupting substitution of at least one, at least two, or all three RuvC catalytic residues.

Conservative substitution tables providing functionally similar amino acids are available from a variety of references (see, for e.g., Creighton, Proteins: Structures and Molecular Properties (W H Freeman & Co.; 2nd edition (December 1993)). The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)

### Overview

The discovery of new Cas enzymes with unique functionality and structure may offer the potential to further disrupt deoxyribonucleic acid (DNA) editing technologies, improving speed, specificity, functionality, and ease of use. Relative to the predicted prevalence of Clustered Regularly Interspaced Short Palindromic Repeats (CRISPR) systems in microbes and the sheer diversity of microbial species, relatively few functionally characterized CRISPR/Cas enzymes exist in the literature. This is partly because a huge number of microbial species may not be readily cultivated in laboratory conditions. Metagenomic sequencing from natural environmental niches containing large numbers of microbial species may offer the potential to drastically increase the number of new documented CRISPR/Cas systems and speed the discovery of new oligonucleotide editing functionalities. A recent example of the fruitfulness of such an approach is demonstrated by the 2016 discovery of CasX/CasY CRISPR systems from metagenomic analysis of natural microbial communities.

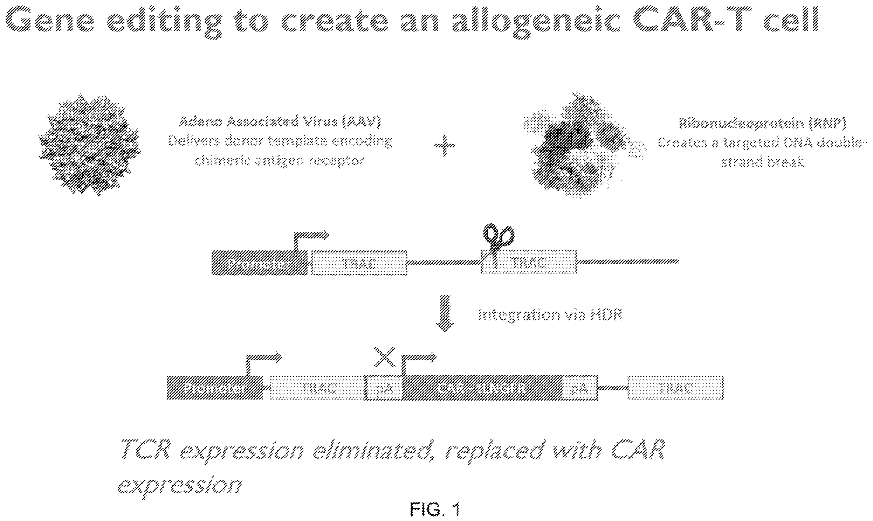

CRISPR/Cas systems are RNA-directed nuclease complexes that have been described to function as an adaptive immune system in microbes. In their natural context, CRISPR/Cas systems occur in CRISPR (clustered regularly interspaced short palindromic repeats) operons or loci, which generally comprise two parts: (i) an array of short repetitive sequences (30-40bp) separated by equally short spacer sequences, which encode the RNA-based targeting element; and (ii) ORFs encoding the Cas encoding the nuclease polypeptide directed by the RNA-based targeting element alongside accessory proteins/enzymes. Efficient nuclease targeting of a particular target nucleic acid sequence generally requires both (i) complementary hybridization between the first 6-8 nucleic acids of the target (the target seed) and the crRNA guide; and (ii) the presence of a protospacer-adjacent motif (PAM) sequence within a defined vicinity of the target seed (the PAM usually being a sequence not commonly represented within the host genome). Depending on the exact function and organization of the system, CRISPR-Cas systems are commonly organized into 2 classes, 5 types and 16 subtypes based on shared functional characteristics and evolutionary similarity (see **FIG. 1****).**

Class I CRISPR-Cas systems have large, multi-subunit effector complexes, and comprise Types I, III, and IV. Class II CRISPR-Cas systems generally have single-polypeptide multidomain nuclease effectors, and comprise Types II, V and VI.

Type II CRISPR-Cas systems are considered the simplest in terms of components. In Type II CRISPR-Cas systems, the processing of the CRISPR array into mature crRNAs does not require the presence of a special endonuclease subunit, but rather a small trans-encoded crRNA (tracrRNA) with a region complementary to the array repeat sequence; the tracrRNA interacts with both its corresponding effector nuclease (e.g. Cas9) and the repeat sequence to form a precursor dsRNA structure, which is cleaved by endogenous RNAse III to generate a mature effector enzyme loaded with both tracrRNA and crRNA. Cas II nucleases are identified as DNA nucleases. Type 2 effectors generally exhibit a structure comprising a RuvC-like endonuclease domain that adopts the RNase H fold with an unrelated HNH nuclease domain inserted within the folds of the RuvC-like nuclease domain. The RuvC-like domain is responsible for the cleavage of the target (e.g., crRNA complementary) DNA strand, while the HNH domain is responsible for cleavage of the displaced DNA strand.

Type V CRISPR-Cas systems are characterized by a nuclease effector (e.g. Cas12) structure similar to that of Type II effectors, comprising a RuvC-like domain. Similar to Type II, most (but not all) Type V CRISPR systems use a tracrRNA to process pre-crRNAs into mature crRNAs; however, unlike Type II systems which requires RNAse III to cleave the pre-crRNA into multiple crRNAs, type V systems are capable of using the effector nuclease itself to cleave pre-crRNAs. Like Type-II CRISPR-Cas systems, Type V CRISPR-Cas systems are again identified as DNA nucleases. Unlike Type II CRISPR-Cas systems, some Type V enzymes (e.g., Cas12a) appear to have a robust single-stranded nonspecific deoxyribonuclease activity that is activated by the first crRNA directed cleavage of a double-stranded target sequence.

CRISPR-Cas systems have emerged in recent years as the gene editing technology of choice due to their targetability and ease of use. The most commonly used systems are the Class 2 Type II SpCas9 and the Class 2 Type V-A Cas12a (previously Cpf1). The Type V-A systems in particular are becoming more widely used since their reported specificity in cells is higher than other nucleases, with fewer or no off-target effects. The V-A systems are also advantageous in that the guide RNA is small (42-44 nucleotides compared with approximately 100 nt for SpCas9) and is processed by the nuclease itself following transcription from the CRISPR array, simplifying multiplexed applications with multiple gene edits. Furthermore, the V-A systems have staggered cut sites, which may facilitate directed repair pathways, such as microhomology-dependent targeted integration (MITI).

The most commonly used Type V-A enzymes require a 5' protospacer adjacent motif (PAM) next to the chosen target site: 5'-TTTV-3' for *Lachnospiraceae* bacterium ND2006 LbCas12a and *Acidaminococcus sp.* AsCas12a; and 5'-TTV-3' for *Francisella novicida* FnCas12a. Recent exploration of orthologs has revealed proteins with less restrictive PAM sequences that are also active in mammalian cell culture, for example YTV, YYN or TTN. However, these enzymes do not fully encompass V-A biodiversity and targetability, and may not represent all possible activities and PAM sequence requirements. Here, thousands of genomic fragments were mined from numerous metagenomes for Type V-A nucleases. The documented diversity of V-A enzymes may have been expanded and novel systems may have been developed into highly targetable, compact, and precise gene editing agents.

### Example embodiments

In some aspects, the present disclosure provides for a method of editing two or more loci within a cell, comprising contacting to, or introducing to, said cell: (a) a class 2, type II Cas endonuclease complex comprising: (i) a class 2, type II Cas endonuclease; and (ii) one or more engineered guide RNAs comprising: an RNA sequence configured to bind to the class 2, type II Cas endonuclease, and a spacer sequence configured to hybridize to a first set of one or more target loci. In some embodiments, the method further comprises contacting to or introducing to said cell (b) a class 2, type V Cas endonuclease complex comprising: (i) a class 2, type V Cas endonuclease; and (ii) one or more engineered guide RNAs comprising: an RNA sequence configured to bind to the class 2, type V Cas endonuclease, and a spacer sequence configured to hybridize to a second set of one or more target loci. In some embodiments, the Cas endonucleases are contacted in the form of ribonucleoprotein (RNP) particles (e.g. in the case of lipid-based or electroporation/nucleofection-based transfection). In some embodiments, the Cas endonucleases are introduced in the form of sequences encoding said endonucleases or associated guide RNAs (e.g. in the case of vectors or in- vitro transcribed mRNA). In some embodiments, editing comprises insertion of an indel, a premature termination codon, a missense codon, a frameshift mutation, an adenine deamination, a cytosine deamination, or any combination thereof.

The Cas endonucleases can be specific Cas endonucleases, introduced under particular parameters, or introduced in a manner to achieve a specific target metric. In some embodiments, said class 2, type II Cas endonuclease is not a Cas9 endonuclease. In some embodiments, said class 2, type II Cas endonuclease is a Cas12a endonuclease. In some embodiments, said class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof. In some embodiments, said class 2, type V Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to SEQ ID NO: 7 or a variant thereof. In some embodiments, said first engineered guide RNA or said second engineered guide RNA comprises a sequence having at least 80%, 85%, 90%, or 95% sequence identity to any one of SEQ ID NOs: 3, 6, or 9. In some embodiments, said method edits genomic sequences of said first locus with at least about 25%, 30%, 35%, 40%, 45%, 50%, 55%, 60%, 65%, 70%, 75%, 80%, 85%, 90%, or more efficiency and/or said second locus with at least about 25%, 30%, 35%, 40%, 45%, 50%, 55%, 60%, 65%, 70%, 75%, 80%, 85%, 90%, or more efficiency. In some embodiments, said first RNA-guided endonuclease or said second RNA-guided endonuclease is introduced at a concentration of 200 pmol or less, 100 pmol or less, 50 pmol or less, 25 pmol or less, 5 pmol or less, or 1 pmol or less. In some embodiments, off-target sites are disrupted at a frequency of less than 0.2% as determined by a genome-wide off-target double-strand break analysis. In some embodiments, off-target sites are disrupted at a frequency of less than 0.01% as determined by a genome-wide off-target double-strand break analysis. In some embodiments, the genome-wide off-target double-strand break analysis comprises an HTGTS assay (high-throughput, genome-wide translocation sequencing; see e.g. Chiarle et al. Cell. 2011 Sep 30;147(1):107-19. doi: 10.1016/j.cell.2011.07.049, which is explicitly incorporated by reference herein for all purposes), a LAM-HTGTS assay (linear amplification mediated high-throughput genome-wide sequencing; see e.g. Hu et al. Nat Protoc. 2016. 11(5):853-71. doi:10.1038/nprot.2016.043, which is explicitly incorporated by reference herein for all purposes), or a Digenome-Seq (in vitro Cas-digested whole genome sequencing; see e.g. Kim et al. Nat Methods. 2015. 12(3):237-43. doi:10.1038/nmeth.3284, which is explicitly incorporated by reference herein for all purposes) assay.

The targeted loci can comprise any loci. The targeted loci can be particular therapeutically-interesting loci, such as the T cell receptor (TCR) locus (including constant regions of the TCR locus that are preserved multiple subtypes of T-cells such as TRAC and TRBC), glucocorticoid receptor locus (aka the GR locus), loci encoding other nuclear hormone receptors (e.g. estrogen receptor, progesterone receptor, or androgen receptor loci) or loci encoding particular oncogenes or tumor suppressors. In some embodiments, said first set of one or more target loci or said second set of one or more target loci comprises a T-cell receptor (TCR) locus. In some embodiments, said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 10-15. In some embodiments, said first set of one or more target loci or said second set of one or more target loci comprises a Nuclear Receptor Subfamily 3 Group C Member 1 (NR3C1) locus. In some embodiments, said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 16, 20, 21, or 22.

Any of the editing methods used herein can be used in conjunction with a donor nucleic acid molecule to e.g. introduce a transgene by homologous recombination at one of the sites targeted by a Cas enzyme or Cas complex. In some embodiments, the method further comprises introducing to said cell a donor DNA sequence comprising an open reading frame encoding a transgenic version of an endogenous gene, a first homology arm comprising a DNA sequence located on a first side of said target sequence and a second homology arm comprising a DNA sequence located on a second side of said target sequence within the locus of the endogenous gene. In some cases, the transgene can be a CAR-T molecule. In some embodiments, the method further comprises introducing to said cell a donor DNA sequence comprising an open reading frame encoding a heterologous engineered T-cell receptor molecule, a first homology arm comprising a DNA sequence located on a first side of said target sequence and a second homology arm comprising a DNA sequence located on a second side of said target sequence within the TCR locus.

In some aspects, the present disclosure provides for a method of making a glucocorticoid-resistant engineered T cell, comprising introducing to a T-cell or a precursor thereof: (a) an RNA guided endonuclease complex targeting a T-cell receptor (TCR) locus, comprising: (i) a first RNA guided endonuclease or DNA encoding said first RNA guided endonuclease; and (ii) a first engineered guide RNA comprising an RNA sequence configured to form a complex with said first RNA guided endonuclease, and a first spacer sequence configured to hybridize to at least part of said TCR locus. In some embodiments, the method further comprises introducing to said T-cell or said precursor thereof: (b) an RNA guided endonuclease complex targeting a T-cell receptor Nuclear Receptor Subfamily 3 Group C Member 1 (NR3C1) locus, comprising: (i) a second RNA guided endonuclease; and (ii) a second engineered guide RNA comprising: an RNA sequence configured to form a complex with said second RNA guided endonuclease, and a second spacer sequence configured to hybridize to at least part of said NR3C1 locus. In some embodiments, said at least part of said TCR locus is within said T-cell locus. In some embodiments, the method further comprises introducing to said cell (b) a donor DNA sequence comprising an open reading frame encoding a heterologous engineered T-cell receptor molecule, a first homology arm comprising a DNA sequence located on a first side of said target sequence and a second homology arm comprising a DNA sequence located on a second side of said target sequence within the TCR locus.

The type II or type V endonucleases can comprise particular Cas endonucleases. In some embodiments, said first RNA guided endonuclease or said second RNA guided endonuclease comprises a class 2, type II or a class 2, type V Cas endonuclease. In some embodiments, said first RNA guided endonuclease comprises said class 2, type II Cas endonuclease and said second RNA guided endonuclease comprises said class 2, type V Cas endonuclease. In some embodiments, said second RNA guided endonuclease comprises said class 2, type II Cas endonuclease and said first RNA guided endonuclease comprises said class 2, type V Cas endonuclease. In some embodiments, said first RNA-guided endonuclease or said second RNA-guided endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1, 4, or 7. In some embodiments, said first engineered guide RNA or said second engineered guide RNA comprises a sequence having at least 80%, 85%, 90%, or 95% sequence identity to any one of SEQ ID NOs: 3, 6, or 9. In some embodiments, said first RNA-guided endonuclease or said second RNA-guided endonuclease is present at a concentration of 100 pmol or less, 50 pmol or less, 25 pmol or less, 5 pmol or less, or 1 pmol or less.

Any of the editing methods used herein can be used in conjunction with a donor nucleic acid molecule to e.g. introduce a transgene by homologous recombination at one of the sites targeted by a Cas enzyme or Cas complex. In some embodiments, said heterologous engineered T-cell receptor is a CAR molecule. In some embodiments, said at least part of said T cell receptor locus is a T Cell Receptor Alpha Constant (TRAC) locus or a T Cell Receptor Beta Constant (TRBC) locus. In some embodiments, said at least part of said T cell receptor locus is a TRAV or TRAJ locus. In some embodiments, said at least part of said T cell receptor locus is a TRBV or TRBJ locus. In some embodiments, said homology arms comprise intronic or exonic regions within the TCR locus proximal to said at least part of said T cell receptor locus. In some embodiments, said at least part of said T cell receptor locus is a first or third exon of TRAC. In some embodiments, said method disrupts genomic sequences of said TCR locus and said NR3C1 locus with at least about 25%, 30%, 35%, 40%, 45%, 50%, 55%, 60%, 65%, 70%, 75%, 80%, 85%, 90%, or more efficiency. In some embodiments, said efficiency is determined by flow cytometry for a protein expressed from said TCR or NR3C1 loci. In some embodiments, said at least part of said NR3C1 locus is exon 2 or exon 3. In some embodiments, said method produces cells positive for the CAR molecule and negative for NR3C1 with at least about 25%, 30%, 35%, 40%, 45%, 50%, 55%, 60%, 65%, 70%, 75%, 80%, 85%, 90%, or more efficiency. In some embodiments, the method comprises introducing (a)-(c) to said T-cell or precursor thereof simultaneously. In some embodiments, said T-cell or said precursor thereof comprises a T-cell, a hematopoietic stem cell (HSC), or peripheral blood mononuclear cell (PBMC). In some embodiments, said second spacer sequence comprises a sequence having at least 80%, 85%, 90%, or 95% sequence identity to any one of SEQ ID NOs: 16, 20, 21, or 22. In some embodiments, said first or said second spacer sequence comprises at least about 19-24 nucleotides, at least about 19 nucleotides, at least about 20 nucleotides, at least about 22 nucleotides, or at least about 24 nucleotides.

.Donor sequences used in conjunction with the methods described herein can be provided in a variety of forms in the method. In some embodiments, donor sequences are provided in the form of nucleic acid molecules (e.g. single- or double-stranded DNA, or RNA). In some embodiments, donor sequences are provided in vectors (e.g. plasmids, YACmids, BACmids, phagemids, or viral vectors). In the case of viral vectors, viral vectors can comprise AAV viruses with particular serotypes. In some embodiments, said donor DNA sequence is delivered in a viral vector. In some embodiments, said viral vector is an AAV or AAV-6 vector.

In some aspects, the present disclosure provides for a population of glucocorticoid-resistant CAR-T cells, comprising:(a) an heterologous sequence within 100, 75, 50, 25, or 10 nucleotides of a hybridization region of any one of SEQ ID NOs: 10-15 within a TCR locus. In some embodiments, the population further comprises (b) an NR3C1 locus comprising an indel. In some embodiments, said heterologous sequence is an indel. In some embodiments, said heterologous sequence comprises an open reading frame comprising a nucleotide sequence encoding a heterologous T-cell receptor or a CAR molecule. In some embodiments, said NR3C1 locus comprises an indel within 100, 75, 50, 25, or 10 nucleotides of a hybridization region of any one of SEQ ID NOs: 16, 20, 21, or 22. In some embodiments, less than 0.2% of cells in said population have indels at off-target loci as determined by a genome-wide off-target double-strand break analysis. In some embodiments, less than 0.01% of cells in said population have indels at off-target loci as determined by a genome-wide off-target double-strand break analysis. In some embodiments, the genome-wide off-target double-strand break analysis comprises an HTGTS assay (high-throughput, genome-wide translocation sequencing; see e.g. Chiarle et al. Cell. 2011 Sep 30;147(1):107-19. doi: 10.1016/j.cell.2011.07.049, which is explicitly incorporated by reference herein for all purposes), a LAM-HTGTS assay (linear amplification mediated high-throughput genome-wide sequencing; see e.g. Hu et al. Nat Protoc. 2016. 11(5):853-71. doi:10.1038/nprot.2016.043, which is explicitly incorporated by reference herein for all purposes), or a Digenome-Seq (in vitro Cas-digested whole genome sequencing; see e.g. Kim et al. Nat Methods. 2015. 12(3):237-43. doi:10.1038/nmeth.3284, which is explicitly incorporated by reference herein for all purposes) assay. In some embodiments, said population of cells is substantially free of chromosomal translocations.

In some aspects, the present disclosure provides for a cell produced by any of the methods described herein.

In some aspects, the present disclosure provides for protein sequences or nucleotide sequences provided in Table 1 below.

**Table 1: Example Protein, Guide RNA, targeting sequences, and homology arms described herein**

| **Description** | **Amino acid sequence or nucleotide sequence** | **SEQ ID NO:** |
|---|---|---|
| MG3-6 protein sequence (Class 2, type II) | MSADSLNYRIGVDVGDRSVGLAAIELDDDGFPLKKLAMVTFRHDGGKDPATGK TPKSRKETAGVARRTMRMRRRKKKRLKDLDKKLRDLGYFVPRDEEPQTYEAWS SRARLAESRFEDPHERGEHLVRAVRHMARHRGWRNPWWSFSQLEEASQEPSET FGRILERAQHEWGERVSDNATLGMLGALAANNNILLRPRRYEHNPKTGKNAEK LNVRGQEPILLDKVRQEDVLAELRRICKVQGIEDQYPELAHAVFTQVRPYVPT ERVGKDPLQPMKIRASRASLEFQEFRIRDAVANLRIRVGGSERRPLTEEEYDR AVDYLMEYSDTTPPTWGEVADELEIAENTLIAPVIDDVRLNVAPYDRSSAIVE AKLKRKTQARQWWDDDANLDLRSQLILLVSDATDDTARVAENSGLLEVFESWS DEEKQTLQDLKFDSGRAAYSIDTLNKLNAYMHEHRVGLHEARQNVFGVSDTWR PPRDRLDEPTGQPTVDRVLTIVRRFILDCERAWGRPQKIVVEHARTGLMGPSQ RADVLKEIARNRNANERIRQELREGGIEAPNRADIRRNSIIQDQESQCLYCGK EIGVLTAELDHIVPRAGGGSSKRENLAAVCRACNASKGSRPFAVWAGPARLER TIQRLRELQAFKTKSKKRTLNAIIRRLKQREEDEPIDERSLASTSYAATSIRE RLEQHFNDDLPDGFAPVAVDVYGGSLTRESRRAGGIDKSIMLRGQSDKNRFDV RHHAIDAAVMTLLNPSVAVTLEQRRMLKQENDYSSPRGQHDNGWRDFIGRGEA SQSKFLHWKKTAVVLADLISEAIEQDTIPVVNPLRLRPQNGSVHKDTVEAVLE RTVGDSWTDKQVSRIVDPNTYIAFLSLLGRKKELDADHQRLVSVSAGVKLLAD ERVQIFPEEAASILTPRGVVKIGDSIHHARLYGWKNQRGDIQVGMLRVFGAEF PWFMRESGVKDILRVPIPQGSQSYRDLAATTRKFIENGQATEFGWITQNDEIE ISAEEYLATDKGDILSDFLGILPEIRWKVTGIEDNRRIRLRPLLLSSEAIPNM LNGRLLTQEEHDLIALVINKGVRVVVSTFLALPSTKIIRRNNLGIPRWRGNGH LPTSLDIQRAATQALEGRD | 1 |
| MG3-6 consensus 3' PAM with sgRNA | nnRGRYY | 2 |
| MG3-6 sgRNA | NNNNNNNNNNNNNNNNNNNNGUUGAGAAUCGAAAGAUUCUUAAUAAGGCAU CCUUCCGAUGCUGACUUCUCACCGUCCGUUUUCCAAUAGGAGCGGGCGGUAUG UUUU | 3 |
| MG3-8 protein sequence (Class 2, type II) | MSTDMKNYRIGVDVGDRSVGLAAIEFDDAGFPIQKLALVTFRHDGGLDPTDNP KSRKETRGEARRRMRMTRRRKQRLRDLDKVLENLGYTVPEGPEPETYEAWTSR ALLASIKLASADELNEHLVRAVRHIARHRGWVNPWWSLDQLERASQEPSETFE IILARARELFGERVPANPTLGMLGALAANNEVLLRPRAEKKKKTGYVRGTPLL AAQVRQIDQVAELRRICEVQGIEEQYETLRNAIFAHKVAYVPTERVGKDPLAP SKNRTIRASLEFQEFRILDSVANLRVRTDSRAKRELTEGEYDAAVEFLMGYTA KEQPSWADVAEEIGVPGNRLIAPVLEDVQQKTAPFDRSSAAFEKAMSKRTEAR QWWEANDDDQLRSLFIMFLADATNDTEEAAAVAGLPELYMSWPAEEREALSNI DFEKGRVAYSHETLSKLSEYMHEHRVGLHEARKAVFGVDDTWRPPLAKLEEPT GQPTVDRVLTILRRFVLDCERQWGRPRAITVEHARIGLVGPAQRQNILKEQED NRKNNECIRDELRKSGVENPSRTEVRRHLVVQDQESQCLYCGAVIRTDTSELD HIVPRAGGGSSRRENLAAVCRYCNSKKDRTLFYDWAGSVRLQETIDRVRQLKA FKDSKKAKMFKNQIRRLRQTEADEPIDERSLASTSYAAVAVRERLEQHFNEGL APDDKNRVVLDVYAGSVTRESRRAGGIDERILLRGERDKNRFDVRHHAIDAAV MTLLNRSVALTLEQRSQLRRAFYEQGLDKLDRDQLKPEEDWRNFIGLSLASQE KFLEWKKVTTVLGDLLAEAIEDDSIAVVSPLRLRPQNGRVHKDTIAAVKKQTL GSAWSADAVKRIVDPEIYLAMKDALGKSKVLPEDSARTLELSDGRYLEADDEV LFFPKNAASILTPRGVAEIGGSIHHARLYSWLTKKGELKIGMLRVYGAEFPWL MRESGSHDVLRMPIHPGSQSFRDMQDTTRKAVESSEAVEFAWITQNDELEFEP EDYIAHGGKDELRQFLEFMPECRWRVDGFKKNYQIRIRPAMLSREQLPSDIQR RLESKTLTENESLLLKALDTGLVVAIGGLLPLGTLKVIRRNNLGFPRWRGNGN LPTSFEVRSSALRALGVEG | 4 |
| MG3-8 consensus 3'PAM | nnRGRTY | 5 |
| MG3-8 sgRNA | NNNNNNNNNNNNNNNNNNNNGUUGAGAAUCGAAAGAUUCUUAAUAAGGCAU CCUUCCGAUGCUGACUUCUCACCGUCCGGCUCCUCUUAGGAACGGGCGGUAUG UUUU | 6 |
| MG29-1 (Class 2, type V-A Cas effector, Cas12a class) protein sequence | MFNNFIKKYSLQKTLRFELKPVGETADYIEDFKSEYLKDTVLKDEQRAKDYQE IKTLIDDYHREYIEECLREPVDKKTGEILDFTQDLEDAFSYYQKLKENPTENR VGWEKEQESLRKKLVTSFVGNDGLFKKEFITRDLPEWLQKKGLWGEYKDTVEN FKKFTTYFSGFHENRKNMYTAEAQSTAIANRLMNDNLPKFFNNYLAYQTIKEK HPDLVFRLDDALLQAAGVEHLDEAFQPRYFSRLFAQSGITAFNELIGGRTTEN GEKIQGLNEQINLYRQQNPEKAKGFPRFMPLFKQILSDRETHSFLPDAFENDK ELLQALRDYVDAATSEEGMISQLNKAMNQFVTADLKRVYIKSAALTSLSQELF HFFGVISDAIAWYAEKRLSPKKAQESFLKQEVYAIEELNQAVVGYIDQLEDQS ELQQLLVDLPDPQKPVSSFILTHWQKSQEPLQAVIAKVEPLFELEELSKNKRA PKHDKDQGGEGFQQVDAIKNMLDAFMEVSHAIKPLYLVKGRKAIDMPDVDTGF YADFAEAYSAYEQVTVSLYNKTRNHLSKKPFSKDKIKINFDAPTLLNGWDLNK ESDNKSIILRKDGNFYLAIMHPKHTKVFDCYSASEAAGKCYEKMNYKLLSGAN KMLPKVFFSKKGIETFSPPQEILDLYKNNEHKKGATFKLESCHKLIDFFKRNI PKYKVHPTDNFGWDVFGFHFSPTSSYGDLSGFYREVEAQGYKLWFSDVSEAYI NKCVEEGKLFLFQIYNKDFSPNSTGKPNLHTLYWKGLFEPENLKDVVLKLNGE AEIFYRKHSIKHEDKTIHRAKDPIANKNADNPKKQSVFDYDIIKDKRYTQDKF FFHVPISLNFKSQGVVRFNDKINGLLAAQDDVHVIGIDRGERHLLYYTVVNGK GEVVEQGSLNQVATDQGYVVDYQQKLHAKEKERDQARKNWSTIENIKELKAGY LSQVVHKLAQLIVKHNAIVCLEDLNFGFKRGRFKVEKQVYQKFEKALIDKLNY LVFKERGATQAGGYLNAYQLAAPFESFEKLGKQTGILYYVRSDYTSKIDPATG FVDFLKPKYESMAKSKVFFESFERIQWNQAKGYFEFEFDYKKMCPSRKFGDYR TRWVVCTFGDTRYQNRRNKSSGQWETETIDVTAQLKALFAAYGITYNQEDNIK DAIAAVKYTKFYKQLYWLLRLTLSLRHSVTGTDEDFILSPVADENGVFFDSRK ATDKQPKDADANGAYHIALKGLWNLQQIRQHDWNVEKPKKLNLAMKNEEWFGF AQKKKFRA | 7 |
| MG29-1 consensus 5' PAM | TTTn | 8 |
| MG29-1 crRNA | UAAUUUCUACUGUUGUAGAUNNNNNNNNNNNNNNNNNNNNNNNN | 9 |
| MG3-6-TRAC-6-22 | 5' - CGAATCCTCCTCCTGAAAGTGG-3' | 10 |
| SpyCas9 TRAC-1-20 | 5'-ACAAAACTGTGCTAGACATG-3' | 11 |
| SpyCas9 TRAC-2-20 | 5'-AGAGCAACAGTGCTGTGGCC-3' | 12 |
| SpyCas9 TRAC-3-20 | 5'-TCTCTCAGCTGGTACACGGC-3' | 13 |
| MG29-1-TRAC-19-22 | 5'-TTGCTCCAGGCCACAGCACTGT-3' | 14 |
| MG29-1-TRAC-35-22 | 5'-GAGTCTCTCAGCTGGTACACGG-3' | 15 |
| MG29-1-GR-29-20 (NR3C1 targeting) | 5'-CAGCTTCCACAAGTTAAGAC-3' | 16 |
| MG29-1-ALB-87 (albumin targeting) | 5'-CGCACTAAGGAAAGTGCAAAGT-3' | 17 |
| MG29-1-ALB-74 (albumin targeting) | 5'- AATAAAGCATAGTGCAATGGAT-3' -3' | 18 |
| MG29-1-ALB-83 (albumin targeting) | 5' - TGAGATCAACAGCACAGGTTTT-3' -3' | 19 |
| NR3C1 spacer target sequence | GTCTGTGGTATACAATTTCA | 20 |
| MG29-1-GR-13-20 ("target B") | | |
| NR3C1 spacer target sequence MG29-1-GR-28-20 ("target C") | GGAGGTGGTCCTGTTGTTGC | 21 |
| NR3C1 spacer target sequence MG29-1-GR-29-20 ("target D") | CAGCTTCCACAAGTTAAGAC | 22 |
| CAR 5' homology arm targeting TRAC | TTAATGCCAACATACCATAAACCTCCCATTCTGCTAATGCCCAGCCTAAGTTG GGGAGACCACTCCAGATTCCAAGATGTACAGTTTGCTTTGCTGGGCCTTTTTC CCATGCCTGCCTTTACTCTGCCAGAGTTATATTGCTGGGGTTTTGAAGAAGAT CCTATTAAATAAAAGAATAAGCAGTATTATTAAGTAGCCCTGCATTTCAGGTT TCCTTGAGTGGCAGGCCAGGCCTGGCCGTGAACGTTCACTGAAATCATGGCCT CTTGGCCAAGATTGATAGCTTGTGCCTGTCCCTGAGTCCCAGTCCATCACGAG CAGCTGGTTTCTAAGATGCTATTTCCCGTATAAAGCATGAGACCGTGACTTGC CAGCCCCACAGAGCCCCGCCCTTGTCCATCACTGGCATCTGGACTCCAGCCTG GGTTGGGGCAAAGAGGGAAATGAGATCATGTCCTAACCCTGATCCTCTTGTCC CACAGATATCCAGAACCCTGACC | 23 |
| CAR 3' homology arm targeting TRAC | CTGCCGTGTACCAGCTGAGAGACTCTAAATCCAGTGACAAGTCTGTCTGCCTA TTCACCGATTTTGATTCTCAAACAAATGTGTCACAAAGTAAGGATTCTGATGT GTATATCACAGACAAAACTGTGCTAGACATGAGGTCTATGGACTTCAAGAGCA ACAGTGCTGTGGCCTGGAGCAACAAATCTGACTTTGCATGTGCAAACGCCTTC AACAACAGCATTATTCCAGAAGACACCTTCTTCCCCAGCCCAGGTAAGGGCAG CTTTGGTGCCTTCGCAGGCTGTTTCCTTGCTTCAGGAATGGCCAGGTTCTGCC CAGAGCTCTGGTCAATGATGTCTAAAACTCCTCTGATTGGTGGTCTCGGCCTT ATCCATTGCCACCAAAACCCTCTTTTTACTAAGAAACAGTGAGCCTTGTTCTG GCAGTCCAGAGAATGACACGGGAAAAAAGCAGATGAAGAGAAGGTGGCAGGAG AGGGCACGTGGCCCAGCCTCAGT | 24 |
| AAVS1 spacer target sequence | GGGGCCACTAGGGACAGGAT | 41 |
| GR isoform 5' homology arm targeting AAVS1 | CTCCTTCTGGGGCCTGTGCCATCTCTCGTTTCTTAGGATGGCCTTCTC CGACGGATGTCTCCCTTGCGTCCCGCCTCCCCTTCTTGTAGGCCTGCA TCATCACCGTTTTTCTGGACAACCCCAAAGTACCCCGTCTCCCTGGCT TTAGCCACCTCTCCATCCTCTTGCTTTCTTTGCCTGGACACCCCGTTC TCCTGTGGATTCGGGTCACCTCTCACTCCTTTCATTTGGGCAGCTCCC CTACCCCCCTTACCTCTCTAGTCTGTGCTAGCTCTTCCAGCCCCCTGT CATGGCATCTTCCAGGGGTCCGAGAGCTCAGCTAGTCTTCTTCCTCCA ACCCGGGCCCCTATGTCCACTTCAGGACAGCATGTTTGCTGCCTCCAG GGATCCTGTGTCCCCGAGCTGGGACCACCTTATATTCCCAGGGCCGGT TAATGTGGCTCTGGTTCTGGGTACTTTTATCTGTCCCCTCCACCCCAC AGTGGGGCCACTAGGGACAG | 42 |
| GR isoform 3' homology arm targeting AAVS1 | GATTGGTGACAGAAAAGCCCCATCCTTAGGCCTCCTCCTTCCTAGTCT CCTGATATTGGGTCTAACCCCCACCTCCTGTTAGGCAGATTCCTTATC TGGTGACACACCCCCATTTCCTGGAGCCATCTCTCTCCTTGCCAGAAC CTCTAAGGTTTGCTTACGATGGAGCCAGAGAGGATCCTGGGAGGGAGA GCTTGGCAGGGGGTGGGAGGGAAGGGGGGGATGCGTGACCTGCCCGGT TCTCAGTGGCCACCCTGCGCTACCCTCTCCCAGAACCTGAGCTGCTCT GACGCGGCCGTCTGGTGCGTTTCACTGATCCTGGTGCTGCAGCTTCCT TACACTTCCCAAGAGGAGAAGCAGTTTGGAAAAACAAAATCAGAATAA GTTGGTCCTGAGTTCTAACTTTGGCTCTTCACCTTTCTAGTCCCCAAT | 43 |
| | TTATATTGTTCCTCCGTGCGTCAGTTTTACCTGTGAGATAAGGCCAGT AGCCAGCCCCGTCCTGGCAG | |
| MG3-6 sgRNA targeting TRAC (MG3-6-TRAC-6) | mC*mG*mA*rArUrCrCrUrCrCrUrCrCrUrGrArArArGrUrGrGr GrUrUrGrArGrArArUrCrGrArArArGrArUrUrCrUrUrArArUr ArArGrGrCrArUrCrCrUrUrCrCrGrArUrGrCrUrGrArCrUrUr CrUrCrArCrCrGrUrCrCrGrUrUrUrUrCrCrArArUrArGrGrAr GrCrGrGrGrCrGrGrUrArUrGrU*mU*mU*mU | 44 |
| MG3-6 sgRNA targeting TRBC1/2 (MG3-6-TRBC-E2) | mC*mG*mG*rCrGrCrUrGrArCrGrArUrCrUrGrGrGrUrGrGrUr UrGrArGrArArUrCrGrArArArGrArUrUrCrUrUrArArUrArAr GrGrCrArUrCrCrUrUrCrCrGrArUrGrCrUrGrArCrUrUrCrUr CrArCrCrGrUrCrCrGrUrUrUrUrCrCrArArUrArGrGrArGrCr GrGrGrCrGrGrUrArUrGrU*mU*mU*mU | 45 |
| MG29-1 sgRNA targeting TRAC (MG29-1-TRAC-35) | mU*rArArUrUrUrCrUrArCrUrGrUrUrGrUrArGrArUrGrArGr UrCrUrCrUrCrArGrCrUrGrGrUrArCrArCrG*mG | 46 |
| MG29-1 sgRNA targeting TRBC1/2 (MG29-1-TRBC-A1) | mU*rArArUrUrUrCrUrArCrUrGrUrUrGrUrArGrArUrArGrCr CrArUrCrArGrArArGrCrArGrArGrArU*mC | 47 |
| MG29-1 sgRNA targeting TRBC1/2 (MG29-1-TRBC-G2) | mU*rArArUrUrUrCrUrArCrUrGrUrUrGrUrArGrArUrGrCrCr CrUrArUrCrCrUrGrGrGrUrCrCrArCrU*mC | 48 |
| MG29-1 sgRNA targeting B2M (MG29-1-B2M-H1) | mU*rArArUrUrUrCrUrArCrUrGrUrUrGrUrArGrArUrArUr CrUrCrUrUrGrUrArCrUrArCrArCrUrGrArA*mU | 49 |
| MG29-1 sgRNA targeting B2M (MG29-1-B2M-D2) | mU*rArArUrUrUrCrUrArCrUrGrUrUrGrUrArGrArUrArGrUr GrGrGrGrGrUrGrArArUrUrCrArGrUrGrUrA*mG | 50 |
| MG29-1 sgRNA targeting B2M (MG29-1-B2M-A3) | mU*rArArUrUrUrCrUrArCrUrGrUrUrGrUrArGrArUrCrArUr UrCrUrCrUrGrCrUrGrGrArUrGrArCrGrUrG*mA | 51 |
| TRAC spacer target sequence (MG3-6-TRAC-6) | CGAATCCTCCTCCTGAAAGTGG | 52 |
| AAVS1 spacer target sequence (MG3-6-AAVS1-D2) | TAGGAAGGAGGAGGCCTAAGGA | 53 |
| TRAC spacer target sequence (MG29-1-TRAC-35) | GAGTCTCTCAGCTGGTACACGG | 54 |
| AAVS1 spacer target sequence (MG29-1-AAVS1-F3) | TCTGTCCCCTCCACCCCACAGT | 55 |
| AAVS1 spacer target sequence (SpCas9, Mali et al. AAVS1 T2) | GGGGCCACTAGGGACAGGATTGG | 56 |
| MG3-6 sgRNA targeting TRAC (MG3-6-TRAC-6) | mC*mG*mA*rArUrCrCrUrCrCrUrCrCrUrGrArArArGrUrGrGr GrUrUrGrArGrArArUrCrGrArArArGrArUrUrCrUrUrArArUr ArArGrGrCrArUrCrCrUrUrCrCrGrArUrGrCrUrGrArCrUrUr CrUrCrArCrCrGrUrCrCrGrUrUrUrUrCrCrArArUrArGrGrAr GrCrGrGrGrCrGrGrUrArUrGrU*mU*mU*mU | 57 |
| MG3-6 sgRNA targeting AAVS1 (MG3-6-AAVS1-D2) | mU*mA*mG*rGrArArGrGrArGrGrArGrGrCrCrUrArArGrGrAr GrUrUrGrArGrArArUrCrGrArArArGrArUrUrCrUrUrArArUr ArArGrGrCrArUrCrCrUrUrCrCrGrArUrGrCrUrGrArCrUrUr CrUrCrArCrCrGrUrCrCrGrUrUrUrUrCrCrArArUrArGrGrAr GrCrGrGrGrCrGrGrUrArUrGrU*mU*mU*mU | 58 |
| MG29-1 sgRNA targeting TRAC (MG29-1-TRAC-35) | mU*rArArUrUrUrCrUrArCrUrGrUrUrGrUrArGrArUrGrArGr UrCrUrCrUrCrArGrCrUrGrGrUrArCrArCrG*mG | 59 |
| MG29-1 sgRNA targeting AAVS1 (MG29-1-AAVS1-F3) | /AltR1/rUrArArUrUrUrCrUrArCrUrGrUrUrGrUrArGrArUr UrCrUrGrUrCrCrCrCrUrCrCrArCrCrCrCrArCrArGrU/AltR 2/ | 60 |
| SpCas9 sgRNA targeting AAVS1 (SpCas9, Mali et al. AAVS1 T2) | mG*mG*mG* rGrCrC rArCrU rArGrG rGrArC rArGrG rArUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU | 61 |
| MG3-6 nuclease (mRNA sequence) | ATGCATGCGCGGCCGCAAGCTTTAATACGACTCACTATAGGAAAAGC CAGCTCCAGCAGGCGCTGCTCACTCCTCCCCATCCTCTCCCTCTGTCC CTCTGTCCCTCTGACCCTGCACTGTCCCAGCACCATGGCCCCCAAGAA GAAGCGGAAAGTTGGCGGCGGAGGCAGCAGCACCGACATGAAGAACTA CCGGATCGGCGTGGACGTGGGCGATAGATCTGTTGGACTGGCCGCCAT CGAGTTCGACGATGATGGACTGCCCATCCAGAAGCTGGCCCTGGTCAC CTTTAGACACGATGGCGGACTGGACCCCACCAAGAACAAGACCCCTAT GAGCCGGAAAGAGACACGGGGAATCGCCAGACGGACCATGCGGATGAA CAGAGAGCGGAAGCGGCGGCTGAGAAACCTGGACAACGTGCTGGAAAA CCTGGGCTACTCTGTGCCTGAGGGCCCTGAGCCTGAGACATATGAGGC CTGGACAAGCAGAGCCCTGCTGGCCTCTATCAAACTGGCCTCTGCCGA CGAGCTGAACGAACACCTTGTCAGAGCCGTGCGGCACATGGCCAGACA | 62 |
| | TAGAGGATGGCCAATCCTTGGTGGTCCCTGGACCAGCTGGAAAAGGC CAGCCAAGAGCCTAGCGAGACATTCGAGATCATCCTGGCCAGAGCCAG AGAGCTGTTCGGCGAGAAGGTGCCCGCTAATCCTACACTGGGAATGCT GGGAGCCCTGGCCGCTAACAATGAGGTGCTGCTGAGGCCCAGGGACGA GAAGAAGAGAAAGACCGGATACGTGCGGGCACCCCTCTGATGTTTGC TCAAGTTCGACAGGGCGATCAGCTGGCCGAGCTGCGGAGAATTTGTGA AGTGCAGGGCATCGAGGACCAGTACGAGGCTCTGAGACTGGGCGTGTT CGACCACAAGCACCCCTACGTGCCCAAAGAAAGAGTGGGCAAAGACCC TCTGAACCCCAGCACCAACAGAACCATCAGAGCCAGCCTGGAATTTCA AGAGTTCCGCATCCTGGACAGCGTGGCCAATCTGAGAGTGCGGATCGG CAGCAGAGCCAAGAGGGAACTGACAGAGGCCGAGTATGATGCCGCCGT GGAATTCCTGATGGACTACGCCGACAAAGAGCAGCCTAGCTGGGCCGA TGTGGCCGAGAAAATTGGCGTGCCCGGCAACAGACTGGTGGCCCCTGT TCTGGAAGATGTGCAGCAGAAAACAGCCCCTTACGACAGAAGCAGCGC CGCCTTTGAGAAGGCCATGGGCAAGAAAACCGAGGCCAGACAGTGGTG GGAGTCCACCGATGATGACCAGCTGAGAAGCCTGCTGATTGCCTTCCT GGTGGACGCCACCAACGACACAGAAGAAGCCGCTGCTGAAGCCGGCCT GAGCGAGCTGTATAAGTCTTGGCCTGCCGAGGAAAGAGAGGCCCTGTC CAACATCGACTTCGAGAAGGGCAGAGTGGCCTACAGCCAAGAAACCCT GAGCAAGCTGAGCGAGTACATGCACGAGTACAGAGTGGGACTGCACGA GGCTAGAAAGGCCGTGTTCGGAGTGGATGATACCTGGCGGCCTCCTCT GGATAAGCTGGAAGAACCTACAGGACAGCCTGCCGTGGACAGAGTGCT GACCATCCTGAGAAGATTCGTGCTGGACTGCGAGCGGCAATGGGGCAG ACCTAGAGCCATCACCGTGGAACACACACGGACAGGCCTGATGGGCCC AACACAGAGACAGAAGATCCTGAACGAGCAGAAGAAGAACCGGGCCGA CAACGAGAGAATCCGGGATGAGCTGAGAGAATCTGGCGTGGACAACCC CTCCAGAGCCGAAGTTCGGAGACACCTGATCGTGCAAGAGCAAGAGTG CCAGTGCCTGTACTGCGGCACCATGATCACCACCACCACAAGCGAGCT GGACCACATCGTTCCTAGAGCCGGTGGCGGCAGCAGCAGAAGGAAAA TCTGGCCGCTGTGTGCAGAGCCTGCAACGCCAAGAAGAAACGCGAGCT GTTCTACGCCTGGGCTGGCCCAGTGAAGTCCCAAGAGACAATCGAGAG AGTCAGACAGCTGAAGGCCTTTAAGGACAGCAAGAAAGCCAAGATGTT CAAGAACCAGATCCGCCGGCTGAACCAGACCGAGGCCGATGAGCCTAT CGACGAAAGAAGCCTGGCCAGCACATCTTACGCCGCTGTGGCCGTTAG AGAGCGGCTGGAACAGCACTTCAACGAAGGCCTGGCACTGGACGACAA GTCCAGAGTGGTGCTGGATGTGTATGCCGGCGCTGTGACCAGAGAGTC TCGTAGAGCTGGCGGCATCGACGAGCGGATTCTGCTGAGAGGCGAGCG GGACAAGAACAGATTCGATGTGCGGCATCACGCCGTGGACGCTGCTGT TATGACCCTGCTGAACAGATCCGTGGCTCTGACCCTGGAACAGAGATC ACAGCTGCGGCGGACCTTCTACGAGCAAGGACTGGACAAACTGGACCG GAACCAGCTGAAGCCCGAGGAAGATTGGAGAGACTTCACCGGACTGGC CCCTGCCTCTCAAGAGAAGTTTCTGGAATGGCGGAAGGCCGCCACCAT CCTGGGAGATTTGCTGGCCGAAGCCATCGAGGATGACTCTATCGCCGT GGTGTCCCCACTGAGACTGAGGCCACAGAATGGCAGCGTGCACCTGGA AACAATCAGCGCCGTGAAGAAGCAGACCCTGGGCTCTGATTGGCCAGC CGACGCCGTGAAAAGAATCGTGGACCCCGAGATCTACCTGGCTATGAA GGATGCCCTGGGAAAGCTGAAAGAGCTGCCCGAGGATAGCGCCAGATC TCTGGAACTGCCCGACGGCAGATTCGTGGAAGCCGATGACGAGGTGCT GTTCTTCCCAGAGAACGCCGCCAGCATTCTGACCCCTAGAGGCGTGGC AGAGATCGGCGGCTCTATTCACCATGCCAGACTGTACGGCTGGCTGAC CAAAAAGGGCGAGCTGAAAGTGGGCATGCTGAGAGTGTACGGCGCCGA GTTTCCCTGGCTGATGAGAGAGTCCGGCTCCAGAAACGTGCTGAGCAT GCCTATCCACAGAGGCAGCCAGAGCTTCCGGGACATGCAGGACACAAC CCGGAAAGCCGTGGAAAGCGGAGAGGCTGTGGAATTCGCCTGGATCAC CCAGAACGATGAGCTGGAATTCGACCCCGACGACTACATTGCCCACGG CGGAAAGGACGAACTGAGACAGTTCCTGGGCTTTATGCCCGAGTGCCG | |
| | TTGGAGAGTGGACGGCTTCAAGAAGAATTACCAGATCAGAATCAGGCC CGCCATGCTGAGCAGAGAGCAGCTGCCTAGCGACATCCAGCGGAGACT GGAAAGCAAGACCCTGACCAAGAACGAGTCCCTGCTGCTGAAAGCCCT GGATACAGGACTGGTGGTGGCCATCGGAGGACTGCTGCCTCTCGAGAC ACTGAAAGTGATCCGGCGCAACAATCTGGGCTTCCCCAGGTGGCGCGG AAACGGAAATCTGCCCACCAGCTTTGAAGTGCGGAGCAGCGCTCTGAG AGCCCTGGGAGTTGAAGGATCTGGCGGAAAAAGACCTGCCGCCACAAA GAAAGCCGGACAGGCCAAGAAAAAGAAGTGACCACACCCCCATTCCCC CACTCCAGATAGAACTTCAGTTATATCTCACGTGTCTGGAGTTGGATC CATGCATGC | |
| Transgene inserted at AAVS1 locus (MND promoter-driven GFP and tEGFR coding sequences flanked by homology arms correspondin g to the cut site of Mali et al. AAVS1 T2 guide) | GGCCGCTTAATTAATAATAAGGAAGTGCCATTCCGCCTGACCTCTCCT TCTGGGGCCTGTGCCATCTCTCGTTTCTTAGGATGGCCTTCTCCGACG GATGTCTCCCTTGCGTCCCGCCTCCCCTTCTTGTAGGCCTGCATCATC ACCGTTTTTCTGGACAACCCCAAAGTACCCCGTCTCCCTGGCTTTAGC CACCTCTCCATCCTCTTGCTTTCTTTGCCTGGACACCCCGTTCTCCTG TGGATTCGGGTCACCTCTCACTCCTTTCATTTGGGCAGCTCCCCTACC CCCCTTACCTCTCTAGTCTGTGCTAGCTCTTCCAGCCCCCTGTCATGG CATCTTCCAGGGGTCCGAGAGCTCAGCTAGTCTTCTTCCTCCAACCCG GGCCCCTATGTCCACTTCAGGACAGCATGTTTGCTGCCTCCAGGGATC CTGTGTCCCCGAGCTGGGACCACCTTATATTCCCAGGGCCGGTTAATG TGGCTCTGGTTCTGGGTACTTTTATCTGTCCCCTCCACCCCACAGTGG GGCCACTAGGGACAGATTTGTGTGAACAGAGAAACAGGAGAATATGGG CCAAACAGGATATCTGTGGTAAGCAGTTCCTGCCCCGGCTCAGGGCCA AGAACAGTTGGAACAGCAGAATATGGGCCAAACAGGATATCTGTGGTA AGCAGTTCCTGCCCCGGCTCAGGGCCAAGAACAGATGGTCCCCAGATG CGGTCCCGCCCTCAGCAGTTTCTAGAGAACCATCAGATGTTTCCAGGG TGCCCCAAGGACCTGAAATGACCCTGTGCCTTATTTGAACTAACCAAT CAGTTCGCTTCTCGCTTCTGTTCGCGCGCTTCTGCTCCCCGAGCTCTA TATAAGCAGAGCTCGTTTAGTGAACCGTCAGATCAAATGCCTGGAGAC GCCATCCACGCTGTTTTGACCTCCATAGAAGACACCGACTCTAGAGGA TCCACCGGTCGCCACCATGGTGAGCAAGGGCGAGGAGCTGTTCACCGG GGTGGTGCCCATCCTGGTCGAGCTGGACGGCGACGTAAACGGCCACAA GTTCAGCGTGTCCGGCGAGGGCGAGGGCGATGCCACCTACGGCAAGCT GACCCTGAAGTTCATCTGCACCACCGGCAAGCTGCCCGTGCCCTGGCC CACCCTCGTGACCACCCTGACCTACGGCGTGCAGTGCTTCAGCCGCTA CCCCGACCACATGAAGCAGCACGACTTCTTCAAGTCCGCCATGCCCGA AGGCTACGTCCAGGAGCGCACCATCTTCTTCAAGGACGACGGCAACTA CAAGACCCGCGCCGAGGTGAAGTTCGAGGGCGACACCCTGGTGAACCG CATCGAGCTGAAGGGCATCGACTTCAAGGAGGACGGCAACATCCTGGG GCACAAGCTGGAGTACAACTACAACAGCCACAACGTCTATATCATGGC CGACAAGCAGAAGAACGGCATCAAGGTGAACTTCAAGATCCGCCACAA CATCGAGGACGGCAGCGTGCAGCTCGCCGACCACTACCAGCAGAACAC CCCCATCGGCGACGGCCCCGTGCTGCTGCCCGACAACCACTACCTGAG CACCCAGTCCGCCCTGAGCAAAGACCCCAACGAGAAGCGCGATCACAT GGTCCTGCTGGAGTTCGTGACCGCCGCCGGGATCACTCTCGGCATGGA CGAGCTGTACAAGGGCAGCGGCGAAGGAAGGGGTTCTCTGTTGACTTG CGGGGATGTTGAAGAAAACCCGGGACCAATGCTTCTCCTGGTGACAAG CCTTCTGCTCTGTGAGTTACCACACCCAGCATTCCTCCTGATCAGGAA GGTGTGCAACGGCATCGGCATCGGCGAGTTCAAGGACAGCCTGAGCAT CAACGCCACCAACATCAAGCACTTCAAGAACTGCACCAGCATCAGCGG CGACCTGCACATCCTGCCCGTGGCCTTCAGGGGCGACAGCTTCACCCA CACCCCCCCCCTGGACCCCCAGGAGCTGGACATCCTGAAGACCGTGAA GGAGATCACCGGCTTCCTGCTGATCCAGGCCTGGCCCGAGAACAGGAC CGACCTGCACGCCTTCGAGAACCTGGAGATCATCAGGGGCAGGACCAA GCAGCACGGCCAGTTCAGCCTGGCCGTGGTGAGCCTGAACATCACCAG CCTGGGCCTGAGGAGCCTGAAGGAGATCAGCGACGGCGACGTGATCAT | 63 |
| | CAGCGGCAACAAGAACCTGTGCTACGCCAACACCATCAACTGGAAGAA GCTGTTCGGCACCAGCGGCCAGAAGACCAAGATCATCAGCAACAGGGG CGAGAACAGCTGCAAGGCCACCGGCCAGGTGTGCCACGCCCTGTGCAG CCCCGAGGGCTGCTGGGGCCCCGAGCCCAGGGACTGCGTGAGCTGCAG GAACGTGAGCAGGGGCAGGGAGTGCGTGGACAAGTGCAACCTGCTGGA GGGCGAGCCCAGGGAGTTCGTGGAGAACAGCGAGTGCATCCAGTGCCA CCCCGAGTGCCTGCCCCAGGCCATGAACATCACCTGCACCGGCAGGGG CCCCGACAACTGCATCCAGTGCGCCCACTACATCGACGGCCCCCACTG CGTGAAGACCTGCCCCGCCGGCGTGATGGGCGAGAACAACACCCTGGT GTGGAAGTACGCCGACGCCGGCCACGTGTGCCACCTGTGCCACCCCAA CTGCACCTACGGCTGCACCGGCCCCGGCCTGGAGGGCTGCCCCACCAA CGGCCCCAAGATCCCCAGCATCGCCACCGGCATGGTGGGCGCCCTGCT GCTGCTGCTGGTGGTGGCCCTGGGCATCGGCCTGTTCATGGGCAGCGG CGAAGGAAGGGGTTCTCTGTTGACTTGCGGGGATGTTGAAGAAAACCC GGGACCAATGGGGAATGAAGCAAGTTATCCATTGGAAATGTGTAGCCA TTTTGATGCTGATGAAATAAAGAGACTCGGAAAACGATTTAAGAAACT CGATCTTGATAATAGTGGATCTCTCTCTGTCGAAGAATTCATGTCCCT TCCTGAACTCCAACAAAATCCACTCGTCCAAAGAGTCATTGATATATT TGATACGGATGGGAATGGTGAAGTCGATTTTAAAGAATTTATTGAAGG GGTTAGTCAATTTTCCGTCAAAGGGGATAAAGAACAGAAACTCCGCTT TGCGTTTCGAATTTATGACATGGACAAGGACGGATACATCTCCAACGG GGAACTCTTTCAAGTTCTCAAAATGATGGTAGGAAATAACACCAAACT TGCGGACACTCAACTCCAACAAATTGTTGATAAAACAATTATTAACGC TGATAAAGATGGAGATGGTCGTATTAGCTTTGAAGAATTTTGCGCAGT TGTCGGCGGTTTGGACATCCACAAGAAGATGGTAGTCGATGTTTGAAA CTTGTTTATTGCAGCTTATAATGGTTACAAATAAAGCAATAGCATCAC AAATTTCACAAATAAAGCATTTTTTTCACTGCATTCTAGTTGTGGTTT GTCCAAACTCATCAATGTATCTTACGCCGATTGGTGACAGAAAAGCCC CATCCTTAGGCCTCCTCCTTCCTAGTCTCCTGATATTGGGTCTAACCC CCACCTCCTGTTAGGCAGATTCCTTATCTGGTGACACACCCCCATTTC CTGGAGCCATCTCTCTCCTTGCCAGAACCTCTAAGGTTTGCTTACGAT GGAGCCAGAGAGGATCCTGGGAGGGAGAGCTTGGCAGGGGGTGGAGG GAAGGGGGGGATGCGTGACCTGCCCGGTTCTCAGTGGCCACCCTGCGC TACCCTCTCCCAGAACCTGAGCTGCTCTGACGCGGCCGTCTGGTGCGT TTCACTGATCCTGGTGCTGCAGCTTCCTTACACTTCCCAAGAGGAGAA GCAGTTTGGAAAAACAAAATCAGAATAAGTTGGTCCTGAGTTCTAACT TTGGCTCTTCACCTTTCTAGTCCCCAATTTATATTGTTCCTCCGTGCG TCAGTTTTACCTGTGAGATAAGGCCAGTAGCCAGCCCCGTCCTGGCAG GGCC | |
| Transgene inserted at TRAC locus (MSCV promoter-driven tLNGFR coding sequence flanked by homology arms correspondin g to the cut site of MG3-6-TRAC-6.) | CAATGGTCCTGTCTCTCAAGAATCCCCTGCCACTCCTCACACCCACCC TGGGCCCATATTCATTTCCATTTGAGTTGTTCTTATTGAGTCATCCTT CCTGTGGTAGCGGAACTCACTAAGGGGCCCATCTGGACCCGAGGTATT GTGATGATAAATTCTGAGCACCTACCCCATCCCCAGAAGGGCTCAGAA ATAAAATAAGAGCCAAGTCTAGTCGGTGTTTCCTGTCTTGAAACACAA TACTGTTGGCCCTGGAAGAATGCACAGAATCTGTTTGTAAGGGGATAT GCACAGAAGCTGCAAGGACAGGAGGTGCAGGAGCTGCAGGCCTCCCC CACCCAGCCTGCTCTGCCTTGGGGAAAACCGTGGGTGTGTCCTGCAGG CCATGCAGGCCTGGGACATGCAAGCCCATAACCGCTGTGGCCTCTTGG TTTTACAGATACGAACCTAAACTTTCAAAACCTGTCAGTGATTGGGTT CCGAATCCTCCTCCTGAAAGTTAATTAATGAATGAATGAAATAAAAGA TCTTTATTTTCATTAGATCTGTGTGTTGGTTTTTTGTGTGATCCTCGA GGGAATGAAAGACCCCACCTGTAGGTTTGGCAAGCTAGCTTAAGTAAC GCCATTTTGCAAGGCATGGAAAATACATAACTGAGAATAGAGAAGTTC AGATCAAGGTTAGGAACAGAGAGACAGCAGAATATGGGCCAAACAGGA TATCTGTGGTAAGCAGTTCCTGCCCCGGCTCAGGGCCAAGAACAGATG GTCCCCAGATGCGGTCCCGCCCTCAGCAGTTTCTAGAGAACCATCAGA | 64 |
| | TGTTTCCAGGGTGCCCCAAGGACCTGAAAATGACCCTGTGCCTTATTT GAACTAACCAATCAGTTCGCTTCTCGCTTCTGTTCGCGCGCTTCTGCT CCCCGAGCTCAATAAAAGAGCCCACAACCCCTCACTCGGCGCGCGCCA GTCCGGTACCAGTCGCCACCATGGCCCTGCCTGTGACAGCTCTGCTCC TCCCTCTGGCCCTGCTGCTCCATGCCGCCAGACCCGACATCGTGCTGA CCCAGAGCCCCCCCAGCCTGGCCATGTCTCTGGGCAAGAGAGCCACCA TCAGCTGCCGGGCCAGCGAGAGCGTGACCATCCTGGGCAGCCACCTGA TCCACTGGTATCAGCAGAAGCCCGGCCAGCCCCCCACCCTGCTGATCC AGCTCGCCAGCAATGTGCAGACCGGCGTGCCCGCCAGATTCAGCGGCA GCGGCAGCAGAACCGACTTCACCCTGACCATCGACCCCGTGGAAGAGG ACGACGTGGCCGTGTACTACTGCCTGCAGAGCCGGACCATCCCCCGGA CCTTTGGCGGAGGCACCAAACTGGAAATCAAGGGCAGCACCAGCGGCT CCGGCAAGCCTGGCTCTGGCGAGGGCAGCACAAAGGGACAGATTCAGC TGGTGCAGAGCGGCCCTGAGCTGAAGAAACCCGGCGAGACAGTGAAGA TCAGCTGCAAGGCCTCCGGCTACACCTTCACCGACTACAGCATCAACT GGGTGAAAAGAGCCCCTGGCAAGGGCCTGAAGTGGATGGGCTGGATCA ACACCGAGACAAGAGAGCCCGCCTACGCCTACGACTTCCGGGGCAGAT TCGCCTTCAGCCTGGAAACCAGCGCCAGCACCGCCTACCTGCAGATCA ACAACCTGAAGTACGAGGACACCGCCACCTACTTTTGCGCCCTGGACT ACAGCTACGCCATGGACTACTGGGGCCAGGGCACCAGCGTGACCGTGT CCAGCTTCGTGCCCGTGTTCCTGCCCGCCAAACCTACCACCACCCCTG CCCCTAGACCTCCCACCCCAGCCCCAACAATCGCCAGCCAGCCTCTGT CTCTGCGGCCCGAAGCCTGTAGACCTGCTGCCGGCGGAGCCGTGCACA CCAGAGGCCTGGACTTCGCCTGCGACATCTACATCTGGGCCCCTCTGG CCGGCACCTGTGGCGTGCTGCTGCTGAGCCTGGTGATCACCCTGTACT GCAACCACCGGAACAGAAGCAAGCGGAGCCGGCTGCTGCACAGCGACT ACATGAACATGACCCCAAGACGGCCTGGCCCCACCCGGAAGCACTACC AGCCTTACGCCCCTCCCAGAGACTTCGCCGCCTACCGGTCCAGAGTGA AGTTCAGCAGATCCGCCGACGCCCCTGCCTACCAGCAGGGACAGAACC AGCTGTACAACGAGCTGAACCTGGGCAGACGGGAAGAGTACGACGTGC TGGACAAGCGGAGAGGCCGGGACCCCGAGATGGGCGGAAAGCCCAGAC GGAAGAACCCCCAGGAAGGCCTGTATAACGAACTGCAGAAAGACAAGA TGGCCGAGGCCTACAGCGAGATCGGCATGAAGGGCGAGCGGAGGCGCG GCAAGGGCCACGATGGCCTGTACCAGGGCCTGAGCACCGCCACCAAGG ACACCTACGACGCCCTGCACATGCAGGCCCTGCCCCCCAGAGGATCCG GCGCTACAAATTTTTCACTGCTGAAACAGGCGGGTGATGTGGAGGAGA ACCCTGGACCCATGGGTGCTGGCGCAACTGGACGCGCTATGGATGGAC CTCGCTTGCTGCTTCTTCTGCTTCTCGGGGTCTCATTGGGTGGTGCTA AGGAAGCATGCCCAACGGGACTTTATACGCATAGCGGAGAGTGTTGCA AAGCTTGTAACCTGGGCGAAGGCGTCGCGCAACCTTGTGGTGCAAATC AAACCGTCTGCGAGCCATGTTTGGACTCTGTTACGTTTAGTGACGTAG TATCTGCGACAGAGCCATGCAAGCCTTGTACGGAATGTGTAGGATTGC AGAGCATGTCTGCCCCTTGTGTAGAAGCCGACGATGCAGTTTGCAGGT GCGCGTATGGCTATTACCAAGACGAAACAACCGGACGATGTGAAGCTT GCCGAGTTTGTGAAGCGGGTTCCGGGCTTGTATTCTCATGTCAGGATA AGCAGAACACCGTCTGCGAAGAGTGCCCCGATGGCACCTACAGCGATG AAGCGAACCATGTAGACCCCTGCCTGCCTTGCACCGTTTGTGAAGACA CGGAACGACAGTTGCGGGAGTGTACCCGGTGGGCAGACGCCGAGTGCG AAGAGATTCCAGGCCGCTGGATCACGCGAAGTACCCCGCCAGAAGGTT CCGACAGTACTGCACCAAGCACCCAAGAACCAGAGGCGCCCCCCGAGC AGGACCTGATTGCCTCCACCGTGGCGGGTGTTGTTACTACGGTTATGG GCTCATCCCAGCCCGTTGTTACCCGAGGAACTACAGACAACCTGATTC CGGTATATTGTTCTATCTTGGCGGCTGTAGTAGTTGGCTTGGTCGCGT ACATCGCTTTCAAAAGATGAAAGCTTGATAATCAACCTCTGGATTACA AAATTTGTGAAAGATTGACTGGTATTCTTAACTATGTTGCTCCTTTTA CGCTATGTGGATACGCTGCTTTAATGCCTTTGTATCATGCTATTGCTT | |
| | CCCGTATGGCTTTCATTTTCTCCTCCTTGTATAAATCCTGGTTAGTTC TTGCCACGGCGGAACTCATCGCCGCCTGCCTTGCCCGCTGCTGGACAG GGGCTCGGCTGTTGGGCACTGACAATTCCGTGGAACTTGTTTATTGCA GCTTATAATGGTTACAAATAAAGCAATAGCATCACAAATTTCACAAAT AAAGCATTTTTTTCACTGCATTCTAGTTGTGGTTTGTCCAAACTCATC AATGTATCTTAGTTTAAACTGGCCGGGTTTAATCTGCTCATGACGCTG CGGCTGTGGTCCAGCTGAGGTGAGGGGCCTTGAAGCTGGGAGTGGGGT TTAGGGACGCGGGTCTCTGGGTGCATCCTAAGCTCTGAGAGCAAACCT CCCTGCAGGGTCTTGCTTTTAAGTCCAAAGCCTGAGCCCACCAAACTC TCCTACTTCTTCCTGTTACAAATTCCTCTTGTGCAATAATAATGGCCT GAAACGCTGTAAAATATCCTCATTTCAGCCGCCTCAGTTGCACTTCTC CCCTATGAGGTAGGAAGAACAGTTGTTTAGAAACGAAGAAACTGAGGC CCCACAGCTAATGAGTGGAGGAAGAGAGACACTTGTGTACACCACATG CCTTGTGTTGTACTTCTCTCACCGTGTAACCTCCTCATGTCCTCTCTC CCCAGTACGGCTCTCTTAGCTCAGTAGAAAGAAGACATTACACTCATA TTACACCCCAATCCTGGCTAGAGTCTCCGCACCCTCCTC | |
| Transgene inserted at TRAC locus (MSCV promoter-driven tLNGFR coding sequence flanked by homology arms correspondin g to the cut site of MG29-1-TRAC-35.) | GCGGCCGCTTAATTAATTAATGCCAACATACCATAAACCTCCCATTCT GCTAATGCCCAGCCTAAGTTGGGGAGACCACTCCAGATTCCAAGATGT ACAGTTTGCTTTGCTGGGCCTTTTTCCCATGCCTGCCTTTACTCTGCC AGAGTTATATTGCTGGGGTTTTGAAGAAGATCCTATTAAATAAAAGAA TAAGCAGTATTATTAAGTAGCCCTGCATTTCAGGTTTCCTTGAGTGGC AGGCCAGGCCTGGCCGTGAACGTTCACTGAAATCATGGCCTCTTGGCC AAGATTGATAGCTTGTGCCTGTCCCTGAGTCCCAGTCCATCACGAGCA GCTGGTTTCTAAGATGCTATTTCCCGTATAAAGCATGAGACCGTGACT TGCCAGCCCCACAGAGCCCCGCCCTTGTCCATCACTGGCATCTGGACT CCAGCCTGGGTTGGGGCAAAGAGGGAAATGAGATCATGTCCTAACCCT GATCCTCTTGTCCCACAGATATCCAGAACCCTGACCTTAATTAATGAA TGAATGAAATAAAAGATCTTTATTTTCATTAGATCTGTGTGTTGGTTT TTTGTGTGATCCTCGAGGGAATGAAAGACCCCACCTGTAGGTTTGGCA AGCTAGCTTAAGTAACGCCATTTTGCAAGGCATGGAAAATACATAACT GAGAATAGAGAAGTTCAGATCAAGGTTAGGAACAGAGAGACAGCAGAA TATGGGCCAAACAGGATATCTGTGGTAAGCAGTTCCTGCCCCGGCTCA GGGCCAAGAACAGATGGTCCCCAGATGCGGTCCCGCCCTCAGCAGTTT CTAGAGAACCATCAGATGTTTCCAGGGTGCCCCAAGGACCTGAAAATG ACCCTGTGCCTTATTTGAACTAACCAATCAGTTCGCTTCTCGCTTCTG TTCGCGCGCTTCTGCTCCCCGAGCTCAATAAAAGAGCCCACAACCCCT CACTCGGCGCGCCAGTCCGGTACCAGTCGCCACCATGGCCCTGCCT GTGACAGCTCTGCTCCTCCCTCTGGCCCTGCTGCTCCATGCCGCCAGA CCCGACATCGTGCTGACCCAGAGCCCCCCCAGCCTGGCCATGTCTCTG GGCAAGAGAGCCACCATCAGCTGCCGGGCCAGCGAGAGCGTGACCATC CTGGGCAGCCACCTGATCCACTGGTATCAGCAGAAGCCCGGCCAGCCC CCCACCCTGCTGATCCAGCTCGCCAGCAATGTGCAGACCGGCGTGCCC GCCAGATTCAGCGGCAGCGGCAGCAGAACCGACTTCACCCTGACCATC GACCCCGTGGAAGAGGACGACGTGGCCGTGTACTACTGCCTGCAGAGC CGGACCATCCCCCGGACCTTTGGCGGAGGCACCAAACTGGAAATCAAG GGCAGCACCAGCGGCTCCGGCAAGCCTGGCTCTGGCGAGGGCAGCACA AAGGGACAGATTCAGCTGGTGCAGAGCGGCCCTGAGCTGAAGAAACCC GGCGAGACAGTGAAGATCAGCTGCAAGGCCTCCGGCTACACCTTCACC GACTACAGCATCAACTGGGTGAAAAGAGCCCCTGGCAAGGGCCTGAAG TGGATGGGCTGGATCAACACCGAGACAAGAGAGCCCGCCTACGCCTAC GACTTCCGGGGCAGATTCGCCTTCAGCCTGGAAACCAGCGCCAGCACC GCCTACCTGCAGATCAACAACCTGAAGTACGAGGACACCGCCACCTAC TTTTGCGCCCTGGACTACAGCTACGCCATGGACTACTGGGGCCAGGGC ACCAGCGTGACCGTGTCCAGCTTCGTGCCCGTGTTCCTGCCCGCCAAA CCTACCACCACCCCTGCCCCTAGACCTCCCACCCCAGCCCCAACAATC GCCAGCCAGCCTCTGTCTCTGCGGCCCGAAGCCTGTAGACCTGCTGCC | 65 |
| | GGCGGAGCCGTGCACACCAGAGGCCTGGACTTCGCCTGCGACATCTAC ATCTGGGCCCCTCTGGCCGGCACCTGTGGCGTGCTGCTGCTGAGCCTG GTGATCACCCTGTACTGCAACCACCGGAACAGAAGCAAGCGGAGCCGG CTGCTGCACAGCGACTACATGAACATGACCCCAAGACGGCCTGGCCCC ACCCGGAAGCACTACCAGCCTTACGCCCCTCCCAGAGACTTCGCCGCC TACCGGTCCAGAGTGAAGTTCAGCAGATCCGCCGACGCCCCTGCCTAC CAGCAGGGACAGAACCAGCTGTACAACGAGCTGAACCTGGGCAGACGG GAAGAGTACGACGTGCTGGACAAGCGGAGAGGCCGGGACCCCGAGATG GGCGGAAAGCCCAGACGGAAGAACCCCCAGGAAGGCCTGTATAACGAA CTGCAGAAAGACAAGATGGCCGAGGCCTACAGCGAGATCGGCATGAAG GGCGAGCGGAGGCGCGGCAAGGGCCACGATGGCCTGTACCAGGGCCTG AGCACCGCCACCAAGGACACCTACGACGCCCTGCACATGCAGGCCCTG CCCCCCAGAGGATCCGGCGCTACAAATTTTTCACTGCTGAAACAGGCG GGTGATGTGGAGGAGAACCCTGGACCCATGGGTGCTGGCGCAACTGGA CGCGCTATGGATGGACCTCGCTTGCTGCTTCTTCTGCTTCTCGGGGTC TCATTGGGTGGTGCTAAGGAAGCATGCCCAACGGGACTTTATACGCAT AGCGGAGAGTGTTGCAAAGCTTGTAACCTGGGCGAAGGCGTCGCGCAA CCTTGTGGTGCAAATCAAACCGTCTGCGAGCCATGTTTGGACTCTGTT ACGTTTAGTGACGTAGTATCTGCGACAGAGCCATGCAAGCCTTGTACG GAATGTGTAGGATTGCAGAGCATGTCTGCCCCTTGTGTAGAAGCCGAC GATGCAGTTTGCAGGTGCGCGTATGGCTATTACCAAGACGAAACAACC GGACGATGTGAAGCTTGCCGAGTTTGTGAAGCGGGTTCCGGCTTGTA TTCTCATGTCAGGATAAGCAGAACACCGTCTGCGAAGAGTGCCCCGAT GGCACCTACAGCGATGAAGCGAACCATGTAGACCCCTGCCTGCCTTGC ACCGTTTGTGAAGACACGGAACGACAGTTGCGGGAGTGTACCCGGTGG GCAGACGCCGAGTGCGAAGAGATTCCAGGCCGCTGGATCACGCGAAGT ACCCCGCCAGAAGGTTCCGACAGTACTGCACCAAGCACCCAAGAACCA GAGGCGCCCCCCGAGCAGGACCTGATTGCCTCCACCGTGGCGGGTGTT GTTACTACGGTTATGGGCTCATCCCAGCCCGTTGTTACCCGAGGAACT ACAGACAACCTGATTCCGGTATATTGTTCTATCTTGGCGGCTGTAGTA GTTGGCTTGGTCGCGTACATCGCTTTCAAAAGATGAAAGCTTGATAAT CAACCTCTGGATTACAAAATTTGTGAAAGATTGACTGGTATTCTTAAC TATGTTGCTCCTTTTACGCTATGTGGATACGCTGCTTTAATGCCTTTG TATCATGCTATTGCTTCCCGTATGGCTTTCATTTTCTCCTCCTTGTAT AAATCCTGGTTAGTTCTTGCCACGGCGGAACTCATCGCCGCCTGCCTT GCCCGCTGCTGGACAGGGGCTCGGCTGTTGGGCACTGACAATTCCGTG GAACTTGTTTATTGCAGCTTATAATGGTTACAAATAAAGCAATAGCAT CACAAATTTCACAAATAAAGCATTTTTTTCACTGCATTCTAGTTGTGG TTTGTCCAAACTCATCAATGTATCTTAGTTTAAACCTGCCGTGTACCA GCTGAGAGACTCTAAATCCAGTGACAAGTCTGTCTGCCTATTCACCGA TTTTGATTCTCAAACAAATGTGTCACAAAGTAAGGATTCTGATGTGTA TATCACAGACAAAACTGTGCTAGACATGAGGTCTATGGACTTCAAGAG CAACAGTGCTGTGGCCTGGAGCAACAAATCTGACTTTGCATGTGCAAA CGCCTTCAACAACAGCATTATTCCAGAAGACACCTTCTTCCCCAGCCC AGGTAAGGGCAGCTTTGGTGCCTTCGCAGGCTGTTTCCTTGCTTCAGG AATGGCCAGGTTCTGCCCAGAGCTCTGGTCAATGATGTCTAAAACTCC TCTGATTGGTGGTCTCGGCCTTATCCATTGCCACCAAAACCCTCTTTT TACTAAGAAACAGTGAGCCTTGTTCTGGCAGTCCAGAGAATGACACGG GAAAAAAGCAGATGAAGAGAAGGTGGCAGGAGAGGGCACGTGGCCCAG CCTCAGTGTTTAAACGCGGCCGC | |

In some cases, any of the endonucleases described herein may comprise a variant having one or more nuclear localization sequences (NLSs). The NLS may be proximal to the N- or C-terminus of the endonuclease. The NLS may be appended N-terminal or C-terminal to any one of SEQ ID NOs: 25-40, or to a variant having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identity to any one of SEQ ID NOs: 25-40. In some cases, the NLS may comprise a sequence substantially identical to any one of SEQ ID NOs: 25-40.

**Table 2: Example NLS Sequences that may be used with Cas Effectors according to the disclosure.**

| **Source** | **NLS amino acid sequence** | **SEQ ID NO:** |
|---|---|---|
| SV40 | PKKKRKV | 25 |
| nucleoplasmin bipartite NLS | KRPAATKKAGQAKKKK | 26 |
| c-myc NLS | PAAKRVKLD | 27 |
| c-myc NLS | RQRRNELKRSP | 28 |
| hRNPA1 M9 NLS | NQSSNFGPMKGGNFGGRSSGPYGGGGQYFAKPRNQGGY | 29 |
| Importin-alpha IBB domain | RMRIZFKNKGKDTAELRRRRVEVSVELRKAKKDEQILKRRNV | 30 |
| Myoma T protein | VSRKRPRP | 31 |
| Myoma T protein | PPKKARED | 32 |
| p53 | PQPKKKPL | 33 |
| mouse c-abl IV | SALIKKKKKMAP | 34 |
| influenza virus NS1 | DRLRR | 35 |
| influenza virus NS1 | PKQKKRK | 36 |
| Hepatitis virus delta antigen | RKLKKKIKKL | 37 |
| mouse Mx1 protein | REKKKFLKRR | 38 |
| human poly(ADP-ribose) polymerase | KRKGDEVDGVDEVAKKKSKK | 39 |
| steroid hormone receptors (human) glucocorticoid | RKCLQAGMNLEARKTKK | 40 |

In some cases, any of the described endonuclease methods herein can further comprise introducing to a cell a single- or double stranded DNA repair template. In some cases, the engineered nuclease system further comprises a single-stranded DNA repair template. In some cases, the engineered nuclease system further comprises a double-stranded DNA repair template. In some cases, the single- or double-stranded DNA repair template may comprise from 5' to 3': a first homology arm comprising a sequence of at least 20 nucleotides 5' to said target deoxyribonucleic acid sequence, a synthetic DNA sequence of at least 10 nucleotides, and a second homology arm comprising a sequence of at least 20 nucleotides 3' to said target sequence.

In some cases, the first homology arm comprises a sequence of at least 40, at least 50, at least 60, at least 70, at least 80, at least 90, at least 100, at least 110, at least 120, at least 130, at least 140, at least 150, at least 175, at least 200, at least 250, at least 300, at least 400, at least 500, at least 750, or at least 1000 nucleotides. In some cases, the second homology arm comprises a sequence of at least 40, at least 50, at least 60, at least 70, at least 80, at least 90, at least 100, at least 110, at least 120, at least 130, at least 140, at least 150, at least 175, at least 200, at least 250, at least 300, at least 400, at least 500, at least 750, or at least 1000 nucleotides.

In some cases, the first and second homology arms are homologous to a genomic sequence of a prokaryote. In some cases, the first and second homology arms are homologous to a genomic sequence of a bacteria. In some cases, the first and second homology arms are homologous to a genomic sequence of a fungus. In some cases, the first and second homology arms are homologous to a genomic sequence of a eukaryote.

In some cases, any of the described endonuclease methods herein can further comprise introducing to a cell a DNA repair template. The DNA repair template may comprise a double-stranded DNA segment. The double-stranded DNA segment may be flanked by one single-stranded DNA segment. The double-stranded DNA segment may be flanked by two single-stranded DNA segments. In some cases, the single-stranded DNA segments are conjugated to the 5' ends of the double-stranded DNA segment. In some cases, the single stranded DNA segments are conjugated to the 3' ends of the double-stranded DNA segment.

In some cases, the single-stranded DNA segments have a length from 1 to 15 nucleotide bases. In some cases, the single-stranded DNA segments have a length from 4 to 10 nucleotide bases. In some cases, the single-stranded DNA segments have a length of 4 nucleotide bases. In some cases, the single-stranded DNA segments have a length of 5 nucleotide bases. In some cases, the single-stranded DNA segments have a length of 6 nucleotide bases. In some cases, the single-stranded DNA segments have a length of 7 nucleotide bases. In some cases, the single-stranded DNA segments have a length of 8 nucleotide bases. In some cases, the single-stranded DNA segments have a length of 9 nucleotide bases. In some cases, the single-stranded DNA segments have a length of 10 nucleotide bases.

In some cases, the single-stranded DNA segments have a nucleotide sequence complementary to a sequence within the spacer sequence. In some cases, the double-stranded DNA sequence comprises a barcode, an open reading frame, an enhancer, a promoter, a protein-coding sequence, a miRNA coding sequence, an RNA coding sequence, or a transgene.

In some cases, sequence identity described herein may be determined by a BLASTP, CLUSTALW, MUSCLE, or MAFFT algorithm, or a CLUSTALW algorithm with the Smith-Waterman homology search algorithm parameters. The sequence identity may be determined by said BLASTP homology search algorithm using parameters of a wordlength (W) of 3, an expectation (E) of 10, and a BLOSUM62 scoring matrix setting gap costs at existence of 11, extension of 1, and using a conditional compositional score matrix adjustment.

Systems or methods of the present disclosure may be used for various applications, such as, for example, nucleic acid editing (e.g., gene editing), binding to a nucleic acid molecule (e.g., sequence-specific binding). Such systems or methods may be used, for example, for addressing (e.g., removing or replacing) a genetically inherited mutation that may cause a disease in a subject, inactivating a gene in order to ascertain its function in a cell, as a diagnostic tool to detect disease-causing genetic elements (e.g. via cleavage of reverse-transcribed viral RNA or an amplified DNA sequence encoding a disease-causing mutation), as deactivated enzymes in combination with a probe to target and detect a specific nucleotide sequence (e.g. sequence encoding antibiotic resistance int bacteria), to render viruses inactive or incapable of infecting host cells by targeting viral genomes, to add genes or amend metabolic pathways to engineer organisms to produce valuable small molecules, macromolecules, or secondary metabolites, to establish a gene drive element for evolutionary selection, to detect cell perturbations by foreign small molecules and nucleotides as a biosensor.

### EXAMPLES

### Example 1 -Editing at the TCAR locus

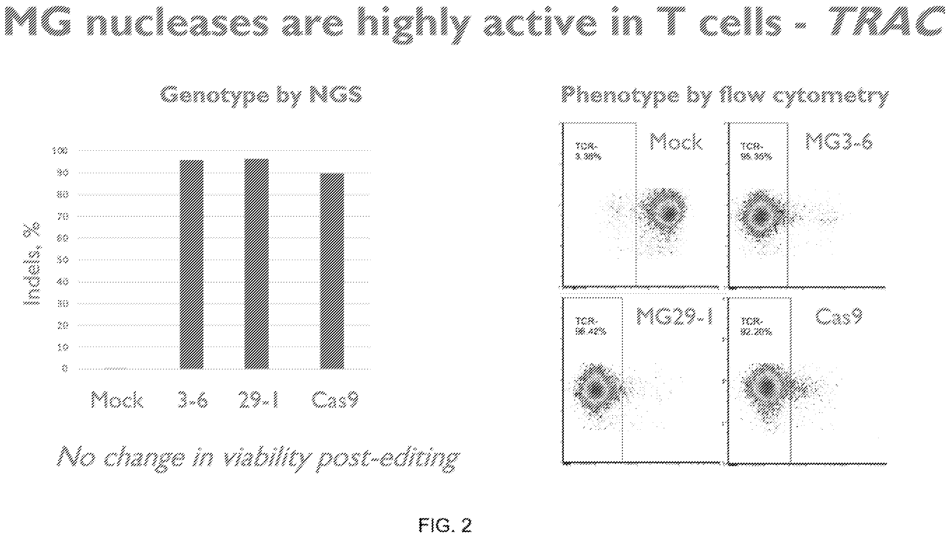

A workflow was developed to produce CAR-T cells (and other T-cell like cells bearing heterologous T-cell receptors) using the nucleases described herein **(****FIG. 1****).** Accordingly, nuclease complexes for targeting the T-cell receptor locus (e.g. TRAC locus) were developed. Spacer sequences (SEQ ID NOs: 10-15) were developed to target the TRAC gene in combination with class 2 type II endonucleases MG3-6 (SEQ ID NO:1) or SpCas9 or class 2, type V endonuclease MG29-1 (SEQ ID NO: 7) and introduced into corresponding sgRNAs for each enzyme (see TABLE 1). RNP complexes containing each TRAC-targeting sgRNA were assembled and nucleofected into human T cells (200,000 T cells with a Lonza 4-D Nucleofector, using program EO-115 and P3 buffer) that had been cultured for four days following purification from PBMCs by negative selection using (Stemcell Technologies Human T cell Isolation Kit #17951) and activation by CD2/3/28 beads (Miltenyi T cell Activation/Expansion Kit #130-091-441). The cells were analyzed by next generation sequencing (NGS) for indel formation in the TRAC gene **(****FIG. 2** **left)** and by flow cytometry for TCR expression **(****FIG.2** **right)** alongside mock-transfected T cells. Analysis by both NGS and flow cytometry indicated that MG3-6and MG29-1 were comparable or better than SpCas9 for inducing indel formation or disruption of T-cell receptor expression in transfected T-cells.

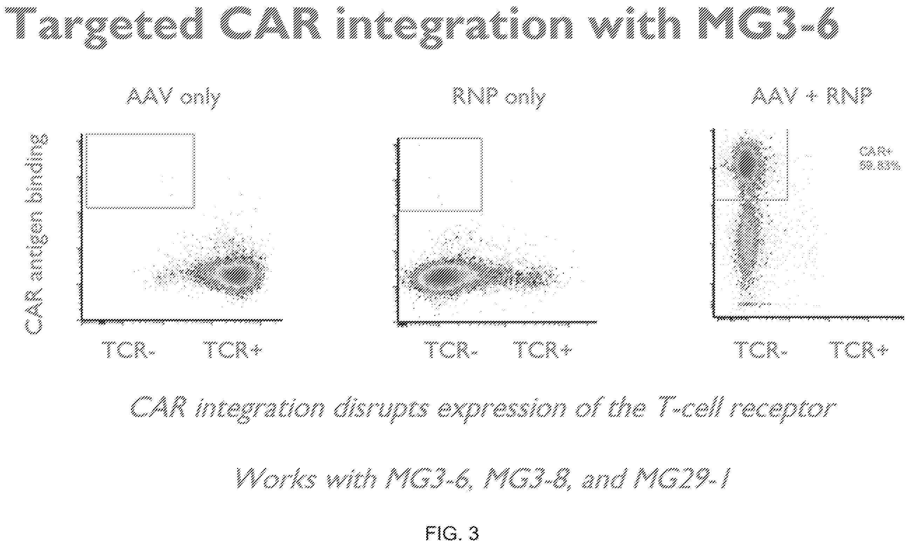

Next, the ability of editing using the RNP complexes above to promote targeted integration of a CAR-T molecule into the TRAC locus was tested. An AAV-6 vector was developed comprising a nucleotide sequence comprising a CAR-T molecule flanked by 5' and 3' homology arms (e.g. SEQ ID NOs: 23-24) targeting the TRAC gene. TRAC targeting using MG29-1 RNPs as above was performed, but then 100,000 vector genomes (vg) of the AAV-6 vector was added to the T-cells following transfection. Cells were analyzed using flow cytometry for the TCR receptor and for expression of the CAR antigen binding domain (FIG. 3). Flow cytometry indicated that approximately 60% of T-cells treated with the AAV-endonuclease combination expressed the CAR antigen binding domain. Similar results were obtained in experiments combining MG3-6 and MG3-8 RNPs targeting TRAC with the AAV-6 / CAR-T integration construct.

### Example 2 -Multiplex Editing in TCR-like cells

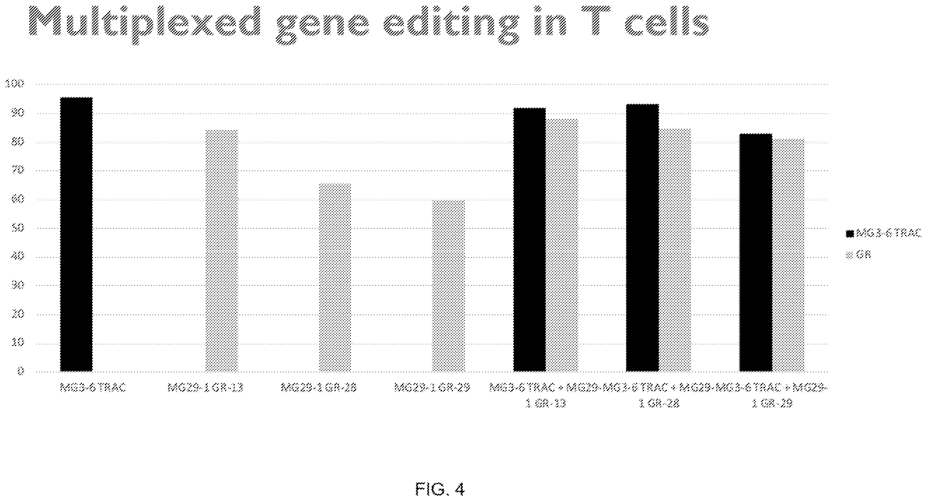

It may be advantageous to modify other genes in combination with modification of the TRAC locus (e.g. CAR-T integration). Thus, the ability of the nuclease complexes described herein to target TRAC plus an additional locus was determined. One such locus is the NR3C1 (aka the GR, or glucocorticoid receptor) locus, which may be advantageous to disrupt to confer non-responsiveness to glucocorticoid agents on CAR-T cells (e.g. in the case of cancer patients being simultaneously treated with glucocorticoids, or in the case of cancer patients having autoimmune disorders that require glucocorticoid maintenance). Three MG29-1-compatible targeting sequences (Targets B-D; SEQ ID NOs: 20, 21, 22) were designed to target the NR3C1 gene and incorporated into MG29-1 guide RNAs. RNP complexes comprising MG29-1 with these guide RNAs were assembled. T-cells were treated by nucleofection with various combinations of the MG29-1/NR3C1 gRNA RNP complexes and the MG3-6/TRAC RNPs described above (FIG.4). Cells were analyzed post-nucleofection using NGS to assess indel formation in each locus. The results indicated that while different guide RNAs had different efficiencies of targeting NR3C1 (see "MG29-1 GR-13", "MG29-1 GR-28", "MG29-1 GR-29"), combinations of MG3-6 complexes targeting TRAC and MG29-1 complexes targeting NR3C1 were able to efficiently induce indel formation in both genes (see rightmost three conditions in **FIG. 4****).**

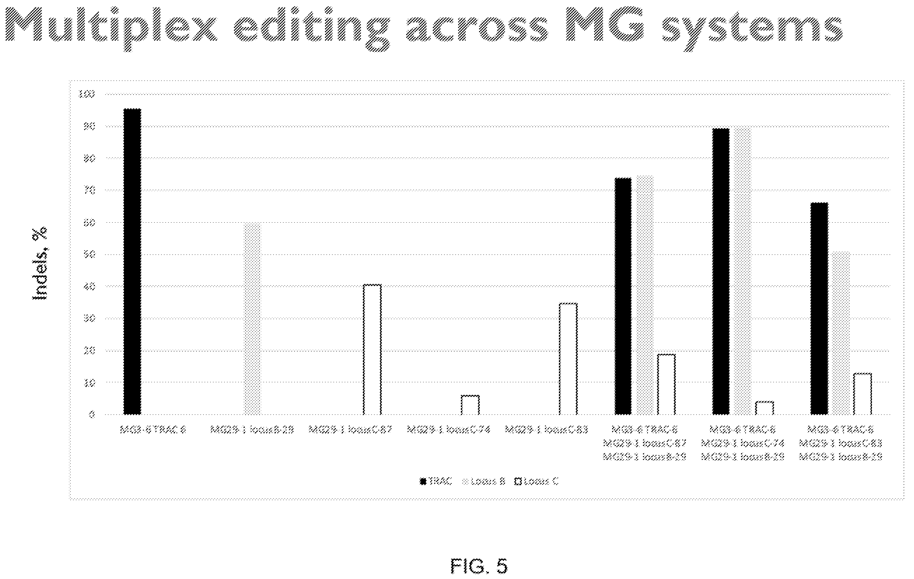

Having established the ability to target two different loci using the nuclease complexes described herein, the ability to target three loci (e.g. selected from TRAC, locus B-29/SEQ ID NO : 16, locus C-87/SEQ ID NO: 17, locus C-74/SEQ ID NO: 18, or locus C-83/SEQ ID NO: 19) was assessed by nucleofecting RNPs corresponding to each of the loci singly and in combinations of threes into T-cells as above and assessing indel formation by NGS **(****FIG. 5****).** The results of this experiment indicated that even in the conditions combining three different RNPs targeting different loci, indels in all three loci were produced at appreciable amounts.

### Example 3 - Multiplex Editing With Multiple Gene Replacement

Having established the ability to edit multiple loci and integrate a transgene into at least one locus, the ability to simultaneously edit two or more loci and simultaneously integrate genes in both loci was tested by editing two different loci within T cells and providing two different donor DNA templates targeting the two different loci. The AAVS1 (safe harbor) locus and the TRAC locus above were selected as target sites. Primary T cells (2x10⁵) prepared as in Examples 1 and 2 were nucleofected with a combination of: (a) SpCas9 (12 pmol) and a compatible sgRNA targeting the AAVS1 locus (60 pmol, SEQ ID NO: 41 denotes spacer sequence) and (b) MG3-6 (52 pmol) and the compatible TRAC3-6 6 sgRNA (60 pmol, SEQ ID NO: 10). Following nucleofection, cells were incubated with two different AAV-6 vectors each at a multiplicity of infection (MOI) of 50,000: (a) one bearing a transgene comprising each of 4 different isoforms of GR (GR-alpha, GR-beta, GR-alpha D3, and GR-beta D3) flanked by 5' and 3' homology arms (SEQ ID NOs: 42, 43) targeting the AAVS1 locus; and (b) one bearing a CAR flanked by 5' and 3' homology arms targeting the TRAC locus (SEQ ID NOs: 23-24). After four days incubation, the T cells were analyzed by: (a) PCR for the presence of the GR transgenes at the AAVS1 locus (see **FIG. 6** for PCR design and results) or (b) flow cytometry for the CAR antigen binding domain and the T-cell receptor to assess integration of CAR at the TRAC locus **(****FIG. 7****).** The data from the PCR and flow cytometry experiments indicated that both transgenes (GR and CAR) were able to be inserted simultaneously without appreciable loss of performance; PCR for the GR transgene (middle four lanes, **FIG. 6B****)** under conditions of dual AAVS1/TCR targeting showed similar integration results to AAVS1 targeting alone (last four lanes, **FIG. 7B** or middle four lanes, **FIG. 7C****)** while flow cytometry for TCR **(****FIG. 7A, 7B, 7C, 7D****)** showed high integration of CAR and loss of TCR even when AAVS1 was simultaneously targeted.

### Example 4 - Specificity Analysis by Genome-Wide Off-Target Double-Strand Break Analysis

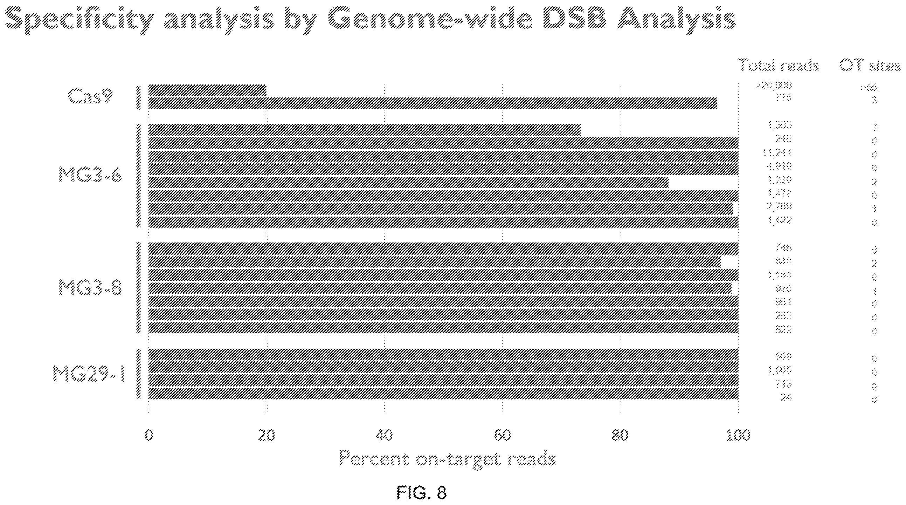

The target specificity of MG3-6, MG3-8, and MG29-1 were assessed via a genome-wide off-target double-strand break analysis alongside SpCas9 ("Cas9"). The results are presented in **FIG. 8****.** Results indicated that MG3-6, MG3-8, and MG29-1 had lower levels of off-target editing than Cas9.

### Example 5 - Multiplex Editing In T Cells

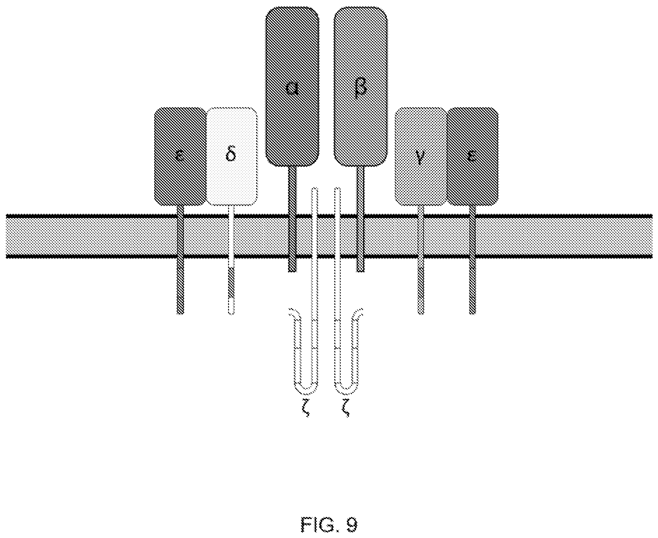

Making a recombinant-TCR-based T-cell product can require introducing new, desirable alpha and beta chains of the TCR into a pool of T cells, as the a/b chains are the subunits of the TCR that give antigen-specific recognition. These new a/b chains can then assemble with the delta/gamma/epsilon chains to make an active, full TCR (see **FIG. 9****).** Unfortunately, in this simple case, the existing a/b chains are still expressed in the recipient cell. This introduces the undesirable possibility that the existing alpha can pair with the new beta and the new alpha can pair with the existing beta, e.g., the new, desirable TCR a/b chains do not know they are "supposed" to pair together. Without further action, the T cells would now express four different TCRs (a/b, a'/b, a/b', a'/b') with one having the engineered specificity. This causes two problems: i) it reduces the expression of the new, desirable TCR by four-fold; ii) the two hybrid a/b pairs (a'/b and a/b') pose a risk of auto-immunity as they will recognize antigens in an unpredictable, potentially self-reactive manner.

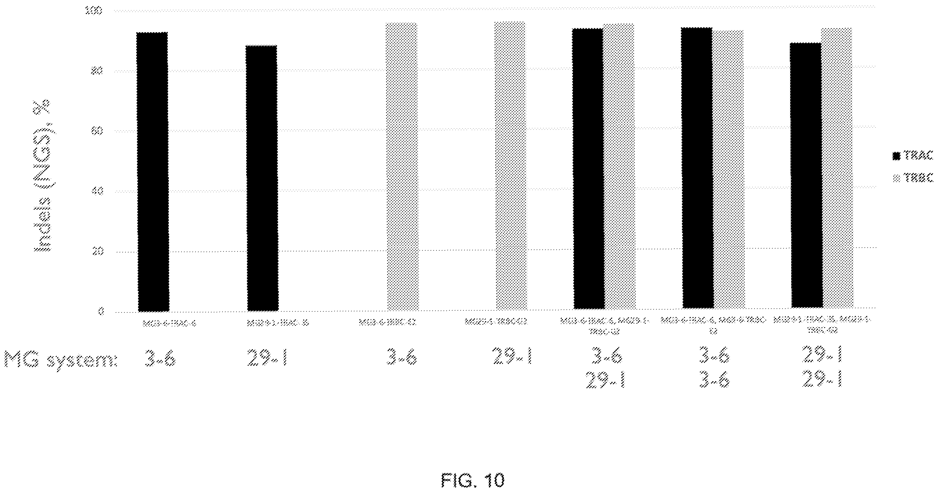

In this experiment, primary T cells, expanded with CD2/3/28 beads, were nucleofected using 200K cells per condition using Lonza 4D electroporator and solution P3, delivering 104 pmol of MG3-6 protein and 128 pmol guide RNA or the same amount of the type V enzyme MG29-1 or both MG3-6 and MG29-1. The MG3-6 guides used were MG3-6-TRAC-6 (SEQ ID NO: 44) and MG3-6-TRBC (SEQ ID NO: 45); lengths are 22 nt. Genomic DNA was harvested after 3 days from these cells and analyzed by NGS (see **FIG. 10****).** The results of **FIG. 10****,** which illustrates the percentage of sequences at the targeted sites with indels, demonstrates that there is duplex TRAC/TRBC knock out in these cells when RNPs targeting both sites are simultaneously introduced into cells.

### Example 6 - Gene-Editing Outcomes By Flow Cytometry For Single-Gene Knock-Out

Primary T cells were purified from PBMCs (peripheral blood mononuclear cells) using a negative selection kit (Miltenyi) according to the manufacturer's recommendations. Nucleofection of RNPs (100 pmol protein and 150 pmol guide RNA) was performed into T cells (200,000) using the Lonza 4D electroporator. For analysis by flow cytometry, 3 days post-nucleofection, 100,000 T cells were stained with anti-CD3 and anti-B2M antibodies for 30 minutes at 4C and analyzed on an Attune Nxt flow cytometer **(****FIG. 11). FIG. 11****,** which shows percentage of analyzed cells containing each of 4 phenotypes assessing knockout of TCR and B2M, illustrates that: (a) all of the TCR targeting conditions efficiently produced TCR knockout, with the MG3-6 TRAC6 and MG3-6 TRBC E2 sgRNAs producing the most efficient TCR knockout; and (b) all of the B2M targeting conditions produced B2M knockout, with B2M H1 and B2M D2 producing the most efficient B2M knockout.

### Example 7 - Gene-Editing Outcomes By Flow Cytometry For Double-Gene Knock-Out

After assessing the performance of the TCR/B2M targeting conditions singly in Example 6, simultaneous dual disruption using combinations of the conditions was also tested for TRAC and B2M targeting **(****FIG. 12****).** Primary T cells were purified from PBMCs using a negative selection kit (Miltenyi) according to the manufacturer's recommendations. Nucleofection of RNPs (100 pmol protein and 150 pmol guide RNA) was performed into T cells (200,000) using the Lonza 4D electroporator. For analysis by flow cytometry, 3 days post-nucleofection, 100,000 T cells were stained with anti-CD3 and anti-B2M antibodies for 30 minutes at 4C and analyzed on an Attune Nxt flow cytometer **(****FIG. 12). FIG. 12****,** which shows percentage of analyzed cells containing each of 4 phenotypes assessing knockout of TCR and B2M, illustrates that the most efficient dual targeting conditions were A4, B4, and C4, involving the MG3-6 TRAC6 condition with the MG29-1 B2M H1, D2, or A3 condition. The most efficient dual targeting condition appeared to be B4, which used the MG3-6 TRAC6 sgRNA and the MG29-1 B2M D2 sgRNA.

### Example 8 - Gene-Editing Outcomes By Flow Cytometry For Triple-Gene Knock-Out

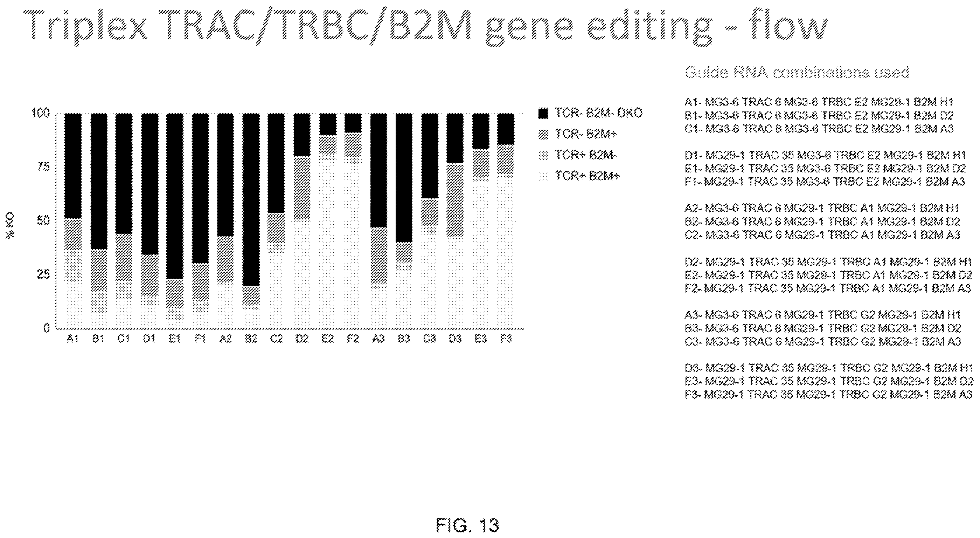

After assessing the performance of the TCR/B2M targeting conditions singly in Example 6 and doubly in Example 7, simultaneous dual disruption using combinations of the conditions was also tested for simultaneous TRAC, TRBC, and B2M targeting. Primary T cells were purified from PBMCs using a negative selection kit (Miltenyi) according to the manufacturer's recommendations. Nucleofection of RNPs (100 pmol protein and 150 pmol guide RNA) was performed into T cells (200,000) using the Lonza 4D electroporator. For analysis by flow cytometry, 3 days post-nucleofection, 100,000 T cells were stained with anti-CD3 and anti-B2M antibodies for 30 minutes at 4C and analyzed on an Attune Nxt flow cytometer **(****FIG. 13****).** The flow cytometry results indicate that conditions B2, E1, and F1 were the most efficient triple-targeting conditions for knock-out.

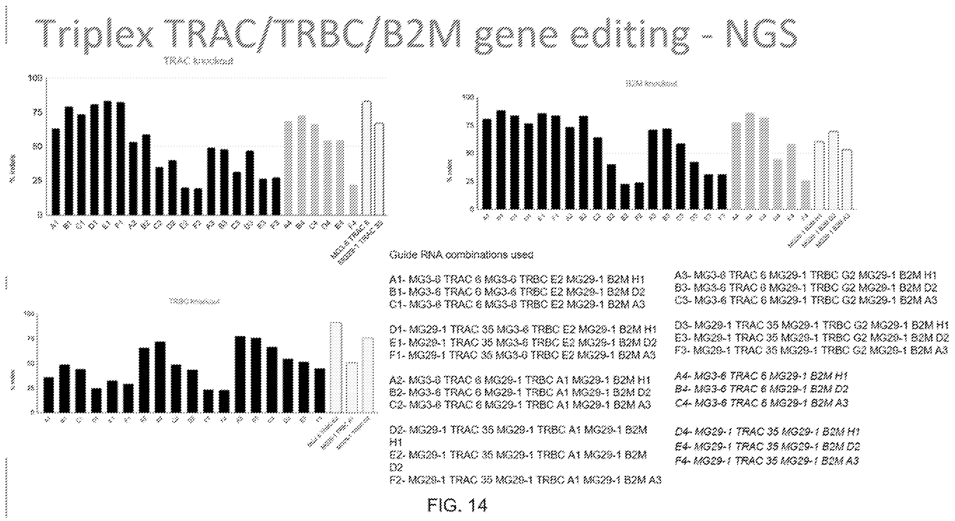

Cells were harvested and genomic DNA prepared five days post-transfection. PCR primers appropriate for use in NGS-based DNA sequencing were generated, optimized, and used to amplify the individual target sequences for each guide RNA. The amplicons were sequenced on an Illumina MiSeq machine and analyzed with a proprietary Python script to measure gene editing **(****FIG. 14****).** The sequencing results here conflicted with those in **FIG. 13** as indels may not necessarily reflect functional disruption of the gene (such as would be measured by flow cytometry).

**Table 3: gRNA combinations used in Example 8**

| **Designation** | **gRNA combination** |
|---|---|
| A1 | MG3-6 TRAC 6 MG3-6 TRBC E2 MG29-1 B2M H1 |
| B1 | MG3-6 TRAC 6 MG3-6 TRBC E2 MG29-1 B2M D2 |
| C1 | MG3-6 TRAC 6 MG3-6 TRBC E2 MG29-1 B2M A3 |
| A3 | MG3-6 TRAC 6 MG29-1 TRBC G2 MG29-1 B2M H1 |
| B3 | MG3-6 TRAC 6 MG29-1 TRBC G2 MG29-1 B2M D2 |
| C3 | MG3-6 TRAC 6 MG29-1 TRBC G2 MG29-1 B2M A3 |
| D1 | MG29-1 TRAC 35 MG3-6 TRBC E2 MG29-1 B2M H1 |
| E1 | MG29-1 TRAC 35 MG3-6 TRBC E2 MG29-1 B2M D2 |
| F1 | MG29-1 TRAC 35 MG3-6 TRBC E2 MG29-1 B2M A3 |
| D3 | MG29-1 TRAC 35 MG29-1 TRBC G2 MG29-1 B2M H1 |
| E3 | MG29-1 TRAC 35 MG29-1 TRBC G2 MG29-1 B2M D2 |
| F3 | MG29-1 TRAC 35 MG29-1 TRBC G2 MG29-1 B2M A3 |
| A2 | MG3-6 TRAC 6 MG29-1 TRBC A1 MG29-1 B2M H1 |
| B2 | MG3-6 TRAC 6 MG29-1 TRBC A1 MG29-1 B2M D2 |
| C2 | MG3-6 TRAC 6 MG29-1 TRBC A1 MG29-1 B2M A3 |
| A4 | MG3-6 TRAC 6 MG29-1 B2M H1 |
| B4 | MG3-6 TRAC 6 MG29-1 B2M D2 |
| C4 | MG3-6 TRAC 6 MG29-1 B2M A3 |
| D2 | MG29-1 TRAC 35 MG29-1 TRBC A1 MG29-1 B2M H1 |
| E2 | MG29-1 TRAC 35 MG29-1 TRBC A1 MG29-1 B2M D2 |
| F2 | MG29-1 TRAC 35 MG29-1 TRBC A1 MG29-1 B2M A3 |
| D4 | MG29-1 TRAC 35 MG29-1 B2M H1 |
| E4 | MG29-1 TRAC 35 MG29-1 B2M D2 |
| F4 | MG29-1 TRAC 35 MG29-1 B2M A3 |

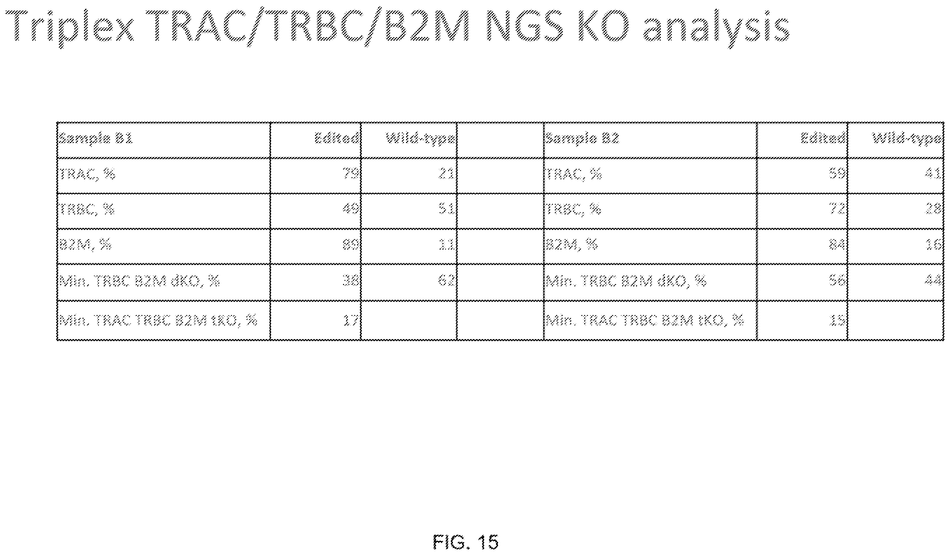

Accordingly, an additional analysis was performed (see **FIG. 15****)** to verify the generation of triple-knockout cells by sequencing. Data demonstrating the successful generation of triple knock-out cells are shown in **FIG. 15****.** Data in the "edited" columns are taken from **FIG. 14****,** while data in the "wild-type" columns are 100% minus the editing percentage. The minimum (Min.) frequencies of duplex and triplex knockout are calculated assuming the least possible overlap between editing events in individual cells. The minimum double-knockout frequency between TRBC and B2M is therefore 100% minus the percentage of cells wild-type for TRBC and minus the percentage of cells wild-type for B2M. The minimum triple-knockout frequency is therefore 100% minus the percentage of cells that might not contain a double-knockout minus the percentage of cells wild-type for TRAC. The high editing frequencies observed rule out the possibility that all of the editing events occurred in separate cells. Hence, the data in FIG. 15 demonstrate that triple-knockout TRAC/TRBC/B2M cells were successfully created.

### Example 9 - Expression Of GFP And Surface Markers In Edited T Cells

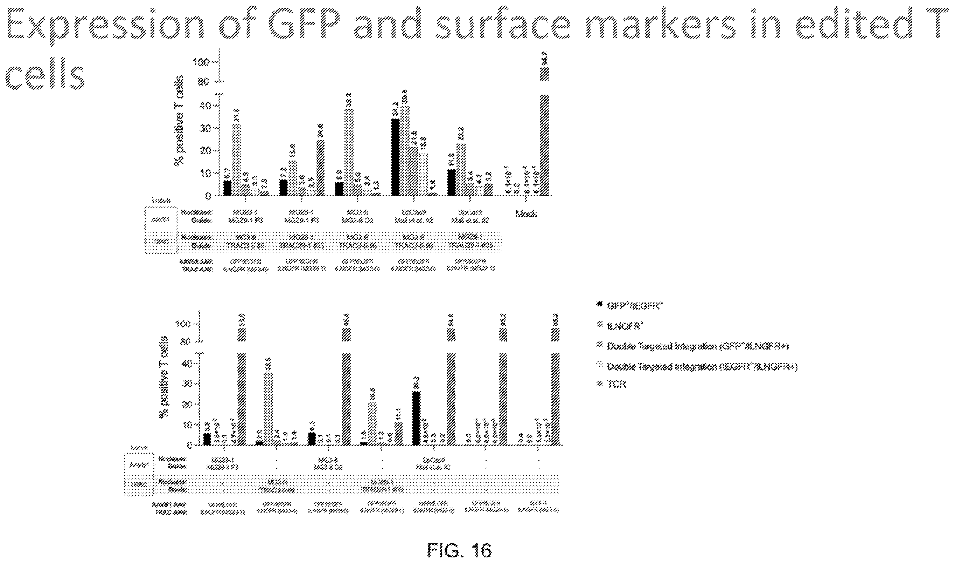

Primary human T cells were purified from PBMCs using a negative selection kit (Miltenyi) according to the manufacturer's recommendations. Nucleofection of MG3-6 mRNA (500 ng/150 pmol guide), MG29-1 RNPs (100 pmol/150 pmol guide), and/or SpCas9 RNPs (12 pmol/60 pmol guide) was performed into T cells (200,000) using a Lonza 4D electroporator. Post-nucleofection, cells were immediately recovered in media containing AAV-6 (50,000 MOI). The AAV vectors used include: (a) an AAV vector delivering a MSCV promoter-driven truncated low-affinity nerve growth factor receptor (tLNGFR) coding sequence flanked by homology arms corresponding to the cut site of MG3-6-TRAC-6 (SEQ ID NO: 64) or MG29-1-TRAC-35 (SEQ ID NO: 65); and (b) an AAV vector delivering an MND promoter-driven polycistronic construct encoding GFP alongside a truncated version of the epithelial growth factor receptor (tEGFR) flanked by homology arms corresponding to the cut site of Mali *et al.* AAVS1 T2 (SEQ ID NO: 63). Four days post-transfection, 100,000 cells were stained for viability (Live/Dead Fixable Aqua Cell Stain Kit; ThermoFisher Scientific) and expression of tLNGFR (CD271) (VioBlue REAfinity^{™}, clone REA844; Miltenyi Biotech), tEGFR (Cetuximab Biosimilar, AlexaFluor ^{®} 647, clone Hu1; R&D Systems), and TCR a/b (Brilliant Violet 785, clone IP26; BioLegend). Cells were stained for 30 min at 4 °C and data was acquired on an Attune NxT flow cytometer. Cells expressing tLNGFR, GFP, tEGFR, and/or TCR a/b were gated on single, live cells **(****FIG. 16****).**

### Example 10 - Indel Analysis At The AAVS1 Site In Edited T Cells

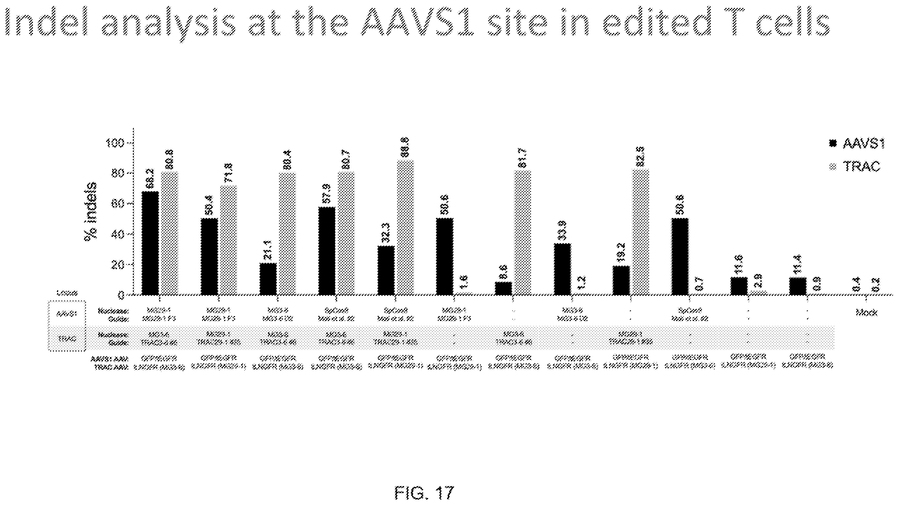

Primary T cells were purified from PBMCs using a negative selection kit (Miltenyi) according to the manufacturer's recommendations. Nucleofection of MG3-6 mRNA (500 ng/150 pmol guide), MG29-1 RNPs (100 pmol/150 pmol guide), and/or SpCas9 RNPs (12 pmol/60 pmol guide) was performed into T cells (200,000) using a Lonza 4D electroporator. Post-nucleofection, cells were immediately recovered in media containing AAV-6 (50,000 MOI). The AAV vectors used include a MSCV promoter-driven truncated low-affinity nerve growth factor receptor (tLNGFR) coding sequence flanked by homology arms corresponding to the cut site of MG3-6-TRAC-6 or MG29-1-TRAC-35, an MND promoter-driven polycistronic construct encoding GFP, and a truncated version of the epithelial growth factor receptor (tEGFR) flanked by homology arms corresponding to the cut site of Mali *et al.* AAVS1 T2. Cells were harvested and genomic DNA prepared four days post-transfection. PCR primers appropriate for use in NGS-based DNA sequencing were generated, optimized, and used to amplify a region comprising the target sites of the different AAVS1 site-specific RNA guides used in these experiments. The amplicons were sequenced on an Illumina MiSeq machine and analyzed with a proprietary Python script to measure gene editing (FIG. 17). The results illustrated that the most efficient dual-targeting condition for TRAC and AAVS1 was the conditions involving MG29-1 with sgRNA F3 and MG3-6 with sgRNA TRAC3-6 #6.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.
The following numbered paragraphs contain statements of broad combinations of technical features in accordance with various aspects of devices and methods disclosed herein:"
1. A method of editing two or more loci within a cell, comprising contacting to said cell:
   (a) a class 2, type II Cas endonuclease complex comprising:
      (i) a class 2, type II Cas endonuclease; and
      (ii) a first engineered guide RNAs comprising:
         an RNA sequence configured to bind to the class 2, type II Cas endonuclease, and
         a spacer sequence configured to hybridize to a first set of one or more target loci;
   (b) a class 2, type V Cas endonuclease complex comprising:
      (i) a class 2, type V Cas endonuclease; and
      (ii) a second engineered guide RNAs comprising:
         an RNA sequence configured to bind to the class 2, type V Cas endonuclease, and
         a spacer sequence configured to hybridize to a second set of one or more target loci.
2. The method of Para 1, wherein said class 2, type II Cas endonuclease is not a Cas9 endonuclease.
3. The method of Para 1 or 2, wherein said class 2, type II Cas endonuclease is a Cas12a endonuclease.
4. The method of any one of Paras 1-3, wherein said class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof.
5. The method of any one of Paras 1-3, wherein said class 2, type V Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to SEQ ID NO: 7 or a variant thereof.
6. The method of any one of Paras 1-5, wherein said first engineered guide RNA or said second engineered guide RNA comprises a sequence having at least 80%, 85%, 90%, or 95% sequence identity to any one of SEQ ID NOs: 3, 6, or 9.
7. The method of any one of Paras 1-6, wherein said method edits genomic sequences of said first locus with at least about 25%, 30%, 35%, 40%, 45%, 50%, 55%, 60%, 65%, 70%, 75%, 80%, 85%, 90%, or more efficiency and/or said second locus with at least about 25%, 30%, 35%, 40%, 45%, 50%, 55%, 60%, 65%, 70%, 75%, 80%, 85%, 90%, or more efficiency.
8. The method of any one of Paras 1-7, wherein said first RNA-guided endonuclease or said second RNA-guided endonuclease is introduced at a concentration of 200 pmol or less, 100 pmol or less, 50 pmol or less, 25 pmol or less, 5 pmol or less, or 1 pmol or less.
9. The method of any one of Paras 1-8, wherein off-target sites within said cell are disrupted at a frequency of less than 0.2% as determined by a genome-wide off-target double-strand break analysis.
10. The method of Para 9, wherein off-target sites within said cell are disrupted at a frequency of less than 0.01% as determined by a genome-wide off-target double-strand break analysis.
11. The method of any one of Paras 1-10, wherein said first set of one or more target loci or said second set of one or more target loci comprises a T-cell receptor (TCR) locus.
12. The method of Para 11, wherein said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 10-15, or a complement thereof.
13. The method of any one of Paras 1-12, wherein said first set of one or more target loci or said second set of one or more target loci comprises an albumin (ALB) locus.
14. The method of Para 13, wherein said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 17-19, or a complement thereof.
15. The method of any one of Paras 1-14, wherein said first set of one or more target loci or said second set of one or more target loci comprises a Nuclear Receptor Subfamily 3 Group C Member 1 (NR3C1) locus.
16. The method of Para 15, wherein said spacer sequence configured to hybridize to said first set of one or more target loci or said spacer sequence configured to hybridize to said second set of one or more target loci has at least 80%, 85%, 90%, or 95% sequence identity to any one of SEQ ID NOs: 16, 20, 21, or 22, or a complement thereof.
17. The method of any one of Paras 1-16, further comprising introducing to said cell a donor DNA sequence comprising an open reading frame encoding a heterologous engineered T-cell receptor molecule, a first homology arm comprising a DNA sequence located on a first side of said first set of one or more target loci and a second homology arm comprising a DNA sequence located on a second side of said first set of one or more target loci.
18. The method of any one of Paras 1-17, wherein editing comprises insertion of an indel, a premature termination codon, a missense codon, a frameshift mutation, an adenine deamination, a cytosine deamination, or any combination thereof.
19. A method of making a glucocorticoid-resistant engineered T cell, comprising introducing to a T-cell or a precursor thereof:
   (a) an RNA guided endonuclease complex targeting a T-cell receptor (TCR) locus, comprising:
      (i) a first RNA guided endonuclease or DNA encoding said first RNA guided endonuclease; and
      (ii) a first engineered guide RNA comprising
         an RNA sequence configured to form a complex with said first RNA guided endonuclease, and
         a first spacer sequence configured to hybridize to at least part of said TCR locus; and
   (b) an RNA guided endonuclease complex targeting a T-cell receptor Nuclear Receptor Subfamily 3 Group C Member 1 (NR3C1) locus, comprising:
      (i) a second RNA guided endonuclease; and
      (ii) a second engineered guide RNA comprising:
         an RNA sequence configured to form a complex with said second RNA guided endonuclease, and
         a second spacer sequence configured to hybridize to at least part of said NR3C1 locus.
20. The method of Para 19, wherein said at least part of said TCR locus is within said T-cell locus.
21. The method of any one of Paras 19-20, further comprising introducing to said cell (b) a donor DNA sequence comprising an open reading frame encoding a heterologous engineered T-cell receptor molecule, a first homology arm comprising a DNA sequence located on a first side of said target sequence and a second homology arm comprising a DNA sequence located on a second side of said target sequence within said TCR locus.
22. The method of any one of Paras 19-21, wherein said first RNA guided endonuclease or said second RNA guided endonuclease comprises a class 2, type II or a class 2, type V Cas endonuclease.
23. The method any one of Paras 19-22, wherein said first RNA guided endonuclease comprises said class 2, type II Cas endonuclease and said second RNA guided endonuclease comprises said class 2, type V Cas endonuclease.
24. The method of any one of Paras 19-22, wherein said second RNA guided endonuclease comprises said class 2, type II Cas endonuclease and said first RNA guided endonuclease comprises said class 2, type V Cas endonuclease.
25. The method of any one of Paras 19-24, wherein said heterologous engineered T-cell receptor is a CAR molecule.
26. The method of any one of Paras 19-25, wherein said at least part of said T cell receptor locus is a T Cell Receptor Alpha Constant (TRAC) locus or a T Cell Receptor Beta Constant (TRBC) locus.
27. The method of any one of Paras 19-26, wherein said homology arms comprise intronic or exonic regions within said TCR locus proximal to said at least part of said T cell receptor locus.
28. The method of any one of Paras 19-26, wherein said at least part of said T cell receptor locus is a first or third exon of TRAC.
29. The method of any one of Paras 19-28, wherein said method disrupts genomic sequences of said TCR locus and said NR3C1 locus with at least about 25%, 30%, 35%, 40%, 45%, 50%, 55%, 60%, 65%, 70%, 75%, 80%, 85%, 90%, or more efficiency.
30. The method of Para 28, wherein said efficiency is determined by flow cytometry for a protein expressed from said TCR and NR3C1 loci.
31. The method of any one of Paras 19-30, wherein said at least part of said NR3C1 locus is exon 2 or exon 3.
32. The method of any one of Paras 19-31, wherein said method produces cells positive for the CAR molecule and negative for NR3C1 with at least about 25%, 30%, 35%, 40%, 45%, 50%, 55%, 60%, 65%, 70%, 75%, 80%, 85%, 90%, or more efficiency.
33. The method of any one of Paras 19-32, comprising introducing (a)-(c) to said T-cell or precursor thereof simultaneously.
34. The method of any one of Paras 19-33, wherein said first RNA-guided endonuclease or said second RNA-guided endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1, 4, or 7
35. The method of any one of Paras 19-34, wherein said first engineered guide RNA or said second engineered guide RNA comprises a sequence having at least 80%, 85%, 90%, or 95% sequence identity to any one of SEQ ID NOs: 3, 6, or 9.
36. The method of any one of Paras 19-35, wherein said first RNA-guided endonuclease or said second RNA-guided endonuclease is present at a concentration of 100 pmol or less, 50 pmol or less, 25 pmol or less, 5 pmol or less, or 1 pmol or less.
37. The method of any one of Paras 19-36, wherein said T-cell or said precursor thereof comprises a T-cell, a hematopoietic stem cell (HSC), or peripheral blood mononuclear cell (PBMC).
38. The method of any one of Paras 19-37, wherein said second spacer sequence comprises a sequence having at least 80%, 85%, 90%, or 95% sequence identity to any one of SEQ ID NOs: 16, 20, 21, or 22, or a complement thereof.
39. The method of any one of Paras 19-38, wherein said first or said second spacer sequence comprises at least about 19-24 nucleotides, at least about 19 nucleotides, at least about 20 nucleotides, at least about 22 nucleotides, or at least about 24 nucleotides.
40. The method of any one of Paras 19-38, wherein said donor DNA sequence is delivered in a viral vector.
41. The method of Para 40, wherein said viral vector is an AAV or AAV-6 vector.
42. A population of glucocorticoid-resistant T cells, comprising:
   (a) an heterologous sequence within 100, 75, 50, 25, or 10 nucleotides of a hybridization region of any one of SEQ ID NOs: 10-15 within a TCR locus;
   (b) an NR3C1 locus comprising an indel.
43. The population of glucocorticoid-resistant T cells of Para 42, wherein said heterologous sequence is an indel.
44. The population of glucocorticoid-resistant T cells of Para 42 or 43, wherein said heterologous sequence comprises an open reading frame comprising a nucleotide sequence encoding a heterologous T-cell receptor or a CAR molecule.
45. The population of glucocorticoid-resistant T cells of any one of Paras 42-44, wherein said NR3C1 locus comprises an indel within 100, 75, 50, 25, or 10 nucleotides of a hybridization region of any one of SEQ ID NOs: 16, 20, 21, or 22.
46. The population of glucocorticoid-resistant T cells of any one of Paras 42-45, wherein less than 0.2% have indels at off-target loci as determined by a genome-wide off-target double-strand break analysis .
47. The population of glucocorticoid-resistant T cells of Para 43, wherein less than 0.01% have indels at off-target loci as determined by a genome-wide off-target double-strand break analysis.
48. The population of glucocorticoid-resistant T cells of any one of Paras 42-47, wherein said population of cells is substantially free of chromosomal translocations
49. A method of editing two or more loci within a cell, comprising contacting to said cell:
   (a) a first Cas endonuclease complex comprising:
      (i) a first Cas endonuclease; and
      (ii) one or more engineered guide RNAs comprising:
         an RNA sequence configured to bind to the class 2, type II Cas endonuclease, and
         a spacer sequence configured to hybridize to a first target sequence;
   (b) a second Cas endonuclease complex comprising:
      (i) a second Cas endonuclease; and
      (ii) one or more engineered guide RNAs comprising:
         an RNA sequence configured to bind to the class 2, type II Cas endonuclease, and
         a spacer sequence configured to hybridize to a second target sequence.
50. The method of Para 49, further comprising introducing to said cell
   (c) a first donor DNA sequence comprising an open reading frame encoding a first transgene, a 5' homology arm comprising a DNA sequence located on a 5' side of said first target sequence and a 3' homology arm comprising a DNA sequence located on a 3' side of said first target sequence; and
   (d) a second donor DNA sequence comprising an open reading frame encoding a second transgene, a 5' homology arm comprising a DNA sequence located on a 5' side of said second target sequence and a 3' homology arm comprising a DNA sequence located on a 3' side of said second target sequence.
51. The method of Para 50, wherein said first transgene and said second transgene are different.
52. The method of any one of Paras 50-51, wherein said first target sequence or said second target sequence is a target sequence within a T-cell receptor locus, TRAC, TRBC, NR3C1, or AAVS1 locus, or any combination thereof.
53. The method of any one of Paras 50-52, wherein said first or second transgene is an alpha, beta, alpha-D3, or beta-D3 isoform of GR, a CAR molecule, a truncated low-affinity nerve growth factor receptor (tLNGFR) sequence, a truncated version of the epithelial growth factor receptor (tEGFR), a GFP coding sequence, or any combination thereof.
54. The method of any one of Paras 50-53, wherein said 5' homology arm comprising a DNA sequence located on a 5' side of said first target sequence or said 5' homology arm comprising a DNA sequence located on a 5' side of said second target sequence comprises SEQ ID NOs: 42 or 23.
55. The method of any one of Paras 50-54, wherein said 3' homology arm comprising a DNA sequence located on a 5' side of said first target sequence or said 3' homology arm comprising a DNA sequence located on a 5' side of said second target sequence comprises SEQ ID NOs: 43 or 24.
56. The method of any one of Paras 49-55, wherein said first or said second class 2, type II Cas endonuclease comprises a sequence having at least about 75%, 80%, 85%, 86%, 87%, 88%, 89%, 90%, 91%, 92%, 93%, 94%, 95%, 96%, 97%, 98%, 99% sequence identity to any one of SEQ ID NOs: 1 or 4, or a variant thereof.
57. The method of any one of Paras 49-56, wherein said first engineered guide RNA or said second engineered guide RNA comprises a sequence having at least 80%, 85%, 90%, or 95% sequence identity to any one of SEQ ID NOs: 3, 6, or 9.
58. The method of any one of Paras 49-57, wherein said spacer sequence configured to hybridize to said first target sequence or said spacer sequence configured to hybridize to said second target sequence has at least 80%, 85%, 90%, or 95% sequence identity to any one of SEQ ID NOs: 16, 20, 21, 22, or 41, or a complement thereof.
59. The method of any one of Paras 49-58, wherein said first or said second endonuclease comprises a class 2, type II Cas endonuclease or a class 2, type V Cas endonuclease, or any combination thereof
60. An isolated nucleic acid comprising the sequence of any one of SEQ ID NOs: 63-65, or a sequence having at least about 75%, 80%, 85%, 86%, 87%, 88%, 89%, 90%, 91%, 92%, 93%, 94%, 95%, 96%, 97%, 98%, or 99% sequence identity thereto.
61. A cell comprising the isolated nucleic acid of Para 60.
62. The cell of Para 61, wherein said cell is a T-cell or precursor thereof.
63. The cell of Para 62, wherein said T-cell or precursor thereof comprises a T-cell, a hematopoietic stem cell (HSC), or a peripheral blood mononuclear cell (PBMC).
64. A vector comprising the isolated nucleic acid sequence of Para 60.
65. The vector of Para 64, wherein said vector is an adeno-associated viral (AAV) vector.
66. The vector of Para 65, wherein said AAV vector is an AAV-6 serotype vector.
67. A vector comprising a sequence having at least about 75%, 80%, 85%, 86%, 87%, 88%, 89%, 90%, 91%, 92%, 93%, 94%, 95%, 96%, 97%, 98%, or 99% sequence identity to any one of SEQ ID NOs: 23, 24, 42, or 43.
68. The vector of Para 67, further comprising a transgene flanked by said sequence having at least about 75%, 80%, 85%, 86%, 87%, 88%, 89%, 90%, 91%, 92%, 93%, 94%, 95%, 96%, 97%, 98%, or 99% sequence identity to any one of SEQ ID NOs: 23, 24, 42, or 43.
69. The vector of Para 68, wherein said transgene comprises an alpha, beta, alpha-D3, or beta-D3 isoform of GR, a CAR molecule, a truncated low-affinity nerve growth factor receptor (tLNGFR) sequence, a truncated version of the epithelial growth factor receptor (tEGFR), a GFP coding sequence, or any combination thereof
70. The vector of Para 67 or 68, comprising an tEGFR coding sequence of SEQ ID NO: 63 or a variant having at least about 75%, 80%, 85%, 86%, 87%, 88%, 89%, 90%, 91%, 92%, 93%, 94%, 95%, 96%, 97%, 98%, or 99% sequence identity thereto.
71. The vector of Para 67 or 68, comprising an tLNGFR coding sequence of SEQ ID NO: 64 or a variant having at least about 75%, 80%, 85%, 86%, 87%, 88%, 89%, 90%, 91%, 92%, 93%, 94%, 95%, 96%, 97%, 98%, or 99% sequence identity thereto.
72. The vector of 67 or 68, comprising an MND promoter of SEQ ID NO: 63 or a variant having at least about 75%, 80%, 85%, 86%, 87%, 88%, 89%, 90%, 91%, 92%, 93%, 94%, 95%, 96%, 97%, 98%, or 99% sequence identity thereto.
73. The vector of Para 67 or 68, comprising an MSCV promoter of SEQ ID NO: 64 or a variant having at least about 75%, 80%, 85%, 86%, 87%, 88%, 89%, 90%, 91%, 92%, 93%, 94%, 95%, 96%, 97%, 98%, or 99% sequence identity thereto.

## Claims

1. An engineered nuclease system for editing two or more loci within a cell, said engineered nuclease system comprising:
a) a first class 2, type II Cas endonuclease or a nucleic acid encoding said first class 2, type II Cas endonuclease; and
b) a first engineered guide ribonucleic acid or a nucleic acid encoding said first engineered guide ribonucleic acid, wherein said first engineered guide ribonucleic acid comprises:
i) a first ribonucleic acid sequence configured to bind to said first class 2, type II Cas endonuclease, and
ii) a first spacer sequence configured to hybridize to a first set of one or more target loci; and
c) a second class 2, type II Cas endonuclease or a nucleic acid encoding said second class 2, type II Cas endonuclease; and
d) a second engineered guide ribonucleic acid or a nucleic acid encoding said second engineered guide ribonucleic acid, wherein said second engineered guide ribonucleic acid comprises:
i) a second ribonucleic acid sequence configured to bind to said second class 2, type II Cas endonuclease, and
ii) a second spacer sequence configured to hybridize to a second set of one or more target loci.

2. The engineered nuclease system of claim 1, wherein said first class 2, type II Cas endonuclease or said second class 2, type II Cas endonuclease is (a) encoded by a nucleic acid comprising a sequence having at least 75% sequence identity to SEQ ID NO: 62 or (b) comprises a sequence having at least 75% sequence identity to SEQ ID NO: 4.

3. The engineered nuclease system of any one of claims 1-2, wherein said first class 2, type II Cas endonuclease is encoded by the nucleic acid comprising a sequence of SEQ ID NO: 62.

4. The engineered nuclease system of any one of claims 1-3, wherein said first engineered guide ribonucleic acid comprises a sequence having at least 80% sequence identity to SEQ ID NO: 3.

5. The engineered nuclease system of any one of claims 1-2, wherein said first class 2, type II Cas endonuclease comprises the sequence of SEQ ID NO: 4.

6. The engineered nuclease system of any one of claims 1-2 or 5, wherein said first engineered guide ribonucleic acid comprises a sequence having at least 80% sequence identity to SEQ ID NO: 6.

7. The engineered nuclease system of any one of claims 1-6, wherein said second class 2, type II Cas endonuclease is encoded by a nucleic acid comprising a sequence of SEQ ID NO: 62.

8. The engineered nuclease system of any one of claims 1-7, wherein said second engineered guide ribonucleic acid comprises a sequence having at least 80% sequence identity to SEQ ID NO: 3.

9. The engineered nuclease system of any one of claims 1-6, wherein said second class 2, type II Cas endonuclease comprises the sequence of SEQ ID NO: 4.

10. The engineered nuclease system of any one of claims 1-6 or 9, wherein said second engineered guide ribonucleic acid comprises a sequence having at least 80% sequence identity to SEQ ID NO: 6.

11. The engineered nuclease system of any one of claims 1-10, wherein said first spacer sequence configured to hybridize to said first set of one or more target loci or said second spacer sequence configured to hybridize to said second set of one or more target loci comprises a sequence having at least 80% sequence identity to any one of SEQ ID NOs: 10-13 or a complement thereof.

12. The engineered nuclease system of any one of claims 1-11, wherein said first set of one or more target loci or said second set of one or more target loci comprises a T-cell receptor (TCR) locus, an albumin (ALB) locus, a beta-2 microglobulin (B2M) locus, or a Nuclear Receptor Subfamily 3 Group C Member 1 (NR3C1) locus.

13. The engineered nuclease system of claim 12, wherein said first set of one or more target loci or said second set of one or more target loci comprises:
(a) said TCR locus, optionally wherein said TCR locus is a T Cell Receptor Alpha Constant (TRAC) locus or a T Cell Receptor Beta Constant (TRBC) locus;
(b) said B2M locus; or
(c) any combination thereof.

14. The engineered nuclease system of any one of claims 1-13, wherein said cell is a T cell or a precursor thereof.

15. The engineered nuclease system of claim 14, further comprising introducing to said T cell or said precursor thereof a donor nucleic acid sequence encoding a Chimeric Antigen Receptor (CAR) molecule.
